(19) Europäisches Patentamt
European Patent Office
Office européen des brevets

(11) Publication number: 0 183 174
B1

(12) # EUROPEAN PATENT SPECIFICATION

(45) Date of publication of patent specification: 26.10.88

(51) Int. Cl.⁴: C 07 C 127/15, C 07 D 295/20, C 07 D 211/16, C 07 D 211/70, C 07 D 265/02, C 07 D 265/06, C 07 D 265/30, C 07 D 221/22, C 07 D 217/06, C 07 D 209/44, C 07 D 223/04

(21) Application number: 85114711.6

(22) Date of filing: 19.11.85

(54) **Substituted phenoxy urea, processes for its preparation and herbicide containing it as active ingredient.**

(30) Priority: 26.11.84 JP 248040/84

(43) Date of publication of application: 04.06.86 Bulletin 86/23

(45) Publication of the grant of the patent: 26.10.88 Bulletin 88/43

(84) Designated Contracting States: CH DE FR GB IT LI

(56) References cited:
EP-A-0 066 987

TETRAHEDRON LETTERS, vol. 25, no. 48, 1984, pages 5537-5540, GB; Y. ENDO et al.: "A novel acid-catalyzed rearrangement of N-aryl-N'-aryloxyureas to biphenyl derivatives. A (5,5)-rearrangement involving three heteroatoms"

(73) Proprietor: KUMIAI CHEMICAL INDUSTRY CO., LTD.
4-26, Ikenohata 1-chome
Taitoh-ku, Tokyo (JP)

(73) Proprietor: MITSUI PETROCHEMICAL INDUSTRIES, LTD.
2-5, Kasumigaseki 3-chome Chiyoda-ku
Tokyo 100 (JP)

(72) Inventor: Hashimoto, Isao
37-25, Hirata 6-chome
Iwakuni-shi Yamaguchi-ken (JP)
Inventor: Ishida, Tatsuyoshi
2-7, Misono 1-chome
Otake-shi Hiroshima-ken (JP)
Inventor: Takahashi, Katsuya
3-8, Misono 1-chome
Otake-shi Hiroshima-ken (JP)
Inventor: Katou, Susumu
11-23, Mabuchi 4-chome
Shizuoka-shi Shizuoka-ken (JP)

(74) Representative: Wächtershäuser, Günter, Dr.
Tal 29
D-8000 München 2 (DE)

EP 0 183 174 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel substituted phenoxy urea, processes for its preparation and a herbicide containing it as an active ingredient.

Various herbicides have been used to ensure good harvest of crops. However, conventional herbicides have various problems such that the suitable period in their application is rather limited or short, or they have phytotoxicity against crop plants. Especially, urea-type compounds such as DCMU widely used as herbicides for upland, have a drawback that they can hardly be usful for paddy fields because their phytotoxicity is too strong.

U.S. Patent 3,332,975 discloses that a m-chlorophenoxy urea which is somewhat similar to the compound of the present invention, is useful as a medicine. However, this compound does show any herbicidal activities, even when used in the same manner as the compound of the present invention, as will be described hereinafter.

The present inventors have conducted extensive researches with an aim to develop a herbicide which is highly safe to the crop plants and which has an extensive period of application ranging from the preemergence of weeds to the postemergence throughout the growing period, and have found that the above-mentioned problems may be solved by the present invention.

The present invention provides a substituted phenoxy urea having the formula:

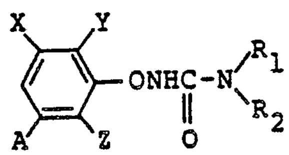

(I)

wherein A is a halogen atom or a trifluoromethyl group, each of X, Y and Z, which may be the same or different, is a hydrogen atom, a halogen atom or a trifluoromethyl group, $R_1$ is a $C_1$—$C_6$ alkyl group, a lower alkoxyl group, a lower alkoxyl-substituted lower alkyl group, a cyclo-lower alkyl-substituted lower alkyl group, a lower alkenyl group, a lower alkynyl group, a lower haloalkyl group, a lower haloalkenyl group or a $C_3$—$C_0$ non-aromatic cyclic hydrocarbon group, and $R_2$ is a hydrogen atom, a $C_1$—$C_6$ alkyl group, a lower alkenyl group or a lower alkynyl group, or $R_1$ and $R_2$ form, together with the nitrogen atom to which they are bonded, a 3—8 member ring (which may be a bicyclo ring) which may contain a double bond or an oxygen atom within the ring and may have one or more branches.

The present invention also provides the following three methods for the production of the substituted phenoxy urea:

(1) A process for preparing a substituted phenoxy urea having the formula:

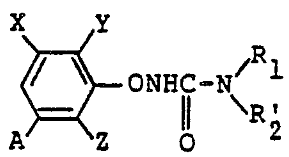

(I-I)

wherein A is a halogen atom or a trifluoromethyl group, each of X, Y and Z is a hydrogen atom, a halogen atom or a trifluoromethyl group, $R^1$ is a $C_1$—$C_6$ alkyl group, a lower alkoxyl group, a lower alkoxy-substituted lower alkyl group, a cyclo-lower alkyl substituted lower alkyl group, a lower alkenyl group, a lower alkynyl group, a lower haloalkyl group, a lower haloalkenyl group or a $C_3$—$C_9$ non-aromatic cyclic hydrocarbon group, and $R'_2$ is a $C_1$—$C_6$ alkyl group, a lower alkenyl group or a lower alkynyl group, or $R_1$ and $R'_2$ form, together with the nitrogen atom to which they are bonded, a 3—8 member ring (which may be a bicyclo ring) which may contain a double bond or an oxygen atom within the ring and may have one or more branches, which comprises reacting a phenoxyamine having the formula:

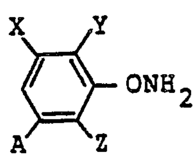

(II)

wherein A, X, Y and Z are as defined above, with a carbamic acid chloride having the formula:

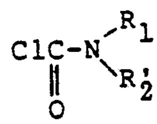

(III)

wherein $R_1$ and $R'_2$ are as defined above, in the presence of a base;

(2) A process for preparing a substituted phenoxy urea having the formula:

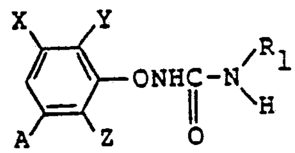

(I-II)

wherein A is a halogen atom or a trifluoromethyl group, each of X, Y and Z is a hydrogen atom, halogen atom or a trifluoromethyl group, and $R_1$ is a $C_1$—$C_6$ alkyl group, a lower alkoxyl group, a lower alkoxyl-substituted lower alkyl group, a cyclo-lower alkyl substituted lower alkyl group, a lower alkenyl group, a lower alkynyl group, a lower haloalkyl group, a lower haloalkenyl group or a $C_3$—$C_9$ non-aromatic cyclic hydrocarbon group, which comprises reacting a phenoxyamine having the formula:

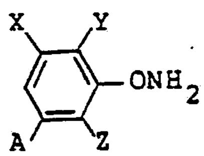

(II)

wherein A, X, Y and Z are as described above, with an isocyanate having the formula:

$$OCN—R_1 \qquad (IV)$$

wherein $R_1$ is as defined above; and

(3) A process for preparing a substituted phenoxy urea having the formula

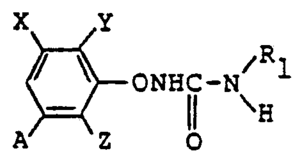

(I-II)

wherein A is a halogen atom or a trifluoromethyl group, each of X, Y and Z is a hydrogen atom, a halogen atom or a trifluoromethyl group, and $R_1$ is a $C_1$—$C_6$ alkyl group, a lower alkoxyl group, a lower alkoxyl-substituted lower alkyl group, a cyclo-lower alkyl-substituted lower alkyl group, a lower alkenyl group, a lower alkynyl group, a lower haloalkyl group, a lower haloalkenyl group or a $C_3$—$C_9$ non-aromatic cyclic hydrocarbon group, which comprises reacting a phenoxy carbamate having the formula:

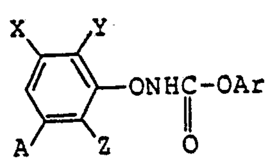

(V)

wherein A, X, Y and Z are as defined above, and Ar is an aryl group, with an amine having the formula:

$$R_1NH_2 \qquad (VI)$$

wherein $R_1$ is as defined above.

Further, the present invention provides a herbicide comprising a herbicidially effective amount of a substituted phenoxy urea of the formula I and a carrier.

Now, the present invention will be described in detail with reference to the preferred embodiments.

The substituted phenoxy urea of the present invention is represented by the formula I. In the formula I, the halogen for A is chlorine, fluorine, bromine or iodine. Likewise, the halogen for each of X, Y and Z is chlorine, fluorine, bromine or iodine. As the $C_1$—$C_6$ alkyl group for $R_1$, there may be mentioned methyl,

ethyl, n-propyl, isopropyl, n-butyl, isobutyl, sec-butyl, tert-butyl, n-amyl, isoamyl, sec-amyl, active amyl, tert-amyl, 1,2-dimethylpropyl, 2,2-dimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethylpropyl and 2-ethylbutyl. Likewise, the lower alkoxyl group is a $C_1$—$C_6$ alkoxyl group such as methoxy, ethoxy, propoxy, isopropoxy or butoxy, preferably methoxy. The lower alkoxyl group of the lower alkoxyl-substituted lower alkyl group may be the same as the above mentioned lower alkoxyl group, and the lower alkyl group thereof may be methyl, ethyl, propyl, isopropyl or butyl. A preferred lower alkoxyl-substituted lower alkyl group is 2-methoxyethyl. The cyclo-lower alkyl group of the cyclo-lower alkyl-substituted lower alkyl group may be a $C_3$—$C_5$ cycloalkyl group, and the lower alkyl group thereof may be thge same as mentioned above, preferably methyl. As the lower alkenyl group, there may be mentioned allyl, 2-butenyl, 3-butenyl, 1-methyl-2-propenyl, 2-methyl-2-propenyl, 1,2-dimethyl-2-propenyl, 2-methyl-2-butenyl, 3-methyl-2-butenyl and 2,3-dimethyl-2-butenyl. As the lower alkynyl group, there may be mentioned 2-propynyl, 2-butynyl, 3-butynyl, 1-methyl-2-propynyl and 1,1-dimethyl-2-propynyl. As the lower haloalkyl group, there may be mentioned chloromethyl, 2-chloroethyl, 2-bromoethyl, 2,2,2-trifluoroethyl and 2,2,3,3-tetrafluoropropyl. As the lower haloalkenyl group, there may be mentioned 2-chloro-2-propenyl, 2-bromo-propenyl, 1-chloromethyl-2-propenyl and 3-chloro-2-butenyl. The $C_3$—$C_9$ non-aromatic cyclic hydrocarbon group may be monocyclo or bicyclo, and may further be branched or contain a double bond. More specifically, there may be mentioned cyclopentyl, cyclobutyl, cyclopentyl, 1-methylcyclopentyl, 2-methylcyclopentyl, 3-methylcyclopentyl, 2-cyclopentenyl, cyclohexyl, 1-methylcyclohehexyl, 2-methylcyclohehexyl, 3-methylcyclohehexyl, 4-methylcyclohexyl, 2-cyclohexenyl, 3-cyclohexenyl, cycloheptyl, cyclooctyl 1-norbornyl and 2-norbornyl. As the $C_1$—$C_6$ alkyl group, the lower alkenyl group, and the lower alkynyl group for $R_2$, the above-mentioned specific, examples for $R_1$ may likewise be mentioned. As examples wherein $R_1$ and $R_2$ form, together with the nitrogen atom to which they are bonded, a 3—8 member ring (which may be a bicyclo ring) which may contain a double bond or an oxygen atom within the ring and may have a branch, there may be mentioned

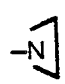 , 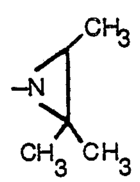 , 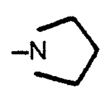 , 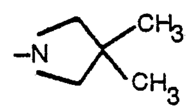 , 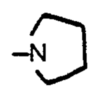 , 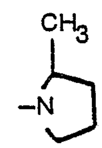 ,

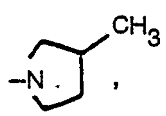 , 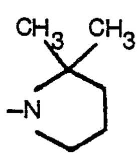 , 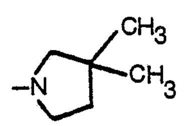 , 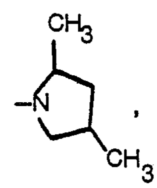 , 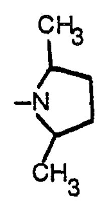 , 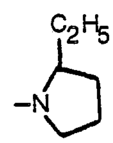 ,

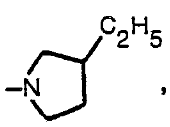 , 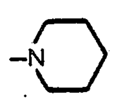 , 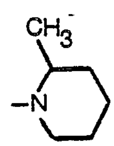 , 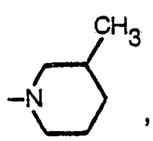 , 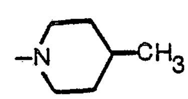 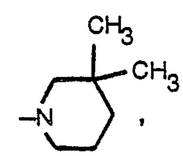 ,

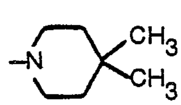 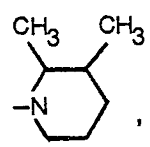 , 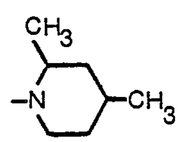 , 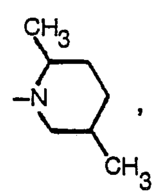 , 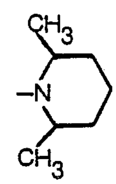 , 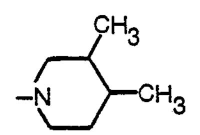 ,

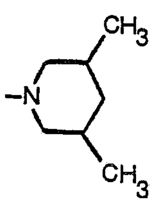 , 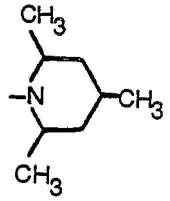 , 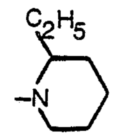 , 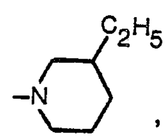 , 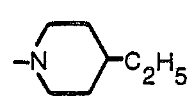 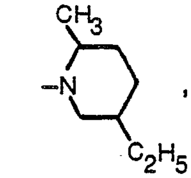 ,

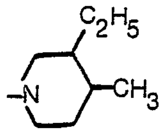 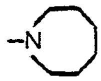 , 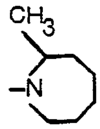 , 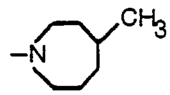 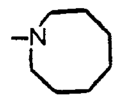 , 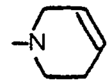 ,

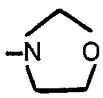 , 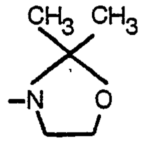 , 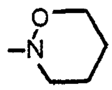 , 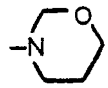 , 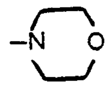 , 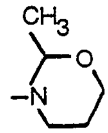 ,

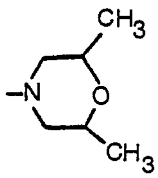 , 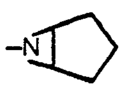 , 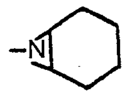 , 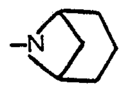 , 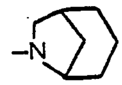 , 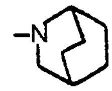 ,

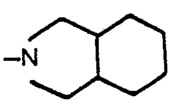 , 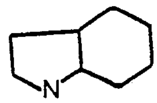 , 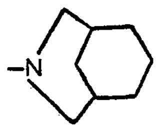 , 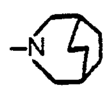 , or 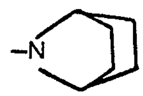 .

Further, as the aryl moiety in the formula I, there may be mentioned 3-chlorophenyl, 2,3-dichlorophenyl, 3,5-dichlorophenyl, 2,5-dichlorophenyl, 2,3,5-trichlorophenyl, 2,3,6-trichlorophenyl, 2,3,5,6-tetrachlorophenyl, 3-trifluoromethylphenyl, 3,5-di(trifluoromethyl)phenyl, 2,3,-di(trifluoromethyl)-phenyl, 2,5,-di(trifluoromethyl)-phenyl, 2-chloro-3-trifluoromethylphenyl, 3-chloro-2-trifluoromethylphenyl, 2-chloro-5-trifluoromethylphenyl, 5-chloro-2-trifluoromethylphenyl, and 3-chloro-5-trifluoromethylphenyl 5-chloro-2-trifluoromethylphenyl and 3-chloro-5-trifluoromethylphenyl.

The substance of the present invention may be in a free state or in the form of a salt, e.g. in the form of an acid addition salt. Such a salt can likewise be employed as a herbicide. As the acid constituting the acid addition salt, there may be mentioned a mineral acid such as hydrochloric acid, sulfuric acid or phosphoric acid, or an organic acid such as acetic acid, chloroacetic acid, dichloroacetic acid, trichloroacetic acid, maleic acid or citric acid.

Among the substituted phenoxy ureas of the present invention, preferred specific examples are listed in Table 1.

Table 1

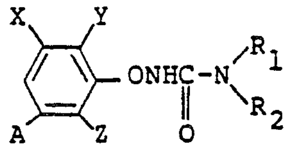

| Comp. No. | A | X | Y | Z | $R_1$ | $R_2$ |
|---|---|---|---|---|---|---|
| 1 | Cl | H | Cl | H | $CH_3$ | $CH_3$ |
| 2 | Cl | H | Cl | H | $-CH_2CH=CH_2$ | H |
| 3 | Cl | H | H | Cl | 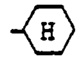 | H |
| 4 | Cl | H | H | H | $CH_3$ | $CH_3$ |
| 5 | Cl | H | H | Cl | $CH_3$ | $CH_3$ |
| 6 | Cl | Cl | H | H | $CH_3$ | $CH_3$ |
| 7 | Cl | Cl | H | Cl | $CH_3$ | $CH_3$ |
| 8 | $CF_3$ | H | H | H | $CH_3$ | $CH_3$ |
| 9 | Cl | H | H | H | $-CH_2C\equiv CH$ | $CH_3$ |
| 10 | Cl | H | H | H | $n-C_4H_9$ | $CH_3$ |
| 11 | Cl | H | H | H | $C_2H_5$ | $C_2H_5$ |
| 12 | Cl | H | H | H | $n-C_3H_7$ | $C_2H_5$ |
| 13 | Cl | H | H | H | $-CH_2CH=CH_2$ | $-CH_2CH=CH_2$ |
| 14 | Cl | H | H | H | 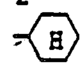 | $CH_3$ |
| 15 | Cl | H | H | H | 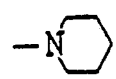 | |
| 16 | Cl | H | H | H | 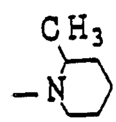 | |
| 17 | Cl | H | H | H | 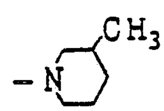 | |
| 18 | Cl | H | H | H | 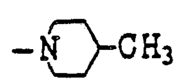 | |

Table 1 (cont'd)

| Comp. No. | A | X | Y | Z | $R_1$ | $R_2$ |
|---|---|---|---|---|---|---|
| 19 | Cl | H | H | H | 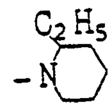 | |
| 20 | Cl | H | H | H | 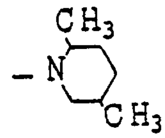 | |
| 21 | Cl | H | H | H | 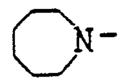 | |
| 22 | Cl | H | H | H | 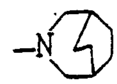 | |
| 23 | Cl | H | H | Cl | $n-C_3H_7$ | H |
| 24 | Cl | H | H | Cl | 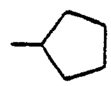 | H |
| 25 | Cl | H | H | Cl | 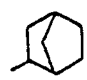 | H |
| 26 | Cl | H | H | Cl | $C_2H_5$ | $C_2H_5$ |
| 27 | Cl | H | H | Cl | $n-C_3H_7$ | $n-C_3H_7$ |
| 28 | Cl | H | H | Cl | 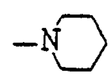 | |
| 29 | Cl | H | H | Cl | 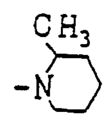 | |
| 30 | Cl | H | H | Cl | 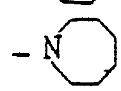 | |
| 31 | Cl | H | Cl | H | $C_2H_5$ | H |
| 32 | Cl | H | Cl | H | $-CH_2CH_2Cl$ | H |
| 33 | Cl | H | Cl | H | $n-C_3H_7$ | H |
| 34 | Cl | H | Cl | H | $i-C_3H_7$ | H |

Table 1 (cont'd)

| Comp. No. | A | X | Y | Z | $R_1$ | $R_2$ |
|---|---|---|---|---|---|---|
| 35 | Cl | H | Cl | H | 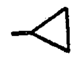 | H |
| 36 | Cl | H | Cl | H | $-CH_2C\equiv CH$ | H |
| 37 | Cl | H | Cl | H | 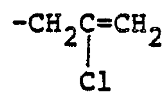 | H |
| 38 | Cl | H | Cl | H | $n-C_4H_9$ | H |
| 39 | Cl | H | Cl | H | $s-C_4H_9$ | H |
| 40 | Cl | H | Cl | H | $i-C_4H_9$ | H |
| 41 | Cl | H | Cl | H | $t-C_4H_9$ | H |
| 42 | Cl | H | Cl | H | 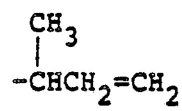 | H |
| 43 | Cl | H | Cl | H | 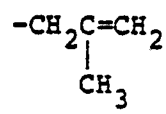 | H |
| 44 | Cl | H | Cl | H | 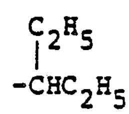 | H |
| 45 | Cl | H | Cl | H | 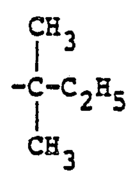 | H |
| 46 | Cl | H | Cl | H | 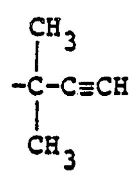 | H |
| 47 | Cl | H | Cl | H | 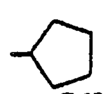 | H |
| 48 | Cl | H | Cl | H | 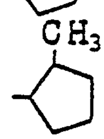 | H |

Table 1 (cont'd)

| Comp. No. | A | X | Y | Z | $R_1$ | $R_2$ |
|---|---|---|---|---|---|---|
| 49 | Cl | H | Cl | H | 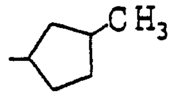 | H |
| 50 | Cl | H | Cl | H | 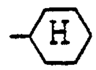 | H |
| 51 | Cl | H | Cl | H | 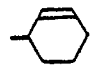 | H |
| 52 | Cl | H | Cl | H | 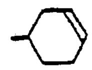 | H |
| 53 | Cl | H | Cl | H | 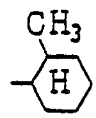 | H |
| 54 | Cl | H | Cl | H | 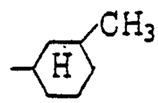 | H |
| 55 | Cl | H | Cl | H | 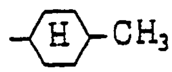 | H |
| 56 | Cl | H | Cl | H | 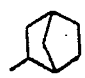 | H |
| 57 | Cl | H | Cl | H | 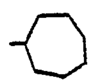 | H |
| 58 | Cl | H | Cl | H | 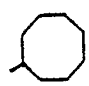 | H |
| 59 | Cl | H | Cl | H | $C_2H_5$ | $CH_3$ |
| 60 | Cl | H | Cl | H | $C_2H_5$ | $C_2H_5$ |
| 61 | Cl | H | Cl | H | $n\text{-}C_3H_7$ | $CH_3$ |
| 62 | Cl | H | Cl | H | $i\text{-}C_3H_7$ | $CH_3$ |
| 63 | Cl | H | Cl | H | $-CH_2CH{=}CH_2$ | $CH_3$ |
| 64 | Cl | H | Cl | H | $-CH_2C{\equiv}CH$ | $CH_3$ |
| 65 | Cl | H | Cl | H | $-CH_2C(Cl){=}CH_2$ | $CH_3$ |
| 66 | Cl | H | Cl | H | $n\text{-}C_4H_9$ | $CH_3$ |

Table 1 (cont'd)

| Comp. No. | A | X | Y | Z | $R_1$ | $R_2$ |
|---|---|---|---|---|---|---|
| 67 | Cl | H | Cl | H | s-$C_4H_9$ | $CH_3$ |
| 68 | Cl | H | Cl | H | i-$C_4H_9$ | $CH_3$ |
| 69 | Cl | H | Cl | H | 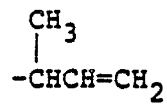 | $CH_3$ |
| 70 | Cl | H | Cl | H | 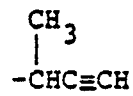 | $CH_3$ |
| 71 | Cl | H | Cl | H | 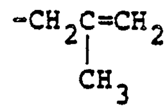 | $CH_3$ |
| 72 | Cl | H | Cl | H | 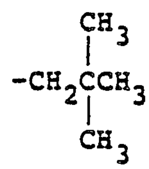 | $CH_3$ |
| 73 | Cl | H | Cl | H | 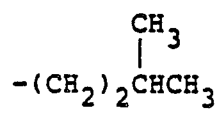 | $CH_3$ |
| 74 | Cl | H | Cl | H | 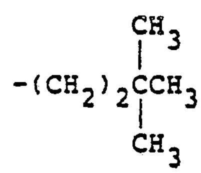 | $CH_3$ |
| 75 | Cl | H | Cl | H | 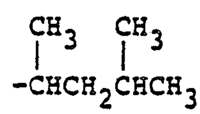 | $CH_3$ |
| 76 | Cl | H | Cl | H | n-$C_3H_7$ | $C_2H_5$ |
| 77 | Cl | H | Cl | H | i-$C_3H_7$ | $C_2H_5$ |
| 78 | Cl | H | Cl | H | n-$C_4H_9$ | $C_2H_5$ |
| 79 | Cl | H | Cl | H | n-$C_3H_7$ | n-$C_3H_7$ |
| 80 | Cl | H | Cl | H | $-CH_2CH=CH_2$ | $-CH_2CH=CH_2$ |
| 81 | Cl | H | Cl | H | $-CH_2C\equiv CH$ | $-CH_2C\equiv CH$ |

Table 1 (cont'd)

| Comp. No. | A | X | Y | Z | $R_1$ | $R_2$ |
|---|---|---|---|---|---|---|
| 82 | Cl | H | Cl | H | 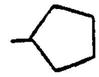 | $CH_3$ |
| 83 | Cl | H | Cl | H | 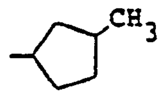 | $CH_3$ |
| 84 | Cl | H | Cl | H | 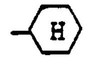 | $CH_3$ |
| 85 | Cl | H | Cl | H | 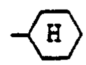 | $C_2H_5$ |
| 86 | Cl | H | Cl | H | 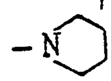 | |
| 87 | Cl | H | Cl | H | 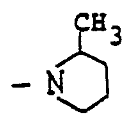 | |
| 88 | Cl | H | Cl | H | 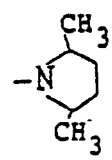 | |
| 89 | Cl | H | Cl | H | 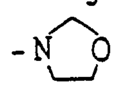 | |
| 90 | Cl | H | Cl | H | 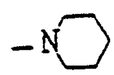 | |
| 91 | Cl | H | Cl | H | 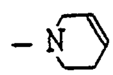 | |
| 92 | Cl | H | Cl | H | 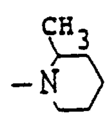 | |
| 93 | Cl | H | Cl | H | 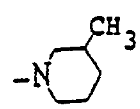 | |
| 94 | Cl | H | Cl | H | 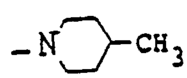 | |
| 95 | Cl | H | Cl | H | 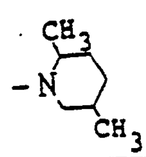 | |

Table 1 (cont'd)

| Comp. No. | A | X | Y | Z | $R_1$ | $R_2$ |
|---|---|---|---|---|---|---|
| 96 | Cl | H | Cl | H | 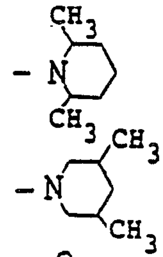 | |
| 97 | Cl | H | Cl | H | | |
| 98 | Cl | H | Cl | H | 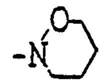 | |
| 99 | Cl | H | Cl | H | 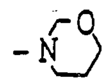 | |
| 100 | Cl | H | Cl | H | 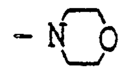 | |
| 101 | Cl | H | Cl | H | 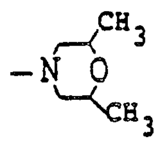 | |
| 102 | Cl | H | Cl | H | 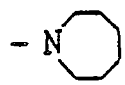 | |
| 103 | Cl | H | Cl | H | 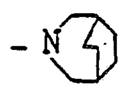 | |
| 104 | Cl | H | Cl | H | 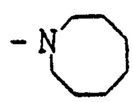 | |
| 105 | Cl | H | Cl | H | 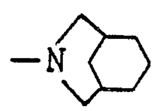 | |
| 106 | Cl | H | Cl | H | 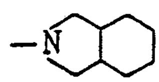 | |
| 107 | Cl | Cl | H | H | $i-C_3H_7$ | H |
| 108 | Cl | Cl | H | H | $t-C_4H_9$ | H |
| 109 | Cl | Cl | H | H | $-C(CH_3)_2-C_2H_5$ | H |

Table 1 (cont'd)

| Comp. No. | A | X | Y | Z | $R_1$ | $R_2$ |
|---|---|---|---|---|---|---|
| 110 | Cl | Cl | H | H | 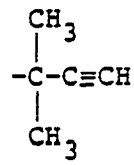 | H |
| 111 | Cl | Cl | H | H | 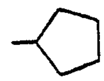 | H |
| 112 | Cl | Cl | H | H | 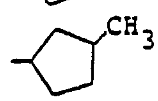 | H |
| 113 | Cl | Cl | H | H | $n-C_3H_7$ | $CH_3$ |
| 114 | Cl | Cl | H | H | $i-C_3H_7$ | $CH_3$ |
| 115 | Cl | Cl | H | H | $-CH_2CH=CH_2$ | $CH_3$ |
| 116 | Cl | Cl | H | H | $-CH_2C\equiv CH$ | $CH_3$ |
| 117 | Cl | Cl | H | H | $-CH_2CF_2CF_2H$ | $CH_3$ |
| 118 | Cl | Cl | H | H | 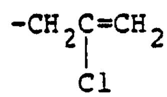 | $CH_3$ |
| 119 | Cl | Cl | H | H | $n-C_4H_9$ | $CH_3$ |
| 120 | Cl | Cl | H | H | $s-C_4H_9$ | $CH_3$ |
| 121 | Cl | Cl | H | H | $i-C_4H_9$ | $CH_3$ |
| 122 | Cl | Cl | H | H | 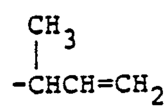 | $CH_3$ |
| 123 | Cl | Cl | H | H | 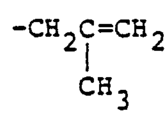 | $CH_3$ |
| 124 | Cl | Cl | H | H | 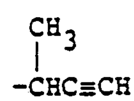 | $CH_3$ |
| 125 | Cl | Cl | H | H | 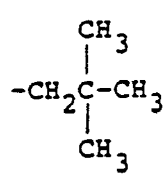 | $CH_3$ |

Table 1 (cont'd)

| Comp. No. | A | X | Y | Z | $R_1$ | $R_2$ |
|---|---|---|---|---|---|---|
| 126 | Cl | Cl | H | H | 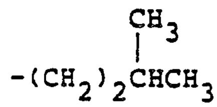 | $CH_3$ |
| 127 | Cl | Cl | H | H | 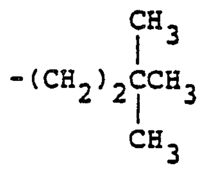 | $CH_3$ |
| 128 | Cl | Cl | H | H | $C_2H_5$ | $C_2H_5$ |
| 129 | Cl | Cl | H | H | $n-C_3H_7$ | $C_2H_5$ |
| 130 | Cl | Cl | H | H | $i-C_3H_7$ | $C_2H_5$ |
| 131 | Cl | Cl | H | H | $n-C_3H_7$ | $n-C_3H_7$ |
| 132 | Cl | Cl | H | H | $-CH_2CH=CH_2$ | $-CH_2CH=CH_2$ |
| 133 | Cl | Cl | H | H | $-CH_2C\equiv CH$ | $-CH_2C\equiv CH$ |
| 134 | Cl | Cl | H | H | 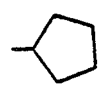 | $CH_3$ |
| 135 | Cl | Cl | H | H | 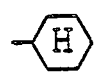 | $CH_3$ |
| 136 | Cl | Cl | H | H | 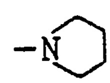 | |
| 137 | Cl | Cl | H | H | 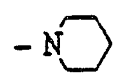 | |
| 138 | Cl | Cl | H | H | 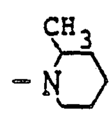 | |
| 139 | Cl | Cl | H | H | 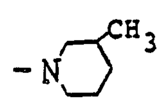 | |
| 140 | Cl | Cl | H | H | 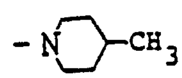 | |

Table 1 (cont'd)

| Comp. No. | A | X | Y | Z | $R_1$ | $R_2$ |
|---|---|---|---|---|---|---|
| 141 | Cl | Cl | H | H | 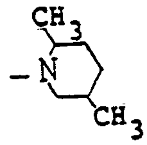 | |
| 142 | Cl | Cl | H | H | 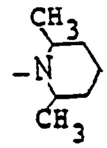 | |
| 143 | Cl | Cl | H | H | 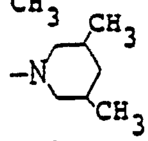 | |
| 144 | Cl | Cl | H | H | 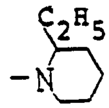 | |
| 145 | Cl | Cl | H | H | 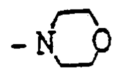 | |
| 146 | Cl | Cl | H | H | 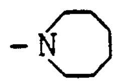 | |
| 147 | Cl | Cl | H | H | 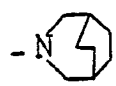 | |
| 148 | Cl | Cl | H | H | 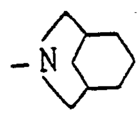 | |
| 149 | Cl | Cl | H | H | 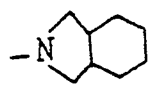 | |
| 150 | $CF_3$ | H | H | H | 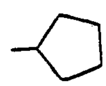 | H |
| 151 | $CF_3$ | H | H | H | $i\text{-}C_3H_7$ | $CH_3$ |
| 152 | $CF_3$ | H | H | H | $-CH_2CH{=}CH_2$ | $CH_3$ |
| 153 | $CF_3$ | H | H | H | $-CH_2C{\equiv}CH$ | $CH_3$ |
| 154 | $CF_3$ | H | H | H | $-CH_2CF_2CF_2H$ | $CH_3$ |

Table 1 (cont'd)

| Comp. No. | A | X | Y | Z | $R_1$ | $R_2$ |
|---|---|---|---|---|---|---|
| 155 | $CF_3$ | H | H | H | $-CH_2C=CH_2$<br>\|<br>Cl | $CH_3$ |
| 156 | $CF_3$ | H | H | H | $n-C_4H_9$ | $CH_3$ |
| 157 | $CF_3$ | H | H | H | $s-C_4H_9$ | $CH_3$ |
| 158 | $CF_3$ | H | H | H | $i-C_4H_9$ | $CH_3$ |
| 159 | $CF_3$ | H | H | H | $CH_3$<br>\|<br>$-CHCH=CH_2$ | $CH_3$ |
| 160 | $CF_3$ | H | H | H | $-CH_2C=CH_2$<br>\|<br>$CH_3$ | $CH_3$ |
| 161 | $CF_3$ | H | H | H | $CH_3$<br>\|<br>$-CHC\equiv CH$ | $CH_3$ |
| 162 | $CF_3$ | H | H | H | $CH_3$<br>\|<br>$-CH_2C-CH_3$<br>\|<br>$CH_3$ | $CH_3$ |
| 163 | $CF_3$ | H | H | H | $CH_3$<br>\|<br>$-(CH_2)_2CHCH_3$ | $CH_3$ |
| 164 | $CF_3$ | H | H | H | $CH_3$ $CH_3$<br>\| \|<br>$-CH-C-CH_3$<br>\|<br>$CH_3$ | $CH_3$ |
| 165 | $CF_3$ | H | H | H | $CH_3$ $CH_3$<br>\| \|<br>$-CHCH_2CHCH_3$ | $CH_3$ |
| 166 | $CF_3$ | H | H | H | $CH_3$<br>\|<br>$-(CH_2)_2CCH_3$<br>\|<br>$CH_3$ | $CH_3$ |

Table 1 (cont'd)

| Comp. No. | A | X | Y | Z | $R_1$ | $R_2$ |
|---|---|---|---|---|---|---|
| 167 | $CF_3$ | H | H | H | $C_2H_5$ | $C_2H_5$ |
| 168 | $CF_3$ | H | H | H | $n-C_3H_7$ | $C_2H_5$ |
| 169 | $CF_3$ | H | H | H | 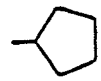 | $CH_3$ |
| 170 | $CF_3$ | H | H | H | 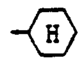 | $CH_3$ |
| 171 | $CF_3$ | H | H | H | 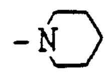 | |
| 172 | $CF_3$ | H | H | H | 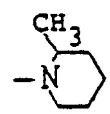 | |
| 173 | $CF_3$ | H | H | H | 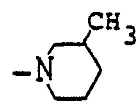 | |
| 174 | $CF_3$ | H | H | H | 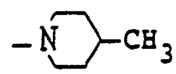 | |
| 175 | $CF_3$ | H | H | H | 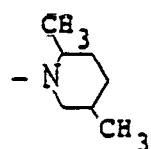 | |
| 176 | $CF_3$ | H | H | H | 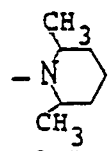 | |
| 177 | $CF_3$ | H | H | H | 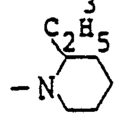 | |
| 178 | $CF_3$ | H | H | H | 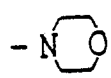 | |
| 179 | $CF_3$ | H | H | H | 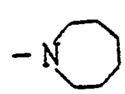 | |

Table 1 (cont'd)

| Comp. No. | A | X | Y | Z | $R_1$ | $R_2$ |
|---|---|---|---|---|---|---|
| 180 | $CF_3$ | H | H | H | 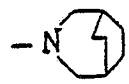 | |
| 181 | $CF_3$ | H | H | H | 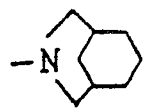 | |
| 182 | $CF_3$ | H | $CF_3$ | H | 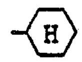 | H |
| 183 | $CF_3$ | $CF_3$ | H | H | 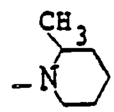 | |
| 184 | 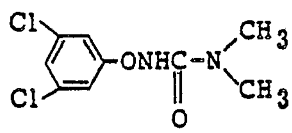 $.CCl_3CO_2H$ | | | | | |
| 185 | 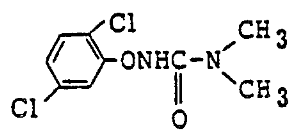 $.CCl_3CO_2H$ | | | | | |
| 186 | 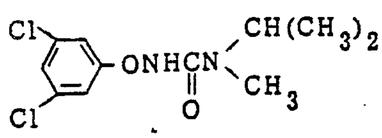 $.CCl_3CO_2H$ | | | | | |
| 187 | Cl | H | Cl | H | $-CH_2CH=CHCH_3$ | $CH_3$ |
| 188 | Cl | H | Cl | H | $-CH(CH_3)-(CH_2)_2CH_3$ | $CH_3$ |
| 189 | Cl | H | Cl | H | $-CH(CH_3)CH(CH_3)_2$ | $CH_3$ |

Table 1 (cont'd)

| Comp. No. | A | X | Y | Z | $R_1$ | $R_2$ |
|---|---|---|---|---|---|---|
| 190 | Cl | H | Cl | H | 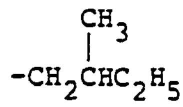 | $CH_3$ |
| 191 | Cl | H | Cl | H | $-CH(C_2H_5)_2$ | $CH_3$ |
| 192 | Cl | H | Cl | H | 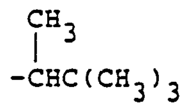 | $CH_3$ |
| 193 | Cl | H | Cl | H | 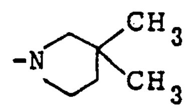 | |
| 194 | Cl | H | Cl | H | 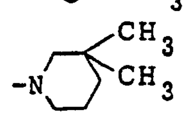 | |
| 195 | Cl | H | Cl | H | 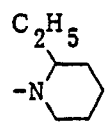 | |
| 196 | Cl | H | Cl | H | 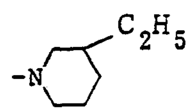 | |
| 197 | Cl | H | Cl | H | 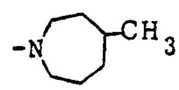 | |
| 198 | Cl | H | Cl | H | 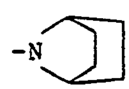 | |
| 199 | Cl | Cl | H | H | $-CH_2CH=CHCH_3$ | $CH_3$ |

Table 1 (cont'd)

| Comp. No. | A | X | Y | Z | $R_1$ | $R_2$ |
|---|---|---|---|---|---|---|
| 200 | Cl | Cl | H | H | $CH_3$<br>\|<br>$-CH(CH_2)_2CH_3$ | $CH_3$ |
| 201 | Cl | Cl | H | H | $CH_3$<br>\|<br>$-CH_2CHC_2H_5$ | $CH_3$ |
| 202 | Cl | Cl | H | H | $CH_3$<br>\|<br>$-CHCH(CH_3)_2$ | $CH_3$ |
| 203 | Cl | Cl | H | H | $-CH(C_2H_5)_2$ | $CH_3$ |
| 204 | Cl | Cl | H | H | $CH_3$ $CH_3$<br>\| \|<br>$-CHCH_2CHCH_3$ | $CH_3$ |
| 205 | Cl | Cl | H | H | $-(CH_2)_2C(CH_3)_3$ | $CH_3$ |
| 206 | Cl | Cl | H | H | 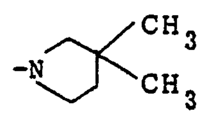 | |
| 207 | Cl | Cl | H | H | 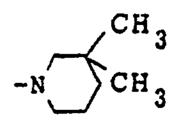 | |
| 208 | Cl | Cl | H | H | 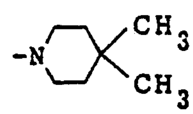 | |
| 209 | Cl | Cl | H | H | 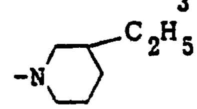 | |
| 210 | Cl | Cl | H | H | 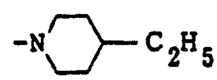 | |

Table 1 (cont'd)

| Comp. No. | A | X | Y | Z | R1 | R2 |
|---|---|---|---|---|---|---|
| 211 | Cl | Cl | H | H | 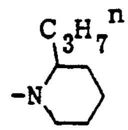 | |
| 212 | Cl | Cl | H | H | 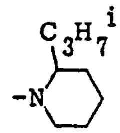 | |
| 213 | Cl | Cl | H | H | 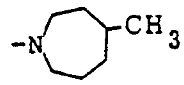 | |
| 214 | Cl | Cl | H | H | 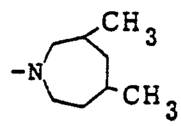 | |
| 215 | Cl | Cl | H | H | 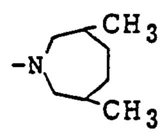 | |
| 216 | Cl | Cl | H | H | 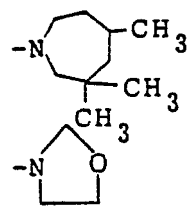 | |
| 217 | Cl | Cl | H | H | | |
| 218 | Cl | Cl | H | H | 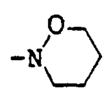 | |
| 219 | Cl | Cl | H | H | 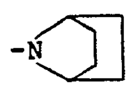 | |
| 220 | Cl | Cl | H | H | 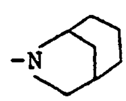 | |
| 221 | Cl | Cl | H | H | 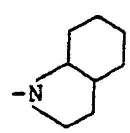 | |

Table 1 (cont'd)

| Comp. No. | A | X | Y | Z | $R_1$ | $R_2$ |
|---|---|---|---|---|---|---|
| 222 | Cl | Cl | H | H | 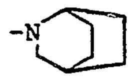 | |
| 223 | Cl | Cl | H | H | 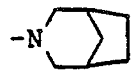 | |
| 224 | Cl | Cl | H | H | 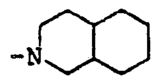 | |
| 225 | $CF_3$ | H | H | H | $-CH_2CH=CHCH_3$ | $CH_3$ |
| 226 | $CF_3$ | H | H | H | $CH_3$<br>\|<br>$-CH-(CH_2)_2CH_3$ | $CH_3$ |
| 227 | $CF_3$ | H | H | H | $CH_3$<br>\|<br>$-CHCH(CH_3)_2$ | $CH_3$ |
| 228 | $CF_3$ | H | H | H | $CH_3$<br>\|<br>$-CH_2CHC_2H_5$ | $CH_3$ |
| 229 | $CF_3$ | H | H | H | $-CH(C_2H_5)_2$ | $CH_3$ |
| 230 | $CF_3$ | H | H | H | 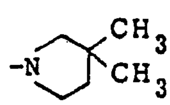 | |
| 231 | $CF_3$ | H | H | H | 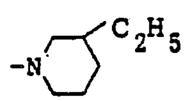 | |

Table 1 (cont'd)

| Comp. No. | A | X | Y | Z | $R_1$ | $R_2$ |
|---|---|---|---|---|---|---|
| 232 | $CF_3$ | H | H | H | 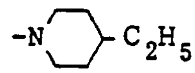 | |
| 233 | $CF_3$ | H | H | H | 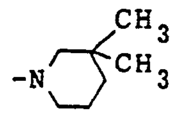 | |
| 234 | $CF_3$ | H | H | H | 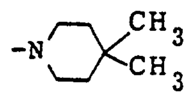 | |
| 235 | $CF_3$ | H | H | H | 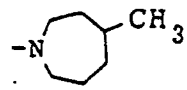 | |
| 236 | $CF_3$ | H | H | H | 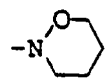 | |
| 237 | $CF_3$ | H | H | H | 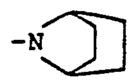 | |
| 238 | $CF_3$ | H | H | H | 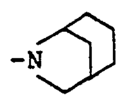 | |
| 239 | $CF_3$ | H | H | H | 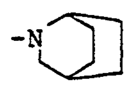 | |
| 240 | $CF_3$ | H | H | H | 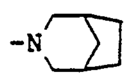 | |
| 241 | $CF_3$ | $CF_3$ | H | H | 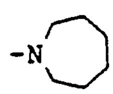 | |
| 242 | Cl | H | Cl | H | $-OCH_3$ | $CH_3$ |

Table 1 (cont'd)

| Comp. No. | A | X | Y | Z | $R_1$ | $R_2$ |
|---|---|---|---|---|---|---|
| 243 | Cl | H | Cl | H | $-(CH_2)_2OCH_3$ | H |
| 244 | Cl | H | Cl | H | $-(CH_2)_2OCH_3$ | $CH_3$ |
| 245 | Cl | H | Cl | H | $-(CH_2)_2OCH_3$ | $C_2H_5$ |
| 246 | Cl | H | Cl | H | $-CH_2-$ 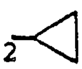 | $CH_3$ |
| 247 | Cl | Cl | H | H | $-(CH_2)_2OCH_3$ | $CH_3$ |
| 248 | Cl | Cl | H | H | $-(CH_2)_2OCH_3$ | $C_3H_7{}^n$ |
| 249 | Cl | Cl | H | H | $-CH_2-$ 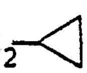 | $CH_3$ |
| 250 | $CF_3$ | H | H | H | $-(CH_2)_2OCH_3$ | $CH_3$ |
| 251 | $CF_3$ | H | H | H | $-CH_2-$ 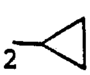 | $CH_3$ |

Table 1 (cont'd)

| Comp. No. | A | X | Y | Z | $R_1$ | $R_2$ |
|---|---|---|---|---|---|---|
| 252 | F | H | F | H | 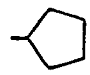 | H |
| 253 | F | H | F | H | 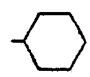 | H |

Process 1

Among the substituted phenoxy ureas of the present invention, those wherein $R_2$ is other than a hydrogen atom, i.e. $R'_2$, can be prepared from compounds having the formulas II and III. As the phenoxyamines of the formula II, there may be employed those having aryl moieties listed above with respect to the compounds of the formula I. Further, $R_1$ and $R'_2$ of the formula III may be those listed above with respect to the compounds of the formula I.

As the base to be used in the reaction, there may be mentioned a pyridine base such as pyridine, picoline, lutidene or collidine; a tertiary amine such as triethylamine, 1,8-diazabicyclo[5,4,0]undecene-7 or N,N-dimethylaniline; or an organic base such as $NaHCO_3$, $KHCO_3$, $Na_2CO_3$ or $K_2CO_3$. Among them, pyridine is preferred. The base is used in an amount of from 0.5 to 20, preferably from 1 to 10 in the molar ratio relative to the carbamic acid chloride of the formula III.

No solvent may be used for the reaction. However, it is possible to employ a solvent inert to the reaction, for instance, an aromatic hydrocarbon such as benzene, toluene or xylene; a halogenated hydrocarbon such as chloroform, dichloromethane, carbon tetrachloride, dichloroethane, trichloroethane, tetrachloroethane, chlorobenzene or dichlorobenzene; tetrahydrofuran; ethyl acetate; or dimethylformamide.

For the reaction, the carbamic acid chloride is used usually in an amount of from 0.8 to 3 mols, preferably from 1 to 2 mols, per mol of the phenoxyamine. The two reactants and the base are mixed without a solvent or in the above-mentioned solvent, and then stirred at a temperature of from 0° to 100°C, preferably from 0 to 60°C for from 0.5 to 30 hours.

After the reaction, the desired product can be obtained in a conventional method as shown in the Examples given hereinafter.

Process 2

Among the substituted phenoxy ureas of the present invention, those wherein $R_2$ is a hydrogen atom, i.e. those represented by the formula I—II, can be produced by reacting a phenoxyamine of the formula II with an isocyanate of the formula IV. In this case, as specific compounds of the phenoxyamine of the formula II, those used for Process 1, may be mentioned. Further, the specific examples of the alkyl group, etc. for $R_1$ of the formula IV may be the same.

For the reaction, in addition to a tertiary amine such as trimethylamine, a Lewis acid such as $AlCl_3$ or $ZnCl_2$ may be used in an amount of from 0.1 to 10 mol% relative to the phenoxyamine of the formula II in reaction solvent listed in Process 1, and the desired compound can be obtained in the same manner as in Process 1 except that an isocyanate of the formula IV is used in an amount of from 0.8 to 2 in a molar relative to the phenoxyamine of the formula II.

Process 3

The substituted phenoxy ureas of the formula I—II of the present invention can be produced also by reacting a phenoxycarbamate of the formula V with an amine of the formula VI.

As the aryl moiety of the formula:

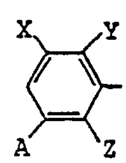

of the compound of the formula V, there may be mentioned those listed above as the aryl moiety in the

formula I, and as Ar, there may be mentioned a phenyl group, a chlorophenyl group or a tolyl group. Further, the phenoxy carbamate of the formula V can be produced from a phenoxyamine of the formula II and ArOCOCl by using a base and solvent as mentioned in Process 1.

As the alkyl group, etc. for $R_1$ of the amine of the formula VI, those listed above with respect to the formula I may be mentioned.

The reaction can be conducted at a temperature of from 0 to 100°C, preferably from 0 to 80°C for 0.5 to 5 hours by using from 0.8 to 5 mols of the amine of the formula VI relative to one mol of the phenoxy carbamate of the formula V. In this case, the same solvents as mentioned as in Process 1 may be used.

Now, the present invention will be described in further detail with reference to Examples. However, it should be understood that the present invention is by no means restricted to those specific Examples.

Example 1

3,(2,5-Dichlorophenoxy)-1,1-dimethyl urea (Compound No. 1)

5.00 g (28.1 mmol) of 2,5-dichlorophenoxyamine was dissolved in 6.8 ml of pyridine, and then 3.02 g (28.1 mmol) of dimethylcarbamoyl chloride was added. The mixture was stirred at a temperature of from 25 to 30°C for 12 hours. After an addition of 200 ml of water, the reaction mixture was extracted with ethyl acetate. The extract was washed with a saturated sodium chloride aqueous solution, and dried over anhydrous magnesium sulfate. Ethyl acetate was distilled off under reduced pressure, and precipitated crystals were washed with hexane, whereby 4.33 g of the desired product was obtained as light yellow crystals (yield: 62%).

Melting point: 119—121°C (decomposed)

Elemental analysis:

C: 43.31%, H: 4.40%, H, 11.22%

Calculated values as $C_9H_{10}Cl_2N_2O_2$

C: 43.40% H: 4.05%, N: 11.25%

Mass spectrum (FD Method)

m/Z 248 (Molecular ion peak)

IR spectrum (KBr tablet, $cm^{-1}$)

3210, 1660, 1578, 1496, 1467, 1376, 1190, 1083, 1067, 900, 852, 802, 770

$^1H$—NMR spectrum ($CDCl_3$ solution, ppm)

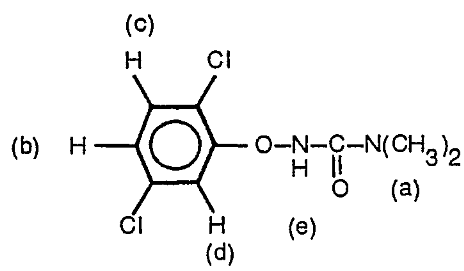

(a) 2.95 (6H, S)
(b) 6.92 (1H, dd J=8, 2Hz)
(c) 7.22 (1H, d, J=8Hz)
(d) 7.31 (1H, d, J=2Hz)
(e) 8.05 (1H, br.s)

Example 2

1-Allyl-3-(2,5-dichlorophenoxy) urea (Compound No. 2)

5.34 g (30.0 mmol) of 2,5-dichlorophenoxyamine was dissolved in 50 ml of toluene, and then 2.74 g (33.0 mmol) of allyl isocyanate was added. The mixture was stirred at a temperature of from 25 to 30°C for 12 hours. The crystals precipitated in the reaction solution were collected by filtration and washed with hexane, whereby 3.96 g of the desired product was obtained as light yellow crystals (yield: 51%).

Melting point: 130—131°C (decomposed)

Mass spectrum (FD Method)

m/Z 260 (Molecular ion peak)

IR spectrum (KBr tablet, cm $^{-1}$)

3320, 3240, 1660, 1640, 1570, 1468, 1253, 1223, 1083, 1067, 995, 915, 898, 868, 802

$^1H$—NMR spectrum ($CDCl_3$ solution, ppm)

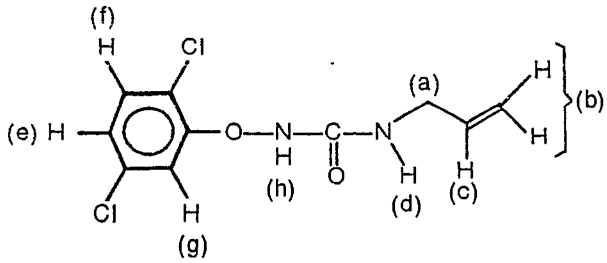

(a) 3.94 (2H, m)
(b) 5.0—5.4 (2H, m)
(c) and (d) 5.7—6.1 (2H, m)
(e) 7.04 (1H, dd, J=8, 2Hz)
(f) 7.30 (1H, d, J=8Hz)
(g) 7.50 (1H, d, J=2Hz)
(h) 8.33 (1H, br.s)

Reference Example 1

N-(2,3-dichlorophenoxy)carbamic acid phenyl ester

9.10 g (51.1 mmol) of 2,3-dichlorophenoxyamine was dissolved in 7.5 ml of pyridine and 70 ml of dichloromethane, and the solution was cooled to —10°C. Then, 30 ml of a dichloromethane solution containing 7.28 g (46.5 mmol) of phenyl chloroformate was dropwise added thereto over a period of 1 hour, and the mixture was stirred at a temperature of from −10 to 25°C for 6 hours. The solvent was distilled off under reduced pressure, and 300 ml of ethyl acetate was added. The mixture was washed with water and a saturated sodium chloride aqueous solution, and dried over anhydrous magnesium sulfate, Ethyl acetate was distilled off under reduced pressure, and the residue thereby obtained was recrystallized from toluene-hexane, whereby 11.5 g of the desired product was obtained as light brown crystals (yield: 83%)

Melting point: 114—115°C (decomposed)
Mass spectrum (FD Method)
m/Z 297 (Molecular ion peak)
IR spectrum (KBr tablet, cm $^{-1}$)
3240, 1736, 1573, 1463, 1265, 1230, 768
$^1$H-NHR spectrum ($CDCl_3$ solution, ppm)

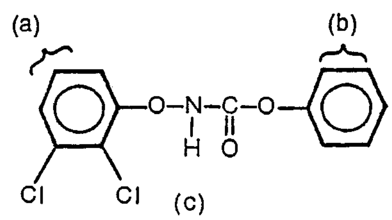

(a) and (b) 7.1—7.5 (8H, m)
(c) 8.36 (1H, br.s)

Example 3

1-Cyclohexyl-3-(2,3-dichlorophenoxy) urea (Compound No. 3)

4.00 g and (13.4 mmol) of N-(2,3-dichlorophenoxy)carbamic acid phenyl ester synthesized in Reference Example 1 was dissolved in 60 ml of ethyl acetate. 3.32 g (33.5 mmol) of cyclohexylamine was added thereto, and the mixture was reacted at 60°C for 12 hours. The reaction mixture was cooled to room temperature, and after an addition of 100 ml of ethyl acetate, it was washed with a 3% sodium hydroxide aqueous solution and then with a saturated sodium chloride solution, and then dried over anhydrous magnesium sulfate. Ethyl acetate was distilled off under reduced pressure, and the residue was recrystallized from ethyl acetate-hexane, whereby 2.84 g of the desired product was obtained as light brown crystals (yield: 70%).

Melting point: 136—138°C (decomposed)
Mass spectrum (FD Method)
m/Z 302 (Molecular ion peak)
IR spectrum (KBr tablet, cm $^{-1}$)
3320, 1650, 1555, 1445, 1425, 1240, 1225, 1185, 765
$^1$NHR spectrum ($CDCl_3$ solution, ppm)

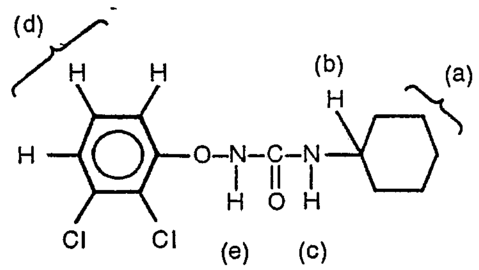

(a) 1.0—2.2 (10H, m)
(b ) 3.70 (1H, m)
(c) 5.72 (1H, m)
(d) 7.1—7.3 (3H, m)
(e) 8.18 (1H, br.s)

Examples 4 to 183

Compound Nos. 4 to 183 listed in Table 1 were synthesized in the same manner as the processes of Examples 1 to 3. The results are shown in Table 2. Processes A, B and C in Table 2 correspond to the processes of Examples 1, 2 and 3, respectively.

Table 2

| Compound No. | Process | Yield (%) | Melting point (℃) | IR spectrum ($cm^{-1}$) | | | $^1H$—NMR spectrum ($CDCl_3$, ppm) |
|---|---|---|---|---|---|---|---|
| | | | | νC=O | νN−H | Other characteristic absorption | |
| 4 | A | 78 | 132–133 (decomposed) | 1663 | 3160 | | |
| 5 | A | 55 | 131–133 (decomposed) | 1670 | 3120 | | |
| 6 | A | 75 | 121–123 (decomposed) | 1660 | 3120 | | |
| 7 | A | 39 | 137–138 (decomposed) | 1657 | 3110 | | |
| 8 | A | 48 | 106–107 | 1660 | 3140 | | |
| 9 | A | 72 | liquid | 1660 | 3210 | 3290(ν≡C-H), 2210(νC≡C) | |
| 10 | A | 50 | 73–74 | 1660 | 3140 | | |
| 11 | A | 70 | 56–57 | 1665 | 3200 | | 1.12(6H, t, J=7Hz), 3.24(4H, q, J=7Hz), 6.8–7.3(4H, m), 8.32(1H, br.s) |

Table 2 (Cont'd)

| | | | | | | | |
|---|---|---|---|---|---|---|---|
| 12 | A | 51 | liquid | 1655 | 3190 | | |
| 13 | A | 43 | liquid | 1660 | 3200 | | 3.92(4H, d, J=5Hz), 5.08-5.50(4H, m), 5.60-6.08(2H, m), 6.90-7.32(4H, m) 7.72(1H, br.s) |
| 14 | A | 46 | 128-129 | 1650 | 3210 | | |
| 15 | A | 78 | 116-117 | 1650 | 3130 | | 1.57(6H, br.s), 3.33(4H, br.s), 6.80-7.28 (4H, m), 8.10(1H, s) |
| 16 | A | 45 | 112-113 | 1650 | 3110 | | |
| 17 | A | 47 | 94-95 | 1650 | 3130 | | |
| 18 | A | 38 | 114-115 | 1655 | 3145 | | |
| 19 | A | 50 | 75-76 | 1649 | 3200 | | 0.87(3H, t, J=8Hz), 1.40-1.60(8H, m), 6.90-7.30(3H, m), 7.75(1H, s) |
| 20 | A | 35 | 91-92 | 1650 | 3170 | | |

Table 2 (Cont'd)

| | | | | | | |
|---|---|---|---|---|---|---|
| 21 | A | 56 | 115-116 | 1653 | 3280 | |
| 22 | A | 77 | 148-149 | 1645 | 3270 | 1.69(8H, m), 2.03(2H, br.s), 3.53(4H, d, J=4Hz), 6.88-7.31(4H, m), 8.08(1H, s) |
| 23 | C | 45 | 106-108 | 1652 | 3300,3250 | |
| 24 | C | 61 | 138-140 (decomposed) | 1650 | 3290,3260 | 1.20-2.20(8H, m), 4.18(1H, m), 5.74(1H, d, J=7Hz), 7.10-7.28(3H, m), 8.00(1H, br.s) |
| 25 | C | 79 | 128-130 (decomposed) | 1658 | 3440,3220 | 1.00-2.00(8H, m), 2.24(2H, br.s), 3.66(1H, dd, J=4, 8Hz), 5.62(1H, d, J=8Hz), 7.14-7.43(3H, m), 8.63(1H, s) |
| 26 | A | 32 | 111-112 | 1650 | 3100 | |
| 27 | A | 34 | 108-109.5 | 1655 | 3100 | 0.89(6H, m), 1.62(4H, m), 3.18(4H, m), 7.06-7.26(3H, m), 7.77(1H, s) |
| 28 | A | 47 | 145-147 | 1657 | 3150 | 1.88(4H, m), 3.38(4H, m), 7.08-7.36(3H, m), 7.89(1H, s) |
| 29 | A | 55 | 130-132 (decomposed) | 1650 | 3185 | |

Table 2 (Cont'd)

| | | | | | | | |
|---|---|---|---|---|---|---|---|
| 30 | A | 45 | 137–139 | 1660 | 3220 | | |
| 31 | B | 69 | 135–136 (decomposed) | 1655 | 3310,3230 | | |
| 32 | B | 45 | 141–142 (decomposed) | 1660 | 3280,3220 | | |
| 33 | B | 67 | 107–108 (decomposed) | 1660 | 3310,3225 | | |
| 34 | B | 62 | 135–136 (decomposed) | 1655 | 3300,3250 | | 1.20(6H, d, J=7Hz), 4.02(1H, m), 5.60(1H, m) 7.02(1H, dd, J=8, 2Hz), 7.28(1H, d, J=8Hz), 7.48(1H, d, J=2Hz), 8.36(1H, br.s) |
| 35 | C | 70 | 131–132 (decomposed) | 1660 | 3290,3260 | | |
| 36 | C | 64 | 151–152 (decomposed) | 1659 | 3290,3240 | 3320(ν≡C–H) | |
| 37 | C | 72 | 135–136 (decomposed) | 1660 | 3300,3100 | | |
| 38 | B | 47 | 99 100 (decomposed) | 1658 | 3300,3240 | | 0.92(3H, m), 1.2–1.8(4H, m), 3.32(2H, m), 5.78 (1H, m), 7.04(1H, dd, J=8, 2Hz), 7.28(1H, d, J=8Hz), 7.50(1H, d, J=2Hz), 8.24(1H, br.s) |

Table 2 (Cont'd)

| | | | | | | | |
|---|---|---|---|---|---|---|---|
| 39 | C | 69 | 130-131 (decomposed) | 1657 | 3300, 3250 | | |
| 40 | C | 85 | 131–132 (decomposed) | 1663 | 3350, 3250 | | |
| 41 | C | 69 | 113–115 (decomposed) | 1665 | 3420, 3080 | | 1.39 (9H, s), 5.73 (1H, s), 7.00 (1H, dd, J=8, 2Hz), 7.30 (1H, d, J=8Hz), 7.47 (1H, d, J=2Hz), 7.93 (1H, s) |
| 42 | C | 65 | 120–121 (decomposed) | 1655 | 3280, 3080 | | |
| 43 | C | 69 | 122–123 | 1645 | 3250, 3090 | | |
| 44 | C | 71 | 132–133 (decomposed) | 1655 | 3290, 3090 | | |
| 45 | C | 55 | 83–85 (decomposed) | 1678 | 3435, 3180 | | |
| 46 | C | 43 | 125–126 (decomposed) | 1670 | 3420, 3170 | 3280 ($\nu$≡C–H), 2140 ($\nu$C≡C) | |
| 47 | C | 79 | 139–140 (decomposed) | 1650 | 3300, 3100 | | |

Table 2 (Cont'd)

| | | | | | | | |
|---|---|---|---|---|---|---|---|
| 48 | C | 80 | 136-138 (decomposed) | 1652 | 3410,3160 | | |
| 49 | C | 53 | 130-132 (decomposed) | 1650 | 3300,3200 | | |
| 50 | C | 70 | 141.5-142.5 (decomposed) | 1665 | 3420,3100 | | |
| 51 | C | 33 | 133-134 (decomposed) | 1655 | 3300,3250 | | |
| 52 | B | 30 | 149-150.5 (decomposed) | 1650 | 3300,3240 | | |
| 53 | C | 58 | 138-139 (decomposed) | 1657 | 3320,3230 | | |
| 54 | C | 43 | 158-159 (decomposed) | 1665 | 3420,3150 | | |
| 55 | C | 58 | 150-152 (decomposed) | 1670 | 3420,3160 | | |
| 56 | C | 86 | 231-233.5 (decomposed) | 1668 | 3430,3140 | | 1.00-2.00(8H, m), 2.25(2H, br.s), 3.66(1H, dt, J=4, 8Hz), 5.64(1H, d, J=8Hz), 7.00(1H, dd, J=8, 2Hz), 7.30(1H, d, J=8Hz), 7.44(1H, d, J=2Hz), 8.10(1H, br.s) |

Table 2 (Cont'd)

| | | | | | | | |
|---|---|---|---|---|---|---|---|
| 57 | C | 82 | 146-147 (decomposed) | 1669 | 3415,3140 | | |
| 58 | C | 74 | 161-163 (decomposed) | 1673 | 3415,3140 | | |
| 59 | A | 51 | 113-114 (decomposed) | 1663 | 3100 | | |
| 60 | A | 53 | 128-129 (decomposed) | 1655 | 3100 | | |
| 61 | A | 66 | 102-103 | 1655 | 3100 | | |
| 62 | A | 42 | 133-134 (decomposed) | 1650 | 3090 | | |
| 63 | A | 65 | 105-106 | 1660 | 3110 | 1640(νC=C) | |
| 64 | A | 88 | 70-72 (decomposed) | 1663 | 3200 | 3280(ν≡C-H) 2120(νC≡C) | 2.33(1H, t, J=2Hz), 3.00(3H, s), 4.13(2H, d, J=2Hz), 6.92(1H, dd, J=8, 2Hz), 7.22(1H, d, J=8Hz), 7.30(1H, d, J=2Hz), 8.26(1H, br. s) |
| 65 | A | 74 | 116-117 | 1660 | 3130 | | |

Table 2 (Cont'd)

| | | | | | | | |
|---|---|---|---|---|---|---|---|
| 66 | A | 64 | 90.5-92 (decomposed) | 1658 | 3120 | | |
| 67 | A | 34 | 124-125 (decomposed) | 1645 | 3130 | | |
| 68 | A | 72 | 117-118 | 1665 | 3180 | | |
| 69 | A | 46 | 114-115 | 1649 | 3120 | | |
| 70 | A | 87 | 122-123 (decomposed) | 1645 | 3110 | 3280($\nu \equiv C-H$) | |
| 71 | A | 68 | 111-112 | 1660 | 3190 | | |
| 72 | A | 50 | 121-122 (decomposed) | 1660 | 3220 | | |
| 73 | A | 47 | 106-107 | 1659 | 3180 | | |
| 74 | A | 62 | 113-114 (decomposed) | 1662 | 3220 | | |

Table 2 (Cont'd)

| | | | | | | | |
|---|---|---|---|---|---|---|---|
| 75 | A | 38 | 120-121 | 1659 | 3220 | | |
| 76 | A | 48 | 104-105 | 1655 | 3110 | | |
| 77 | A | 29 | 135-137 (decomposed) | 1645 | 3130 | | |
| 78 | A | 33 | 99-100 | 1668 | 3140 | | |
| 79 | A | 37 | 101-102 (decomposed) | 1665 | 3210 | | |
| 80 | A | 74 | 70-71 | 1655 | 3110 | 1640($\nu$C=C) | 3.92(4H, d, J=7Hz), 5.20(2H, m), 5.32(2H, m), 5.8(2H, m), 6.96(1H, dd, J=8, 2Hz), 7.22(1H, d, J=8Hz), 7.28(1H, d, J=2Hz), 7.83(1H, br. s) |
| 81 | A | 46 | 122-123 (decomposed) | 1650 | 3120 | 3290($\nu$≡C-H)<br>2120($\nu$C≡C) | |
| 82 | A | 69 | 131-132 (decomposed) | 1645 | 3120 | | |
| 83 | A | 50 | 111-113 (decomposed) | 1645 | 3140 | | |

Table 2 (Cont'd)

| | | | | | | | |
|---|---|---|---|---|---|---|---|
| 84 | A | 48 | 135-136 (decomposed) | 1650 | 3140 | | |
| 85 | A | 20 | 120-122 (decomposed) | 1645 | 3180 | | |
| 86 | A | 60 | 144-145 (decomposed) | 1660 | 3180 | | |
| 87 | A | 35 | 131.5-133.5 (decomposed) | 1650 | 3140 | | |
| 88 | A | 22 | 136-137 (decomposed) | 1650 | 3140 | | |
| 89 | A | 54 | 135-136 (decomposed) | 1660 | 3200 | | 3.53(2H, t, J=6Hz), 4.05(2H, t, J=6Hz), 4.92(2H, s), 7.00(1H, dd, J=8, 2Hz), 7.28(1H, d, J=8Hz), 7.44(1H, d, J=2Hz), 7.80(1H, br.s) |
| 90 | A | 55 | 139-140 (decomposed) | 1660 | 3120 | | |
| 91 | A | 75 | 140-141 (decomposed) | 1660 | 3090 | 1642(νC=C) | 2.17(2H, m), 3.50(2H, t, J=6Hz), 3.87(2H, m), 5.64(1H, m), 5.84(1H, m), 6.95(1H, dd, J=8, 2Hz), 7.23(1H, d, J=8Hz), 7.34(1H, d, J=2Hz), 8.32(1H, s) |
| 92 | A | 47 | 123-124 (decomposed) | 1652 | 3100 | | 1.22(3H, d, J=7Hz), 1.62(6H, m), 3.00(1H, m), 3.76(1H, m), 4.28(1H, m), 6.94(1H, dd, J=8, 2Hz), 7.24(1H, d, J=8Hz), 7.34(1H, d, J=2Hz), 7.90(1H, s) |

Table 2 (Cont'd)

| | | | | | | | |
|---|---|---|---|---|---|---|---|
| 93 | A | 76 | 135-136 (decomposed) | 1655 | 3120 | | |
| 94 | A | 79 | 129-130 (decomposed) | 1654 | 3100 | | |
| 95 | A | 57 | 126-127 (decomposed) | 1645 | 3120 | | |
| 96 | A | 27 | 118-120 (decomposed) | 1640 | 3100 | | |
| 97 | A | 66 | 129-130 (decomposed) | 1652 | 3120 | | |
| 98 | A | 49 | 141-142 (decomposed) | 1660 | 3200 | | 1.80(4H, m), 3.72(2H, m), 4.05(2H, m), 6.96 (1H, dd, J=8, 2Hz), 7.24(1H, d, J=8Hz), 7.37 (1H, d, J=2Hz), 8.64(1H, br.s) |
| 99 | A | 73 | 117-118 (decomposed) | 1660 | 3250 | | |
| 100 | A | 52 | 140-141 (decomposed) | 1650 | 3120 | | 3.44(4H, m), 3.68(4H, m), 6.96(1H, dd, J=8, 2Hz), 7.22(1H, d, J=8Hz), 7.30(1H, d, J=2Hz), 7.72(1H, br.s) |
| 101 | A | 72 | 124-125 (decomposed) | 1645 | 3120 | | |

Table 2 (Cont'd)

| | | | | | | | |
|---|---|---|---|---|---|---|---|
| 102 | A | 40 | 131–132 (decomposed) | 1655 | 3100 | | 1.65(8H, m), 3.44(4H, m) 6.92(1H, dd, J=8, 2Hz), 7.22(1H, d, J=8Hz), 7.32(1H, d, J=2Hz), 7.76(1H, br.s) |
| 103 | A | 48 | 152–154 (decomposed) | 1632 | 3250 | | |
| 104 | A | 30 | 129–130.5 (decomposed) | 1645 | 3100 | | |
| 105 | A | 33 | 156–157 (decomposed) | 1655 | 3250 | | |
| 106 | A | 26 | 145–146 (decomposed) | 1656 | 3180 | | |
| 107 | B | 53 | 163–164 (decomposed) | 1635 | 3360, 3170 | | |
| 108 | C | 56 | 115–116 | 1670 | 3360, 3230 | | |
| 109 | C | 71 | 107–108 | 1670 | 3370, 3160 | | |
| 110 | C | 56 | 138–139.5 (decomposed) | 1670 | 3360, 3220 | 3270(ν≡C–H) 2100(νC≡C) | 1.67(6H, s), 2.37(1H, s), 5.60(1H, br.s), 7.10(3H, m), 7.80(1H, br.s) |

Table 2 (Cont'd)

| | | | | | | | |
|---|---|---|---|---|---|---|---|
| 111 | C | 90 | 160-161 (decomposed) | 1639 | 3350,3190 | | 1.20—2.20(8H, m), 4.18(1H, m), 5.38(1H, d, J=7Hz), 7.10(3H, m), 8.08(1H, br.s) |
| 112 | C | 69 | 146-148 (decomposed) | 1635 | 3340,3180 | | |
| 113 | A | 58 | 133.5-135 | 1655 | 3120 | | 0.90(3H, t, J=7Hz), 1.58(2H, m), 2.92(3H, s), 3.22(2H, t, J=7Hz), 7.01(3H, s), 7.93(1H, br.s) |
| 114 | A | 56 | 121-122 | 1660 | 3100 | | |
| 115 | A | 88 | 115-116 | 1655 | 3090 | | |
| 116 | A | 70 | 95-96 | 1660 | 3120 | 3290(ν≡C-H), 2110(νC≡C) | |
| 117 | A | 83 | 115-116 | 1655 | 3120 | | |
| 118 | A | 89 | 87-88 | 1660 | 3130 | 1645(νC=C) | |
| 119 | A | 79 | 97-98 | 1665 | 3120 | | |

Table 2 (Cont'd)

| | | | | | | | |
|---|---|---|---|---|---|---|---|
| 120 | A | 60 | 114-115 | 1655 | 3110 | | |
| 121 | A | 81 | 71-72 | 1650 | 3140 | | |
| 122 | A | 51 | 97-98 | 1657 | 3120 | | |
| 123 | A | 83 | 98-99 | 1665 | 3100 | | |
| 124 | A | 93 | 122-123 (decomposed) | 1650 | 3120 | 3260($\nu \equiv C-H$) | |
| 125 | A | 68 | 117-118 | 1660 | 3150 | | |
| 126 | A | 92 | liquid | 1660 | 3150 | | |
| 127 | A | 52 | 96-97 | 1660 | 3150 | | |
| 128 | A | 51 | 111-112 | 1650 | 3100 | | |

Table 2 (Cont'd)

| | | | | | | | |
|---|---|---|---|---|---|---|---|
| 129 | A | 50 | 98.5–100.5 | 1657 | 3130 | | |
| 130 | A | 42 | 121–123 | 1648 | 3100 | | |
| 131 | A | 48 | 101–102 | 1655 | 3120 | | |
| 132 | A | 79 | 84–86 | 1658 | 3155 | | 3.90(4H, d, J=5Hz), 5.02–5.40(4H, m), 5.52–6.02(2H, m), 7.00(3H, s), 8.10(1H, s) |
| 133 | A | 80 | 133–134 (decomposed) | 1650 | 3150 | 3290(ν≡C–H), 2110(νC≡C) | |
| 134 | A | 74 | 140–141 | 1665 | 3210 | | |
| 135 | A | 77 | 157–158 | 1655 | 3140 | | |
| 136 | A | 70 | 138–140 | 1662 | 3190 | | |
| 137 | A | 85 | 107–108 | 1650 | 3150 | | |

Table 2 (Cont'd)

| | | | | | | | |
|---|---|---|---|---|---|---|---|
| 138 | A | 50 | 129–130.5 | 1650 | 3120 | | |
| 139 | A | 92 | 138.5–140.5 | 1650 | 3125 | | |
| 140 | A | 83 | 133–134 | 1660 | 3250 | | |
| 141 | A | 65 | 146–148 | 1645 | 3120 | | |
| 142 | A | 53 | 117–119 | 1650 | 3140 | | |
| 143 | A | 79 | 138–141 | 1653 | 3140 | | |
| 144 | A | 56 | 118–121 | 1655 | 3118 | | 0.88(3H, t, J=7Hz), 1.20–1.90(8H, m), 3.76 (2H, m), 3.96(1H, m), 6.99(3H, s), 8.00(1H, br.s) |
| 145 | A | 91 | 127–128 | 1650 | 3130 | | |
| 146 | A | 44 | 125–126 | 1658 | 3230 | | 1.60(8H, m), 3.38(4H, m), 6.98(3H, m), 8.30 (1H, br.s) |

Table 2 (Cont'd)

| | | | | | | | |
|---|---|---|---|---|---|---|---|
| 147 | A | 54 | 161–162 | 1653 | 3215 | | |
| 148 | A | 39 | 152–153 (decomposed) | 1650 | 3240 | | |
| 149 | A | 40 | 172–173 (decomposed) | 1658 | 3170 | | |
| 150 | C | 59 | 103–104 | 1650 | 3300, 3250 | | |
| 151 | A | 52 | 89–90 | 1655 | 3220 | | |
| 152 | A | 61 | 75–76 | 1660 | 3140 | | |
| 153 | A | 66 | 95–96 | 1656 | 3160 | 3295($\nu \equiv C-H$), 2115($\nu C \equiv C$) | |
| 154 | A | 62 | 66–67 | 1661 | 3200 | | |
| 155 | A | 58 | 70–71 | 1660 | 3210 | 1639($\nu C=C$) | |

| | | | | | | | |
|---|---|---|---|---|---|---|---|
| 156 | A | 64 | 76−77 | 1660 | 3140 | | |
| 157 | A | 43 | 75−76 | 1660 | 3180 | | |
| 158 | A | 61 | 73−74 | 1665 | 3190 | | |
| 159 | A | 48 | 81−82 | 1657 | 3230 | | |
| 160 | A | 59 | 69−70 | 1665 | 3200 | 1650(νC=C) | |
| 161 | A | 47 | 90−91 | 1660 | 3250 | 3290(ν≡C−H) | |
| 162 | A | 66 | 127−128 | 1650 | 3210 | | |
| 163 | A | 79 | 92−93 | 1660 | 3190 | | |
| 164 | A | 60 | 109−110 | 1655 | 3180 | | |

Table 2 (Cont'd)

| | | | | | | | |
|---|---|---|---|---|---|---|---|
| 165 | A | 60 | 76-77 | 1656 | 3180 | | |
| 166 | A | 63 | 95-96 | 1660 | 3140 | | |
| 167 | A | 50 | 58-60 | 1650 | 3140 | | 1.12(6H, t, J=7Hz), 3.24(4H, q, J=7Hz), 7.26(4H, m), 8.28(1H, br.s) |
| 168 | A | 50 | liquid | 1660 | 3200 | | 0.87(3H, t, J=8Hz), 1.15(3H, t, J=7Hz), 1.40-1.88(2H, m), 3.26(2H, q, J=8Hz), 3.34(2H, q, J=7Hz), 7.27(4H, m), 8.00(1H, br.s) |
| 169 | A | 68 | 118-119 | 1660 | 3190 | | |
| 170 | A | 43 | 122-123 | 1655 | 3220 | | |
| 171 | A | 70 | 111-112 | 1660 | 3150 | | |
| 172 | A | 54 | 79-80.5 | 1650 | 3140 | | |
| 173 | A | 57 | 88-91 | 1655 | 3120 | | 0.97(3H, d, J=7Hz), 1.10-1.96(5H, m), 2.33-3.00(2H, m), 3.83(2H, m), 7.32(4H, m), 7.96(1H, br.s) |

| | | | | | | | |
|---|---|---|---|---|---|---|---|
| 174 | A | 40 | 107–109 | 1656 | 3260 | | |
| 175 | A | 45 | 126–127 | 1640 | 3160 | | |
| 176 | A | 45 | 111–113 | 1645 | 3160 | | |
| 177 | A | 57 | 103–104 | 1650 | 3210 | | |
| 178 | A | 54 | 121–122 | 1660 | 3240 | | |
| 179 | A | 50 | 100.5–101.5 | 1656 | 3280 | | |
| 180 | A | 54 | 154–155 | 1650 | 3270 | | |
| 181 | A | 43 | 150–151 (decomposed) | 1650 | 3300 | | |
| 182 | C | 80 | 125–126 | 1665 | 3300,3090 | | |
| 183 | A | 30 | liquid | 1655 | 3170 | | |

Example 184

3-(3,5-Dichlorophenoxy)-1,1-dimethyl urea trichloroacetate (Compound No. 184)

2.25 g (9 mmol) of 3-(3,5-dichlorophenoxy)-1,1-dimethyl urea was dissolved in 300 ml of diethyl ether, and then 50 ml of a diethyl ether solution containing 1.47 g (9 mmol) of trichloroacetic acid was added. The mixture was stirred at a temperature of from 25 to 30°C for 24 hours. Diethyl ether was distilled off and 100 ml of hexane was added. The crystals thereby precipitated were collected by filtration and dried, whereby 3.52 g of the desired product was obtained as colorless acicular crystals (yield: 95%).

Melting point: 120—121°C

Elemental analysis:

C: 32.43%, H: 2.76%, H, 6.74%

Calculated values as $C_{11}H_{11}Cl_5N_2O_4$

C: 32.03%, H: 2.69%, N: 6.79%

IR spectrum (KBr tablet, $cm^{-1}$)

3180, 2680—2000 (broad), 1755, 1620, 1580, 1503, 1395, 1250, 1095, 1070, 1017, 920, 855, 842, 832, 778, 703, 678, 672

$^1$H-NMR spectrum ($CDCl_3$ solution, ppm)

3.00 (6H, s), 7.03 (3H, s), 8.33 (2H, br. s).

Example 185

3-(2,5-Dichlorophenoxy)-1,1-dimethyl urea trichloroacetate (Compound No. 185)

3-(2,5-Dichlorophenoxy)-1,1-dimethyl urea was synthesized in the same manner as in Example 184 except that 3-(2,5-dichlorophenoxy)-1,1-dimethyl urea was used instead of 3-(3,5-dichlorophenoxy)-1,1-dimethyl urea (yield: 54%).

Colorless acicular crystals

Melting point: 110—112°C (Decomposed)

Elemental analysis:

C: 32.42%, H: 2.75%, H, 6.68%

Calculated values as $C_{11}H_{11}Cl_5N_2O_4$

C: 32.03%, H: 2.69%, N: 6.79%

IR spectrum (KBr tablet, $cm^{-1}$)

3160, 2680—2000 (broad), 1760, 1628, 1580, 1505, 1470, 1394, 1257, 1240, 1095, 900, 866, 833, 815, 703, 677

$^1$H-NMR spectrum ($CDCl_3$ solution, ppm)

3.00 (6H, s), 7.00 (1H, dd, J=8, 2Hz), 7.24 (1H, d, J=8Hz), 7.36 (1H, d, J=2Hz), 8.52 (2H, br. s).

Example 186

3-(3,5-Dichlorophenoxy)-1-isopropyl-1-methyl urea trichloroacetate (Compound No. 186)

3-(3,5-Dichlorophenoxy)-1-isopropyl-1-methyl urea was synthesized in the same manner as in Example 184 except that 3-(3,5-dichlorophenoxy)-1-isopropyl-1-methyl urea was used instead of 3-(3,5-dichlorophenoxy)-1,1-dimethyl urea (yield: 57%).

Slightly yellow powder

Melting point: 74—75°C

IR spectrum (KBr tablet, $cm^{-1}$)

3310, 3290, 3050, 2950, 1727, 1579, 1495, 1428, 1387, 1264, 1091, 843, 827, 702, 675.

Examples 187 to 241, 252 and 253

Compound Nos. 187 to 241 listed in Table 1 were synthesized in the same manner as the processes of Examples 1 to 3. The results are shown in Table 3. Processes A, B and C in Table 3 correspond to the processes of Examples 1, 2 and 3, respectively.

Table 3

| Compound No. | Process | Yield (%) | Melting point (°C) | IR spectrum ($cm^{-1}$) νC=O | νN-H | Other characteristic absorption | $^1$H-NMR spectrum ($CDCl_3$, ppm) |
|---|---|---|---|---|---|---|---|
| 187 | A | 65 | 91-93 | 1652 | 3130 | | |
| 188 | A | 41 | 112-114 | 1661 | 3200 | | |
| 189 | A | 32 | 136-139 (decomposed) | 1660 | 3210 | | |
| 190 | A | 40 | 105-107 | 1660 | 3180 | | |
| 191 | A | 22 | 122-124 (decomposed) | 1655 | 3210 | | |
| 192 | A | 41 | 145-143 (decomposed) | 1655 | 3240 | | |
| 193 | A | 39 | 138-140 (decomposed) | 1662 | 3140 | | |
| 194 | A | 52 | 132-133 (decomposed) | 1650 | 3130 | | |
| 195 | A | 28 | 128-130 (decomposed) | 1655 | 3200 | | |
| 196 | A | 70 | 116-117 (decomposed) | 1652 | 3130 | | |
| 197 | A | 26 | 120-122 (decomposed) | 1650 | 3100 | | |
| 198 | A | 64 | 162-163 (decomposed) | 1650 | 3180 | | |
| 199 | A | 74 | 101-103 | 1655 | 3120 | | |
| 200 | A | 57 | 93-94 | 1655 | 3130 | | |
| 201 | A | 40 | 77-78 | 1660 | 3120 | | |

Table 3 (cont'd)

| | | | | | | | |
|---|---|---|---|---|---|---|---|
| 202 | A | 53 | 143-144 | 1650 | 3130 | | |
| 203 | A | 54 | 123-124 | 1655 | 3120 | | |
| 204 | A | 61 | 83-85 | 1652 | 3120 | | |
| 205 | A | 52 | 95-96 | 1660 | 3150 | | |
| 206 | A | 67 | 168-170 (decomposed) | 1660 | 3130 | | |
| 207 | A | 78 | 137-138 | 1660 | 3140 | | |
| 208 | A | 70 | 159-161 | 1655 | 3270 | | 1.00 (6H, s), 1.35 (4H, m) 3.38 (4H, m), 6.93-7.08 (3H, m), 9.58 (1H, s) |
| 209 | A | 81 | 95-96 | 1650 | 3130 | | |
| 210 | A | 75 | 134-135 | 1655 | 3140 | | |
| 211 | A | 62 | 50-52 | 1655 | 3170 | | |
| 212 | A | 40 | 123-124 | 1656 | 3190 | | |
| 213 | A | 49 | 104-106 | 1655 | 3120 | | |
| 214 | A | 50 | 101-105 | 1652 | 3120 | | 0.8-2.3 (12H, m), 2.7-3.0 (1H; m), 3.3-3.75 (3H, m), 7.06 (3H, s), 7.81 (1H, s) |
| 215 | A | 51 | 141-142 | 1655 | 3140 | | |
| 216 | A | 46 | 113-114 | 1650 | 3130 | | |
| 217 | A | 44 | 134-136 | 1655 | 3140 | | |
| 218 | A | 72 | 144-146 | 1675 | 3220 | | |
| 219 | A | 60 | 167-168 (decomposed) | 1650 | 3120 | | 1.3-1.9 (8H, m), 4.2-4.4 (2H, m), 7.0-7.1 (2H, m), 7.1-7.2 (1H, m), 7.8 (1H, s) |
| 220 | A | 47 | 168-170 (decomposed) | 1660 | 3190 | | |

Table 3 (cont'd)

| | | | | | | | |
|---|---|---|---|---|---|---|---|
| 221 | A | 56 | 154-155 | 1665 | 3200 | | |
| 222 | A | 31 | 159-161 (decomposed) | 1655 | 3200 | | 1.4-2.2 (9H, m), 2.40 (2H, d, J= 2Hz), 4.08 (1H, m), 7.1 (3H, m), 7.45 (1H, s) |
| 223 | A | 64 | 163-165 (decomposed) | 1655 | 3190 | | |
| 224 | A | 60 | 118-119 | 1653 | 3130 | | |
| 225 | A | 60 | 53-55 | 1655 | 3150 | | |
| 226 | A | 85 | liquid | 1658 | 3190 | | |
| 227 | A | 62 | 103-104 | 1650 | 3230 | | |
| 228 | A | 40 | 78-79 | 1660 | 3180 | | |
| 229 | A | 38 | 67-68 | 1655 | 3150 | | |
| 230 | A | 71 | 154-156 | 1660 | 3140 | | |
| 231 | A | 47 | 106-107 | 1655 | 3140 | | |
| 232 | A | 73 | 114-115 | 1645 | 3130 | | |
| 233 | A | 65 | 97-98 | 1655 | 3130 | | |
| 234 | A | 73 | 153-155 (decomposed) | 1650 | 3250 | | |
| 235 | A | 53 | 89-90 | 1653 | 3270 | | |
| 236 | A | 79 | 100-101 | 1675 | 3280 | | |
| 237 | A | 64 | 145-147 (decomposed) | 1645 | 3200 | | |
| 238 | A | 72 | 159-162 (decomposed) | 1660 | 3220 | | |
| 239 | A | 43 | 163-164 (decomposed) | 1655 | 3230 | | |

Table 3 (cont'd)

| | | | | | | | |
|---|---|---|---|---|---|---|---|
| 240 | A | 86 | 154-156 (decomposed) | 1650 | 3260 | | |
| 241 | A | 40 | 124-125 | 1665 | 3130 | | |
| 252 | C | 75 | 122-125 (decomposed) | 1650 | 3340, 3190 | | |
| 253 | C | 77 | 138-140 (decomposed) | 1660 | 3290, 3200 | | |

## Example 242

3-(2,5-Dichlorophenoxy)-1-methoxy-1-methyl urea (Compound No. 242)

4.5 g (25 mmol) of 2,5-dichlorophenoxyamine was dissolved in 10 ml of pyridine, and then 5.6 g (38 mmol) of methoxymethylcarbamoyl chloride was added. The mixture was stirred at a temperature of from 25 to 30°C for 12 hours. After an addition of 200 ml of water, the reaction mixture was extracted with ethyl acetate. The extract was washed with a saturated sodium chloride aqueous solution, and dried over anhydrous magnesium sulfate. Ethyl acetate was distilled off, and precipitated crystals were recrystallized from ethyl acetate-hexane, whereby 4.8 g of the desired product was obtained (yield: 72%).

White powder
Melting point: 119—120°C (decomposed)
Mass spectrum (FD Method)
m/Z 264 (Molecular ion peak)
IR spectrum (KBr tablet, $cm^{-1}$)
3200 ($\nu_{N-H}$), 1685 ($\nu_{C=O}$)
$^1$H-NMR spectrum ($CDCl_3$ solution, ppm)
3.18 (3H, s), 3.76 (3H, s), 6.84 (1H, dd, J=2, 8Hz), 7.16—7.36 (2H, m), 8.76 (1H, br. s).

## Example 243

3-(2,5-Dichlorophenoxy)-1-methoxyethyl urea (Compound No. 243)

4.5 g (25 mmol) of 2,5-dichlorophenoxyamine was dissolved in 30 ml of toluene, and then 70 ml of an ethyl acetate solution containing 3.8 g (38 mmol) of 2-methoxyethyl cyanate was added. The mixture was stirred at a temperature of from 20 to 30°C for 12 hours. After distilling off the solvent under reduced pressure, the mixture was purified by silica gel column chromatography (developer: ethyl acetate/hexane = 1/4), and recrystallized from ethyl acetate-hexane, whereby 3.2 g of the desired product was obtained (yield: 48%).

Slightly yellow powder
Melting point: 116—118°C (decomposed)
IR spectrum (KBr tablet, $cm^{-1}$)
3360 ($\nu_{N-H}$), 3160 ($\nu_{N-H}$), 1670 ($\nu_{C=O}$).

## Examples 244 to 251

Compound Nos. 244 to 251 listed in Table 1 were synthesized in the same manner as in Example 242. The results are shown in Table 4.

Table 4

| Compound No. | Yield (%) | Melting point (°C) | IR spectrum ($cm^{-1}$) | |
|---|---|---|---|---|
| | | | νN-H | νC=O |
| 244 | 63 | 98-99 | 3290 | 1702 |
| 245 | 34 | 73-75 | 3220 | 1680 |
| 246 | 53 | 111-113 (decomposed) | 3200 | 1665 |
| 247 | 42 | 82-84 | 3070 | 1653 |
| 248 | 36 | 75-76 | 3140 | 1653 |
| 249 | 77 | 103-104 | 3140 | 1655 |
| 250 | 94 | liquid | 3190 | 1660 |
| 251 | 79 | 78-79 | 3230 | 1660 |

The herbicide of the present invention comprises the compound of the formula I as the active ingredient. The compound of the formula I may be used by itself as a herbicide. However, better results can be obtained if it is used in the form of appropriate formulations such as granules, wettable powder, emulsifiable concentrates, dusts or fine granules.

The herbicides of such various formulations may be prepared by combining the compound of the formula I appropriately with a solid carrier such as talc, bentonite, clay, calcite, kaolin, diatomaceous earth, white carbon, vermiculite, lime, siliceous sand, ammonium sulfate or urea, or a liquid carrier such as xylene, kerosine, ethylene glycol, ethyl cellosolve, methyl ethyl ketone, alcohols, dioxane, acetone, cyclohexanone, methyl naphtalene or dimethylformamide, and an emulsifier or dispersing agent such as an alkyl sulfate, an alkyl aryl sulfonate, a polyoxyethylene glycol ether, a polyoxyethylene alkyl aryl ether or a polyoxyethylene sorbitan monoalkylate, and other adjuvants such as carboxymethyl cellulose or gum arabic.

The amount of the active ingredient is usually from 0.5 to 20% by weight in the case of granules, and from 5 to 70% by weight in the case of an emulsifiable concentrate or wettable powder, although it may be varied as the case requires.

The herbicide of the present invention may be applied as it is, or after being diluted with or suspended in e.g. water, in an amount effective to provide herbicidal activities. The dose of the active ingredient of the compound of the present invention varies depending upon the soil condition, the formulation, the season of the application and the method of application and the types of crop plants and the types of weeds. It is usually in a range of 10 g to 5 kg per 1 hectare.

Other herbicides or fertilizers can be incorporated in the herbicidal composition of the present invention, if necessary.

Now, specific formulations will be given in the following Formulation Examples. However, it should be understood that the types of the additives and the proportions of various ingredients are not restricted to these specific Examples and may optionally be modified in a wide range. In these Examples, the "%" represents "% by weight", and likewise "parts" represents "parts by weight".

Formulation Example 1: Granules

10% of the compound of the present invention, 2% of sodium lauryl alcohol sulfate, 5% of sodium lignin sulfonate, 2% of carboxymethyl cellulose and 81% of clay were uniformly mixed and pulverized.

To 80 parts of this mixture, 20 parts of water was added. The mixture was kneaded, then formed into granules having a size of from 14 to 32 meshes, by an extrusion granulating machine, and dried to obtain granules.

Formulation Example 2: Granules

2% of sodium lauryl alcohol sulfate, 5% of sodium lignin sulfonate, 2% of carboxymethyl cellulose and 91% of a clay-montmorillonite mixture were uniformly mixed and pulverized. To 78 parts of this mixture, 22 parts of water was added. The mixture was kneaded, then formed into granules having a size of from 14 to 32 meshes, by an extrusion granulating machine, and dried to obtain an adsorbent. On 80 parts of the adsorbent, 20 parts of a mixture obtained by mixing and dissolving 20% of the compound of the present invention in 80% of polyethylene glycol, was uniformly adsorbed to obtain granules.

Formulation Example 3: Wettable powder

10% of the compound of the present invention, 85% of diatomaceous earth, 2% of sodium dinaphthylmethane disulfonate and 3% of sodium lignin sulfonate were uniformly mixed and pulverized to obtain a wettable powder.

Formulation Example 4: Emulsifiable concentrate

30% of the compound of the present invention, 20% of cyclohexane, 11% of polyoxyethylene alkyl aryl ether, 4% of calcium alkylbenzene sulfonate and 35% of methylnaphthalene were uniformly dissolved to obtain an emulsifiable concentrate.

Formulation Example 5: Dust

4% of the compound of the present invention, 5% of diatomaceous earth and 91% of clay were uniformly mixed and pulverized to obtain a dust.

As compared with commercially available other herbicides, the herbicide of the present invention exhibits superior herbicidal effects not only against annual plants such as barnyardgrass, smallflower flatsedge (*Cyperus difformis* L.), monochoria (*Monochoria vaginalis* Presl), *Rotala indica* Koehne, common false pimpernel (*Lindernie pyxidaria* L.) or *Dopatrium Junceum* Hamilt., but also against perennial plants such as Japanese bulrush (*Scirpus juncoides* Roxb.), spikerush (*Eleocharis acicularis* R. *var lengiseta* Sven.), Cyperus *serotinus Rottb.* or *Alisma canaliculatam* A.Br. when applied during the emergence stage and growing period.

On the other hand, the herbicide of the present invention exhibits no substantial phytotoxicity to useful crop plants, particularly paddy field rice even at a high dose, and it has an extremely high selectivity. Further, the herbicide of the present invention exhibits high herbicidal effects, by soil treatment or foliage treatment, against *Gramineae* weeds such as common barnyardgrass, crabgrass (*Digitaria sanguinalis* L.), green foxtail (*Setaria viridis* L.), annual meadowgrass (*Poa annua* L.) or floating foxtail (*Alopeurus geniculatus* L.), Cyperaceae weeds such as rice flatsedge (*Cyperus iria* L.), and broad leaved weeds such as redroot pogweed (*Amaranthus retroflexus* L.) or common lambsquarters (*Chenopodium album* L.), and yet

it has a feature that it is highly safe against main crop plants such as rice, wheat, maize, soybean and cotton. Furthermore, the herbicide of the present invention can be applied to orchards, meadow, lawn and non-cultured grounds.

Now, the effects of the herbicide of the present invention will be described with reference to Test Examples.

Test Example 1

Herbicidal test by water treatment in a paddy field

Into each porcelain pot having a diameter of 10 cm, paddy soil was filled and puddled, and seeds of barnyardgrass (*Echinochloa crus-galli* L.), smallflower flatsedge, monochoria and Japanese bulrush were sown, and water was filled in a depth of 3 cm. One day later, a predetermined amount of the wettable powder prepared in Formulation Example 3 and diluted with water was dropwise applied to the surface of the water (dose of the active ingredient: 4 kg/hectare). Thereafter, they were grown in a green house. Thirty days after the application, the herbicidal effects were evaluated in accordance with the standards as identified in Table 5. The results thereby obtained are shown in Table 6.

Table 5

| | Evaluation standards for herbicidal effects and phytotoxicity |
|---|---|
| 5 | Withered |
| 4.5 | Herbicidal effect (or phytotoxicity) in a range of 90 to 99% |
| 4 | Herbicidal effect (or phytotoxicity) in a range of 80 to 89% |
| 3.5 | Herbicidal effect (or phytotoxicity) in a range of 70 to 79% |
| 3 | Herbicidal effect (or phytotoxicity) in a range of 60 to 69% |
| 2.5 | Herbicidal effect (or phytotoxicity) in a range of 50 to 59% |
| 2 | Herbicidal effect (or phytotoxicity) in a range of 40 to 49% |
| 1.5 | Herbicidal effect (or phytotoxicity) in a range of 30 to 39% |
| 1 | Herbicidal effect (or phytotoxicity) in a range of 20 to 29% |
| 0.5 | Herbicidal effect (or phytotoxicity) in a range of 1 to 19% |
| 0 | No herbicidal effect (or no phytotoxicity) |

Table 6

| Test compound | Herbicidal effects: Barnyard grass | Small flower flatsedge | Monochoria | Japanese bulrush | Test compound | Herbicidal effects: Barnyard grass | Small flower flatsedge | Monochoria | Japanese bulrush |
|---|---|---|---|---|---|---|---|---|---|
| 1 | 5 | 5 | 5 | 5 | 45 | 5 | 5 | 5 | 5 |
| 2 | 5 | 5 | 5 | 5 | 46 | 5 | 5 | 5 | 5 |
| 3 | 5 | 5 | 5 | 5 | 47 | 5 | 5 | 5 | 5 |
| 4 | 5 | 5 | 5 | 5 | 48 | 5 | 5 | 5 | 5 |
| 5 | 5 | 5 | 5 | 5 | 49 | 5 | 5 | 5 | 5 |
| 6 | 5 | 5 | 5 | 5 | 50 | 5 | 5 | 5 | 5 |
| 7 | 5 | 5 | 5 | 5 | 51 | 5 | 5 | 5 | 5 |
| 8 | 5 | 5 | 5 | 5 | 52 | 5 | 5 | 5 | 5 |
| 9 | 5 | 5 | 5 | 5 | 53 | 5 | 5 | 5 | 5 |
| 10 | 5 | 5 | 5 | 5 | 54 | 5 | 5 | 5 | 5 |
| 11 | 5 | 5 | 5 | 5 | 55 | 5 | 5 | 5 | 5 |
| 12 | 5 | 5 | 5 | 5 | 56 | 5 | 5 | 5 | 5 |
| 13 | 5 | 5 | 5 | 5 | 57 | 5 | 5 | 5 | 5 |
| 14 | 5 | 5 | 5 | 5 | 58 | 5 | 5 | 5 | 4 |
| 15 | 5 | 5 | 5 | 5 | 59 | 5 | 5 | 5 | 5 |
| 16 | 5 | 5 | 5 | 5 | 60 | 5 | 5 | 5 | 5 |
| 17 | 5 | 5 | 5 | 5 | 61 | 5 | 5 | 5 | 5 |
| 18 | 5 | 5 | 5 | 5 | 62 | 5 | 5 | 5 | 5 |
| 19 | 5 | 5 | 5 | 5 | 63 | 5 | 5 | 5 | 5 |
| 20 | 5 | 5 | 5 | 5 | 64 | 5 | 5 | 5 | 5 |
| 21 | 5 | 5 | 5 | 5 | 65 | 5 | 5 | 5 | 5 |
| 22 | 5 | 5 | 5 | 5 | 66 | 5 | 5 | 5 | 5 |
| 23 | 5 | 5 | 5 | 5 | 67 | 5 | 5 | 5 | 5 |
| 24 | 5 | 5 | 5 | 5 | 68 | 5 | 5 | 5 | 5 |
| 25 | 5 | 5 | 5 | 4 | 69 | 5 | 5 | 5 | 5 |
| 26 | 5 | 5 | 5 | 5 | 70 | 5 | 5 | 5 | 5 |
| 27 | 5 | 5 | 5 | 5 | 71 | 5 | 5 | 5 | 5 |
| 28 | 5 | 5 | 5 | 5 | 72 | 5 | 5 | 5 | 4 |
| 29 | 5 | 5 | 5 | 5 | 73 | 5 | 5 | 5 | 5 |
| 30 | 5 | 5 | 5 | 5 | 74 | 5 | 5 | 4 | 4 |
| 31 | 5 | 5 | 5 | 5 | 75 | 5 | 5 | 5 | 5 |
| 32 | 5 | 5 | 5 | 5 | 76 | 5 | 5 | 4 | 5 |
| 33 | 5 | 5 | 5 | 5 | 77 | 5 | 5 | 5 | 5 |
| 34 | 5 | 5 | 5 | 5 | 78 | 5 | 5 | 5 | 5 |
| 35 | 5 | 5 | 5 | 5 | 79 | 5 | 5 | 5 | 5 |
| 36 | 5 | 5 | 5 | 5 | 80 | 5 | 5 | 5 | 5 |
| 37 | 5 | 5 | 5 | 5 | 81 | 5 | 5 | 5 | 5 |
| 38 | 5 | 5 | 5 | 5 | 82 | 5 | 5 | 5 | 5 |
| 39 | 5 | 5 | 5 | 5 | 83 | 5 | 5 | 5 | 5 |
| 40 | 5 | 5 | 5 | 5 | 84 | 5 | 5 | 5 | 5 |
| 41 | 5 | 5 | 5 | 5 | 85 | 5 | 5 | 5 | 5 |
| 42 | 5 | 5 | 5 | 5 | 86 | 5 | 5 | 5 | 5 |
| 43 | 5 | 5 | 5 | 5 | 87 | 5 | 5 | 5 | 5 |
| 44 | 5 | 5 | 5 | 5 | 88 | 5 | 5 | 4 | 5 |

Table 6 (cont'd)

| Test compound | Herbicidal effects | | | | Test compound | Herbicidal effects | | | |
|---|---|---|---|---|---|---|---|---|---|
| | Barnyard grass | Small flower flatsedge | Monochoria | Japanese bulrush | | Barnyard grass | Small flower flatsedge | Monochoria | Japanese bulrush |
| 89 | 5 | 5 | 5 | 5 | 133 | 5 | 5 | 5 | 5 |
| 90 | 5 | 5 | 5 | 5 | 134 | 5 | 5 | 5 | 5 |
| 91 | 5 | 5 | 5 | 5 | 135 | 5 | 5 | 5 | 5 |
| 92 | 5 | 5 | 5 | 5 | 136 | 5 | 5 | 5 | 5 |
| 93 | 5 | 5 | 5 | 5 | 137 | 5 | 5 | 5 | 5 |
| 94 | 5 | 5 | 5 | 5 | 138 | 5 | 5 | 5 | 5 |
| 95 | 5 | 5 | 5 | 5 | 139 | 5 | 5 | 5 | 5 |
| 96 | 5 | 5 | 5 | 5 | 140 | 5 | 5 | 5 | 5 |
| 97 | 5 | 5 | 5 | 5 | 141 | 5 | 5 | 5 | 5 |
| 98 | 5 | 5 | 5 | 5 | 142 | 5 | 5 | 5 | 5 |
| 99 | 5 | 5 | 5 | 5 | 143 | 5 | 5 | 5 | 5 |
| 100 | 5 | 5 | 5 | 5 | 144 | 5 | 5 | 5 | 5 |
| 101 | 5 | 5 | 5 | 5 | 145 | 5 | 5 | 5 | 5 |
| 102 | 5 | 5 | 5 | 5 | 146 | 5 | 5 | 5 | 5 |
| 103 | 5 | 5 | 5 | 5 | 147 | 5 | 5 | 5 | 5 |
| 104 | 5 | 5 | 5 | 5 | 148 | 5 | 5 | 5 | 5 |
| 105 | 5 | 5 | 5 | 4 | 149 | 5 | 5 | 5 | 5 |
| 106 | 5 | 5 | 5 | 4 | 150 | 5 | 5 | 4 | 4 |
| 107 | 5 | 4 | 4 | 4 | 151 | 5 | 5 | 5 | 5 |
| 108 | 5 | 5 | 5 | 5 | 152 | 5 | 5 | 5 | 5 |
| 109 | 5 | 5 | 5 | 5 | 153 | 5 | 5 | 5 | 5 |
| 110 | 5 | 5 | 5 | 5 | 154 | 5 | 4 | 4 | 4 |
| 111 | 5 | 5 | 5 | 5 | 155 | 5 | 5 | 5 | 5 |
| 112 | 4 | 5 | 5 | 5 | 156 | 5 | 5 | 5 | 5 |
| 113 | 5 | 5 | 5 | 5 | 157 | 5 | 5 | 5 | 5 |
| 114 | 5 | 5 | 5 | 5 | 158 | 5 | 5 | 5 | 5 |
| 115 | 5 | 5 | 5 | 5 | 159 | 5 | 5 | 5 | 4 |
| 116 | 5 | 5 | 5 | 5 | 160 | 5 | 5 | 5 | 5 |
| 117 | 5 | 5 | 5 | 5 | 161 | 5 | 5 | 5 | 5 |
| 118 | 5 | 5 | 5 | 5 | 162 | 5 | 5 | 4 | 4 |
| 119 | 5 | 5 | 5 | 5 | 163 | 5 | 5 | 5 | 4 |
| 120 | 5 | 5 | 5 | 5 | 164 | 5 | 5 | 5 | 4 |
| 121 | 5 | 5 | 5 | 5 | 165 | 5 | 5 | 5 | 5 |
| 122 | 5 | 5 | 5 | 5 | 166 | 5 | 5 | 4 | 4 |
| 123 | 5 | 5 | 5 | 5 | 167 | 5 | 5 | 5 | 5 |
| 124 | 5 | 5 | 5 | 5 | 168 | 5 | 5 | 5 | 5 |
| 125 | 5 | 5 | 4 | 4 | 169 | 5 | 5 | 5 | 5 |
| 126 | 5 | 5 | 5 | 5 | 170 | 5 | 5 | 5 | 5 |
| 127 | 5 | 5 | 5 | 4 | 171 | 5 | 5 | 5 | 5 |
| 128 | 5 | 5 | 5 | 5 | 172 | 5 | 5 | 5 | 5 |
| 129 | 5 | 5 | 5 | 5 | 173 | 5 | 5 | 5 | 5 |
| 130 | 5 | 5 | 5 | 5 | 174 | 5 | 5 | 5 | 5 |
| 131 | 5 | 5 | 5 | 5 | 175 | 5 | 5 | 5 | 5 |
| 132 | 5 | 5 | 5 | 5 | 176 | 5 | 5 | 5 | 4 |

Table 6 (cont'd)

| Test compound | Herbicidal effects: Barnyard grass | Small flower flatsedge | Monochoria | Japanese bulrush |
|---|---|---|---|---|
| 177 | 5 | 5 | 5 | 5 |
| 178 | 5 | 5 | 5 | 5 |
| 179 | 5 | 5 | 5 | 5 |
| 180 | 5 | 5 | 5 | 5 |
| 181 | 5 | 5 | 5 | 5 |
| 182 | 5 | 5 | 5 | 5 |
| 183 | 5 | 5 | 5 | 5 |
| 184 | 5 | 5 | 5 | 5 |
| 185 | 5 | 5 | 5 | 5 |
| 186 | 5 | 5 | 5 | 5 |
| 187 | 5 | 5 | 5 | 5 |
| 188 | 5 | 5 | 5 | 5 |
| 189 | 5 | 5 | 5 | 5 |
| 190 | 5 | 5 | 5 | 5 |
| 191 | 5 | 5 | 5 | 5 |
| 192 | 5 | 4 | 5 | 2 |
| 193 | 5 | 5 | 5 | 5 |
| 194 | 5 | 5 | 5 | 5 |
| 195 | 5 | 5 | 5 | 5 |
| 196 | 5 | 5 | 5 | 5 |
| 197 | 5 | 5 | 5 | 5 |
| 198 | 5 | 5 | 5 | 5 |
| 199 | 5 | 5 | 5 | 5 |
| 200 | 5 | 5 | 5 | 5 |
| 201 | 5 | 5 | 5 | 5 |
| 202 | 5 | 5 | 5 | 5 |
| 203 | 5 | 5 | 5 | 5 |
| 204 | 5 | 5 | 5 | 5 |
| 205 | 5 | 5 | 5 | 5 |
| 206 | 5 | 5 | 5 | 5 |
| 207 | 5 | 5 | 5 | 5 |
| 208 | 5 | 5 | 5 | 5 |
| 209 | 5 | 5 | 5 | 5 |
| 210 | 5 | 5 | 5 | 5 |
| 213 | 5 | 5 | 5 | 5 |
| 214 | 5 | 5 | 5 | 5 |
| 216 | 5 | 5 | 5 | 5 |
| 217 | 5 | 5 | 5 | 5 |
| 218 | 5 | 5 | 5 | 5 |
| 219 | 5 | 5 | 5 | 5 |
| 220 | 5 | 5 | 5 | 5 |
| 222 | 5 | 5 | 5 | 5 |
| 223 | 5 | 5 | 5 | 5 |
| 225 | 5 | 5 | 5 | 5 |
| 226 | 5 | 5 | 5 | 5 |
| 227 | 5 | 5 | 5 | 5 |
| 228 | 5 | 5 | 5 | 5 |
| 229 | 5 | 5 | 5 | 5 |
| 230 | 5 | 5 | 5 | 5 |
| 231 | 5 | 5 | 5 | 5 |
| 232 | 5 | 5 | 5 | 5 |
| 233 | 5 | 5 | 5 | 5 |
| 234 | 5 | 5 | 5 | 5 |
| 235 | 5 | 5 | 5 | 5 |
| 236 | 5 | 5 | 5 | 5 |
| 237 | 5 | 5 | 5 | 5 |
| 238 | 5 | 5 | 5 | 5 |
| 239 | 5 | 5 | 5 | 5 |
| 240 | 5 | 5 | 5 | 5 |
| 242 | 5 | 5 | 5 | 5 |
| 243 | 5 | 5 | 5 | 5 |
| 244 | 5 | 5 | 5 | 5 |
| 245 | 5 | 5 | 5 | 5 |
| 246 | 5 | 5 | 5 | 5 |
| 247 | 5 | 5 | 5 | 5 |
| 248 | 5 | 5 | 5 | 5 |
| 249 | 5 | 5 | 5 | 5 |
| 250 | 5 | 5 | 5 | 5 |
| 251 | 5 | 5 | 5 | 5 |
| Comparative Compound* | 0 | 0 | 0 | 0 |

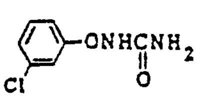 (Compound disclosed in U.S. Patent 3,332,975)

Test Example 2

Herbicidal test for selectivity between rice and barnyardgrass

Into each 1/5000 are Wager pot, paddy soil was filled and puddled, then seeds of barnyardgrass were sown and grown until they reached 2-leaf stage. The barnyardgrass of 2-leaf stage was thinned out to adjust the number of barnyardgrass to fifteen per pot, and rice seedlings of 2-leaf stage were transplanted in the same pot. One day after the transplant, a predetermined amount of the wettable powder prepared from the test compound in accordance with Formulation Example 3 and diluted with water, was dropwise applied to the surface of the water. Thereafter, they were grown in a green house, and thirty days after the application, the herbicidal effects and phytotoxity were evaluated in accordance with the standards as identified in Table 5. The results thereby obtained are shown in Table 7.

Table 7

| Test compound | Dose kg/Ha | Herbicidal effects Barnyard grass | Phytotoxicity Paddy rice |
|---|---|---|---|
| 1 | 1 | 5 | 1 |
| | 0.5 | 5 | 0 |
| | 0.25 | 5 | 0 |
| 2 | 1 | 5 | 1 |
| | 0.5 | 5 | 0 |
| 5 | 1 | 5 | 1 |
| | 0.5 | 5 | 0 |
| | 0.25 | 5 | 0 |
| 6 | 1 | 5 | 0 |
| | 0.5 | 5 | 0 |
| | 0.25 | 5 | 0 |
| 16 | 0.5 | 5 | 0 |
| | 0.25 | 5 | 0 |
| 21 | 0.5 | 5 | 1 |
| | 0.25 | 5 | 0 |
| 26 | 1 | 5 | 0 |
| | 0.5 | 5 | 0 |
| 27 | 1 | 5 | 0 |
| | 0.5 | 5 | 0 |
| 28 | 1 | 5 | 1 |
| | 0.5 | 5 | 0 |
| | 0.25 | 5 | 0 |
| 29 | 1 | 5 | 0 |
| | 0.5 | 5 | 0 |
| | 0.25 | | 0 |
| 30 | 1 | 5 | 0 |
| | 0.5 | 4.5 | 0 |
| 31 | 1 | 5 | 0 |
| | 0.5 | 4 | 0 |
| 33 | 1 | 5 | 0 |
| | 0.5 | 5 | 0 |
| | 0.25 | 5 | 0 |
| 34 | 1 | 5 | 1 |
| | 0.5 | 5 | 0 |
| | 0.25 | 5 | 0 |

| Test compound | Dose kg/Ha | Herbicidal effects Barnyard grass | Phytotoxicity Paddy rice |
|---|---|---|---|
| 38 | 1 | 5 | 0 |
| | 0.5 | 5 | 0 |
| | 0.25 | 5 | 0 |
| 39 | 0.5 | 5 | 0 |
| | 0.25 | 5 | 0 |
| 40 | 1 | 5 | 0 |
| | 0.5 | 5 | 0 |
| | 0.25 | 5 | 0 |
| 41 | 1 | 5 | 1.5 |
| | 0.5 | 5 | 0 |
| | 0.25 | 4 | 0 |
| 42 | 1 | 5 | 0 |
| | 0.5 | 5 | 0 |
| | 0.25 | 5 | 0 |
| 43 | 1 | 5 | 0.5 |
| | 0.5 | 5 | 0 |
| 44 | 1 | 5 | 0 |
| | 0.5 | 5 | 0 |
| 45 | 1 | 5 | 0 |
| | 0.5 | 5 | 0 |
| | 0.25 | 4.5 | 0 |
| 47 | 1 | 5 | 1 |
| | 0.5 | 5 | 1 |
| | 0.25 | 5 | 0 |
| 49 | 1 | 5 | 0 |
| | 0.5 | 5 | 0 |
| 50 | 1 | 5 | 0 |
| | 0.5 | 5 | 0 |
| | 0.25 | 5 | 0 |
| 51 | 1 | 5 | 0 |
| | 0.5 | 5 | 0 |
| | 0.25 | 4.5 | 0 |
| 52 | 1 | 5 | 0 |
| | 0.5 | 5 | 0 |
| | 0.25 | 4 | 0 |
| 55 | 1 | 5 | 0 |
| | 0.5 | 4.5 | 0 |

Table 7 (cont'd)

| Test compound | Dose kg/Ha | Herbicidal effects: Barnyard grass | Phytotoxicity: Paddy rice |
|---|---|---|---|
| 57 | 1 | 5 | 0 |
| | 0.5 | 5 | 0 |
| | 0.25 | 5 | 0 |
| 59 | 1 | 5 | 1 |
| | 0.5 | 5 | 0.5 |
| | 0.25 | 5 | 0 |
| 60 | 1 | 5 | 0 |
| | 0.5 | 5 | 0 |
| | 0.25 | 5 | 0 |
| 61 | 1 | 5 | 1 |
| | 0.5 | 5 | 1 |
| | 0.25 | 3 | 0 |
| 62 | 1 | 5 | 0 |
| | 0.5 | 4.5 | 0 |
| | 0.25 | 4.5 | 0 |
| 67 | 1 | 5 | 0 |
| | 0.5 | 4 | 0 |
| | 0.25 | 4 | 0 |
| 68 | 1 | 5 | 1 |
| | 0.5 | 5 | 0 |
| | 0.25 | 5 | 0 |
| 76 | 1 | 5 | 0 |
| | 0.5 | 4 | 0 |
| | 0.25 | | |
| 77 | 1 | 5 | 0 |
| | 0.5 | 5 | 0 |
| 79 | 1 | 5 | 1 |
| | 0.5 | 5 | 0 |
| | 0.25 | 5 | 0 |
| 80 | 1 | 5 | 2 |
| | 0.5 | 4 | 0 |
| | 0.25 | 4 | 0 |
| 84 | 1 | 5 | 0 |
| | 0.5 | 5 | 0 |
| | 0.25 | 4.5 | |
| 86 | 1 | 5 | 1 |
| | 0.5 | 5 | 0 |
| 87 | 1 | 5 | 0 |
| | 0.5 | 5 | 0 |
| | 0.25 | 5 | 0 |
| 89 | 1 | 5 | 1 |
| | 0.5 | 5 | 1 |
| | 0.25 | | 0 |
| 92 | 1 | 5 | 0 |
| | 0.5 | 5 | 0 |
| 98 | 1 | 5 | 2 |
| | 0.5 | 5 | 0 |
| 100 | 1 | 5 | 0 |
| | 0.5 | 4.5 | 0 |
| 101 | 1 | 5 | 1 |
| | 0.5 | 5 | 0 |
| 103 | 1 | 5 | 0 |
| | 0.5 | 5 | 0 |
| 104 | 1 | 5 | 0 |
| | 0.5 | 4.5 | 0 |
| | 0.25 | 4 | 0 |
| 128 | 0.5 | 5 | 0 |
| | 0.25 | 5 | 0 |
| 138 | 0.5 | 5 | 0 |
| | 0.25 | 5 | 0 |
| 146 | 1 | 5 | 1 |
| | 0.5 | 5 | 1 |
| | 0.25 | 5 | 0 |
| 171 | 1 | 5 | 1 |
| | 0.5 | 5 | 0 |
| | 0.25 | 5 | 0 |
| 172 | 0.5 | 5 | 1 |
| | 0.25 | 5 | 0 |
| 179 | 0.5 | 5 | 1 |
| | 0.25 | 5 | 0.5 |
| 182 | 1 | 5 | 0 |
| | 0.5 | 5 | 0 |

Table 7 (cont'd)

| Test compound | Dose kg/Ha | Herbicidal effects Barnyard grass | Phytotoxicity Paddy rice | Test compound | Dose kg/Ha | Herbicidal effects Barnyard grass | Phytotoxicity Paddy rice |
|---|---|---|---|---|---|---|---|
| 183 | 1<br>0.5<br>0.25 | 5<br>5<br>5 | 1<br>0<br>0 | 200 | 1<br>0.5<br>0.25 | 5<br>5<br>5 | 1.5<br>1.5<br>0 |
| 186 | 0.5<br>0.25 | 5<br>5 | 1<br>0 | 201 | 1<br>0.5<br>0.25 | 5<br>5<br>5 | 3<br>0<br>0 |
| 187 | 1<br>0.5<br>0.25 | 5<br>5<br>5 | 1<br>0<br>0 | 202 | 1<br>0.5<br>0.25 | 5<br>5<br>5 | 3<br>3<br>0 |
| 188 | 1<br>0.5<br>0.25 | 5<br>5<br>5 | 1.5<br>0<br>0 | 203 | 0.5<br>0.25 | 5<br>5 | 3<br>0 |
| 189 | 1<br>0.5<br>0.25 | 5<br>5<br>5 | 1<br>1<br>0 | 204 | 1<br>0.5<br>0.25 | 5<br>5<br>5 | 0<br>0<br>0 |
| 190 | 1<br>0.5<br>0.25 | 5<br>5<br>5 | 1<br>1<br>0 | 205 | 1<br>0.5<br>0.25 | 5<br>4.5<br>3 | 0<br>0<br>0 |
| 191 | 1<br>0.5<br>0.25 | 5<br>5<br>5 | 3<br>1.5<br>1 | 206 | 1<br>0.5<br>0.25 | 5<br>5<br>5 | 3<br>1<br>0 |
| 193 | 1<br>0.5<br>0.25 | 5<br>5<br>5 | 3<br>1<br>1 | 207 | 1<br>0.5<br>0.25 | 5<br>5<br>5 | 0<br>0<br>0 |
| 194 | 1<br>0.5<br>0.25 | 5<br>5<br>5 | 0.5<br>0<br>0 | 208 | 1<br>0.5<br>0.25 | 5<br>5<br>5 | 0.5<br>0<br>0 |
| 195 | 1<br>0.5<br>0.25 | 5<br>5<br>5 | 0<br>0<br>0 | 209 | 1<br>0.5<br>0.25 | 5<br>5<br>4.5 | 0<br>0<br>0 |
| 196 | 1<br>0.5<br>0.25 | 5<br>4<br>2 | 0<br>0<br>0 | 210 | 1<br>0.5<br>0.25 | 5<br>5<br>5 | 0<br>0<br>0 |
| 197 | 1<br>0.5<br>0.25 | 5<br>5<br>5 | 0<br>0<br>0 | 213 | 1<br>0.5<br>0.25 | 5<br>5<br>5 | 1<br>1<br>1 |
| 199 | 1<br>0.5<br>0.25 | 5<br>5<br>4 | 2<br>1<br>0 | 214 | 1<br>0.5<br>0.25 | 5<br>5<br>4 | 0<br>0<br>0 |

Table 7 (cont'd)

| Test compound | Dose kg/Ha | Herbicidal effects Barnyard grass | Phytotoxicity Paddy rice |
|---|---|---|---|
| 216 | 1 | 5 | 0 |
| | 0.5 | 4 | 0 |
| | 0.25 | 4 | 0 |
| 218 | 1 | 5 | 0 |
| | 0.5 | 5 | 0 |
| | 0.25 | 4 | 0 |
| 219 | 0.25 | 5 | 0 |
| 220 | 1 | 5 | 1 |
| | 0.5 | 5 | 1 |
| | 0.25 | 5 | 0 |
| 222 | 1 | 5 | 1 |
| | 0.5 | 5 | 0 |
| | 0.25 | 5 | 0 |
| 223 | 1 | 5 | 2 |
| | 0.5 | 5 | 0 |
| | 0.25 | 5 | 0 |
| 226 | 1 | 5 | 2 |
| | 0.5 | 5 | 0 |
| | 0.25 | 5 | 0 |
| 227 | 1 | 5 | 3 |
| | 0.5 | 5 | 1 |
| | 0.25 | 5 | 0 |
| 228 | 0.5 | 5 | 1 |
| | 0.25 | 5 | 0 |
| 229 | 1 | 5 | 1 |
| | 0.5 | 5 | 0 |
| | 0.25 | 4 | 0 |
| 230 | 1 | 5 | 1.5 |
| | 0.5 | 5 | 1.5 |
| | 0.25 | 5 | 1 |
| 231 | 1 | 5 | 0 |
| | 0.5 | 5 | 0 |
| | 0.25 | 5 | 0 |
| 232 | 1 | 5 | 1 |
| | 0.5 | 5 | 0 |
| | 0.25 | 4 | 0 |
| 233 | 1 | 5 | 2 |
| | 0.5 | 5 | 0 |
| | 0.25 | 4.5 | 0 |
| 234 | 1 | 5 | 0 |
| | 0.5 | 5 | 0 |
| | 0.25 | 4.5 | 0 |
| 235 | 0.25 | 5 | 0 |
| 237 | 0.5 | 5 | 0 |
| | 0.25 | 5 | 0 |
| 238 | 1 | 5 | 0 |
| | 0.5 | 5 | 0 |
| | 0.25 | 5 | 0 |
| 239 | 1 | 5 | 0 |
| | 0.5 | 5 | 0 |
| | 0.25 | 5 | 0 |
| 240 | 1 | 5 | 2 |
| | 0.5 | 5 | 1 |
| | 0.25 | 5 | 0 |
| 244 | 1 | | 0 |
| | 0.5 | | 0 |
| | 0.25 | | 0 |
| 245 | 1 | | 0 |
| | 0.5 | | 0 |
| | 0.25 | | 0 |
| 246 | 1 | 5 | 0 |
| | 0.5 | 5 | 0 |
| | 0.25 | 3 | 0 |
| 247 | 1 | | 0 |
| | 0.5 | | 0 |
| | 0.25 | | 0 |
| 248 | 1 | 5 | 0 |
| | 0.5 | 5 | 0 |
| | 0.25 | 2 | 0 |
| 249 | 0.5 | 5 | 0 |
| | 0.25 | 5 | 0 |
| 250 | 1 | 5 | 0 |
| | 0.5 | 5 | 0 |
| | 0.25 | 4 | 0 |

Table 7 (cont'd)

| Test compound | Dose kg/Ha | Herbicidal effects Barnyard grass | Phytotoxicity Paddy rice | Test compound | Dose kg/Ha | Herbicidal effects Barnyard grass | Phytotoxicity Paddy rice |
|---|---|---|---|---|---|---|---|
| 251 | 1 | 5 | 3.5 | | | | |
| | 0.5 | 4 | 0 | | | | |
| | 0.25 | 3.5 | 0 | | | | |
| DCMU (Comparative Compound) | 1 | 5 | 5 | | | | |
| | 0.5 | 5 | 4 | | | | |
| | 0.25 | 3 | 2 | | | | |

Test Example 3

Herbicidal test by upland soil treatment

Into each plastic pot of 600 $cm^2$, upland soil was filled, then seeds of barnyardgrass, crabgrass, green foxtail, redroot pigweed, rice flatsedge, rice, wheat, maize, soybean, cotton and sugar beet were sown, and they were covered with soil. A predetermined amount of the wettable powder prepared from the test compound in accordance with Formulation Example 3 and diluted with water, was uniformly applied to the surface of the soil at an application rate of 1000 liter/hectare by means of a small size spray. They were grown in a greenhouse, and twenty days after the application, the herbicidal effects and phytotoxicity were evaluated in accordance with the standards as identified in Table 5. The results thereby obtained are shown in Table 8.

Table 8

| Test compound | Dose kg/HA | Herbicidal effects: Barnyard-grass | Crab-grass | Green foxtail | Redroot pigweed | Rice flatsedge | Phytotoxicity: Rice | Wheat | Maize | Soybean | Cotton | Sugar beet |
|---|---|---|---|---|---|---|---|---|---|---|---|---|
| 1 | 4 | 5 | 5 | 5 | 5 | 5 | 0 | 0 | 0 | 0 | 0 | 3 |
| | 1 | 5 | 5 | 5 | 5 | 5 | 0 | 0 | 0 | 0 | 0 | 2 |
| 5 | 4 | 5 | 5 | 5 | 5 | 5 | 0 | 0 | 0 | 0 | 0 | 3 |
| | 1 | 5 | 5 | 5 | 5 | 5 | 0 | 0 | 0 | 0 | 0 | 2 |
| 7 | 4 | 5 | 5 | 5 | 5 | 5 | 2 | 0.5 | 4 | 0 | 0 | 1 |
| | 1 | 5 | 4 | 5 | 3 | 5 | 0 | 0 | 0.5 | 0 | 0 | 0.5 |
| 21 | 4 | 5 | 5 | 5 | 5 | 5 | 4.5 | 4.5 | 5 | 4 | 0.5 | 2.5 |
| | 1 | 5 | 5 | 5 | 5 | 5 | 5 | 3 | 5 | 1.5 | 0 | 2 |
| 26 | 4 | 5 | 5 | 5 | 3 | 5 | 5 | 2 | 4.5 | 0 | 0 | 1.5 |
| | 1 | 5 | 5 | 5 | 1 | 5 | 4 | 0.5 | 4 | 0 | 0 | 1 |
| 27 | 4 | 5 | 5 | 5 | 1 | 5 | 2.5 | 2 | 4 | 0 | 0 | 0.5 |
| | 1 | 5 | 5 | 5 | 0 | 5 | 0.5 | 0 | 2 | 0 | 0 | 0 |
| 30 | 4 | 5 | 5 | 5 | 5 | 5 | 3.5 | 1 | 3.5 | 0 | 0 | - |
| | 1 | 5 | 5 | 5 | 5 | 5 | 1 | 0 | 0 | 0 | 0 | - |
| 59 | 4 | 5 | 5 | 5 | 5 | 5 | 4.5 | 2.5 | 4.5 | 2 | 1 | 1.5 |
| | 1 | 5 | 5 | 5 | 5 | 5 | 0 | 0 | 1.5 | 0 | 0.5 | 0.5 |
| 60 | 4 | 5 | 5 | 5 | 5 | 5 | 0 | 0.5 | 3.5 | 0 | 0 | 0 |
| | 1 | 5 | 5 | 5 | 5 | 5 | 0 | 0 | 4 | 0 | 0 | 0 |
| 61 | 4 | 5 | 5 | 5 | 5 | 5 | 3.5 | 2.5 | 4 | 0 | 0 | 0.5 |
| | 1 | 5 | 5 | 5 | 5 | 5 | 0.5 | 0 | 3.5 | 0 | 0 | 0 |
| 62 | 4 | 5 | 5 | 5 | 5 | 5 | 3 | 1.5 | 4 | 0 | 0 | 0 |
| | 1 | 5 | 5 | 5 | 5 | 5 | 1 | 0 | 3 | 0 | 0 | 0 |
| 66 | 4 | 5 | 5 | 5 | 5 | 5 | 0 | 1.5 | 3 | 0 | 0 | 0 |
| | 1 | 5 | 5 | 5 | 1 | 5 | 0 | 0.5 | 0.5 | 0 | 0 | 0 |
| 67 | 4 | 5 | 5 | 5 | 4 | 5 | 3 | 3 | 4 | 0 | 0 | 0 |
| | 1 | 5 | 5 | 5 | 4 | 5 | 0.5 | 0.5 | 3.5 | 0 | 0 | 0 |
| 68 | 4 | 5 | 5 | 5 | 5 | 5 | 1.5 | 1.5 | 4 | 0 | 0 | 0 |
| | 1 | 5 | 5 | 5 | 4.5 | 5 | 0.5 | 1 | 3 | 0 | 0 | 0 |
| 76 | 4 | 5 | 5 | 5 | 5 | 5 | 2 | 2 | 2.5 | 0 | 0 | 0.5 |
| | 1 | 5 | 4.5 | 5 | 4 | 5 | 0 | 1 | 0 | 0 | 0 | 0 |
| 80 | 4 | 5 | 5 | 4.5 | 5 | 5 | 0 | 1 | 1 | 0 | 0 | 0.5 |
| | 1 | 5 | 5 | 5 | 5 | 5 | 0 | 0 | 0 | 0 | 0 | 0 |
| 81 | 4 | 5 | 5 | 5 | 5 | 5 | 0.5 | 0 | 3.5 | 0 | 0 | 0 |
| | 1 | 5 | 4 | 5 | 4 | 4 | 0 | 0 | 0 | 0 | 0 | 0 |
| 86 | 4 | 5 | 5 | 5 | 5 | 5 | 0 | 0.5 | 3.5 | 0 | 0 | 1 |
| | 1 | 5 | 5 | 5 | 5 | 5 | 0 | 0.5 | 2.5 | 0 | 0 | 0.5 |
| 91 | 4 | 5 | 4 | 5 | 4 | 5 | 0 | 0.5 | 0 | 0 | 0 | 0.5 |
| | 1 | 5 | 3 | 4.5 | 4 | 5 | 0 | 0 | 0 | 0 | 0 | 0 |
| 100 | 4 | 5 | 5 | 5 | 5 | 5 | 0.5 | 0 | 3.5 | 0 | 0 | 1 |
| | 1 | 5, | 4.5 | 5 | 4.5 | 5 | 0 | 0 | 1.5 | 0 | 0 | 0 |
| 102 | 4 | 5 | 5 | 5 | 5 | 5 | 0 | 0.5 | 1.5 | 0 | 0 | 0 |
| | 1 | 5 | 5 | 5 | 5 | 5 | 0 | 0.5 | 1.5 | 0 | 0 | 0 |
| 104 | 4 | 5 | 5 | 5 | 0 | 5 | 0 | 0.5 | 0.5 | 0.5 | 0 | 0.5 |
| | 1 | 5 | 5 | 5 | 0 | 5 | 0 | 0.5 | 0 | 0 | 0 | 0 |

Table 8 (cont'd)

| Test compound | Dose kg/HA | Herbicidal effects: Barnyard-grass | Crab-grass | Green foxtail | Redroot pigweed | Rice flatsedge | Phytotoxicity: Rice | Wheat | Maize | Soybean | Cotton | Sugar beet |
|---|---|---|---|---|---|---|---|---|---|---|---|---|
| 128 | 4 | 5 | 5 | 5 | 5 | 5 | 5 | 4.5 | 5 | 0.5 | 0 | 1.5 |
| | 1 | 5 | 5 | 5 | 4 | 5 | 1 | 1.5 | 3.3 | 0 | 0 | 0.5 |
| 146 | 4 | 5 | 5 | 5 | 5 | 5 | 1 | 1.5 | 4.5 | 1.5 | 1 | 1.5 |
| | 1 | 5 | 5 | 5 | 5 | 5 | 0 | 0.3 | 1 | 0 | 0 | 0.5 |
| 171 | 4 | 5 | 5 | 5 | 5 | 5 | 5 | 5 | 5 | 2.5 | 0.5 | 3.5 |
| | 1 | 5 | 5 | 5 | 5 | 5 | 1.5 | 2.5 | 5 | 0.5 | 0 | 2 |
| 172 | 4 | 5 | 5 | 5 | 5 | 5 | 2 | 3.5 | 4 | 0.5 | 0 | 2 |
| | 1 | 5 | 5 | 5 | 5 | 5 | 0 | 1 | 2.5 | 0 | 0 | 1 |
| 179 | 4 | 5 | 5 | 5 | 5 | 5 | 5 | 5 | 5 | 3 | 1 | 2 |
| | 1 | 5 | 5 | 5 | 5 | 5 | 5 | 4 | 5 | 0.5 | 0 | 1.5 |
| 182 | 4 | 5 | 5 | 5 | 5 | 5 | | | | | | |
| | 1 | 5 | 5 | 5 | 3 | 5 | | | | | | |
| 186 | 4 | 5 | 5 | | 5 | 5 | | | | | | |
| | 1 | 5 | 5 | | 5 | 5 | | | | | | |
| 187 | 4 | 5 | 5 | | 5 | 5 | | | | | | |
| | 1 | 5 | 4 | | 5 | 4 | | | | | | |
| 188 | 4 | 5 | 5 | 5 | 5 | 5 | | | | 0 | 0 | 0.5 |
| | 1 | 5 | 5 | 5 | 2 | 5 | | | | 0 | 0 | 0 |
| 189 | 4 | 5 | 5 | 5 | 3 | 5 | | | | 0 | 0 | 0 |
| | 1 | 5 | 5 | 5 | 2 | 5 | | | | 0 | 0 | 0 |
| 190 | 4 | 5 | 5 | 5 | 5 | 5 | | | | 0 | 0 | |
| | 1 | 5 | 5 | 5 | 5 | 5 | | | | 0 | 0 | |
| 191 | 4 | 5 | 5 | 5 | 5 | 5 | | | | 0 | 0 | |
| | 1 | 5 | 5 | 5 | 5 | 5 | | | | 0 | 0 | |
| 193 | 4 | 5 | 5 | 5 | 5 | 5 | | | | 1 | 1 | 1.5 |
| | 1 | 5 | 5 | 5 | 5 | 5 | | | | 0 | 0 | 0.5 |
| 194 | 4 | 5 | 5 | 5 | 3 | 5 | | | | 0 | 0 | 0.5 |
| | 1 | 5 | 5 | 5 | 1 | 5 | | | | 0 | 0 | 0.5 |
| 195 | 5 | 5 | 5 | 5 | 4 | 5 | | | | 0 | 0 | 1 |
| | 1 | 5 | 5 | 5 | 3 | 5 | | | | 0 | 0 | 0 |
| 196 | 4 | 5 | 5 | | 2 | 5 | | | | | | |
| | 1 | 4 | 4 | | 0 | 5 | | | | | | |
| 197 | 4 | 5 | 5 | 5 | 2 | 5 | | | | 0 | 0 | 0 |
| | 1 | 5 | 5 | 5 | 2 | 5 | | | | 0 | 0 | 0 |
| 198 | 4 | 5 | 5 | 5 | 5 | 5 | | | | 0 | 1 | 2.5 |
| | 1 | 5 | 5 | 5 | 5 | 5 | | | | 0 | 0 | 2 |
| 199 | 4 | 5 | 5 | | 3 | 5 | | | | | | |
| | 1 | 5 | 4 | | 3 | 2 | | | | | | |
| 200 | 4 | 5 | 5 | 5 | 3 | 5 | | | | 0 | 0 | 1 |
| | 1 | 5 | 5 | 5 | 1 | 5 | | | | 0 | 0 | 0.5 |
| 201 | 4 | 5 | 5 | | 5 | 5 | | | | | | |
| | 1 | 5 | 5 | | 3 | 5 | | | | | | |
| 202 | 4 | 5 | 5 | 5 | 5 | 5 | | | | 0 | 0 | 1 |
| | 1 | 5 | 5 | 5 | 2 | 5 | | | | 0 | 0 | 0.5 |

Table 9 (cont'd)

| Test compound | Dose kg/HA | Herbicidal effects: Barnyard-grass | Crab-grass | Green foxtail | Redroot pigweed | Rice flatsedge | Phytotoxicity: Rice | Wheat | Maize | Soybean | Cotton | Sugar beet |
|---|---|---|---|---|---|---|---|---|---|---|---|---|
| 203 | 4 | 5 | 5 | 5 | 5 | 5 | | | | 0 | 0.5 | 0.5 |
| | 1 | 5 | 5 | 5 | 3 | 5 | | | | 0 | 0 | 0 |
| 204 | 4 | 5 | 5 | | 3 | 5 | | | | | | |
| | 1 | 2 | 4 | | 0 | 5 | | | | | | |
| 205 | 4 | 5 | 5 | | 2 | 5 | | | | | | |
| | 1 | 2 | 4 | | 2 | 5 | | | | | | |
| 206 | 4 | 5 | 5 | 5 | 5 | 5 | | | | 2.5 | 0.5 | 3 |
| | 1 | 5 | 5 | 5 | 1 | 5 | | | | 0.5 | 0 | 1 |
| 207 | 4 | 5 | 5 | 5 | 4 | 5 | | | | 0 | 0 | 1 |
| | 1 | 5 | 5 | 5 | 3 | 5 | | | | 0 | 0 | 1 |
| 208 | 4 | 5 | 5 | 5 | 4 | 5 | | | | 0 | 0 | 1 |
| | 1 | 5 | 5 | 5 | 1 | 5 | | | | 0 | 0 | 0.5 |
| 209 | 4 | 5 | 5 | | 3 | 5 | | | | | | |
| | 1 | 5 | 5 | | 0 | 5 | | | | | | |
| 210 | 4 | 5 | 5 | | 4 | 5 | | | | | | |
| | 1 | 5 | 5 | | 2 | 5 | | | | | | |
| 213 | 4 | 5 | 5 | 5 | 5 | 5 | | | | 0 | 1.5 | 1.5 |
| | 1 | 5 | 5 | 5 | 5 | 5 | | | | 0 | 0 | 0 |
| 214 | 4 | 5 | 4 | | 2 | 5 | | | | | | |
| | 1 | 3 | 3 | | 0 | 5 | | | | | | |
| 217 | 4 | 5 | 5 | | 5 | 5 | | | | | | |
| | 1 | 4 | 5 | | 1 | 5 | | | | | | |
| 218 | 4 | 5 | 5 | | 1 | 5 | | | | | | |
| | 1 | 5 | 5 | | 0 | 5 | | | | | | |
| 219 | 4 | 5 | 5 | 5 | 5 | 5 | | | | 0.5 | 1 | 3.5 |
| | 1 | 5 | 5 | 5 | 5 | 5 | | | | 0 | 0 | 2 |
| 220 | 4 | 5 | 5 | | 5 | 5 | | | | | | |
| | 1 | 5 | 5 | | 3 | 5 | | | | | | |
| 222 | 4 | 5 | 5 | | 3 | 5 | | | | | | |
| | 1 | 5 | 5 | | 3 | 5 | | | | | | |
| 223 | 4 | 5 | 5 | 5 | 5 | 5 | | | | 0.5 | 0 | 1.5 |
| | 1 | 5 | 5 | 5 | 4 | 5 | | | | 0 | 0 | 0.5 |
| 225 | 4 | 5 | 5 | | 3 | 5 | | | | | | |
| | 1 | 5 | 4 | | 0 | 2 | | | | | | |
| 226 | 4 | 5 | 5 | | 3 | 5 | | | | | | |
| | 1 | 4 | 4 | | 1 | 5 | | | | | | |
| 227 | 4 | 5 | 5 | | 3 | 5 | | | | | | |
| | 1 | 5 | 5 | | 2 | 5 | | | | | | |
| 228 | 4 | 5 | 5 | | 5 | 5 | | | | | | |
| | 1 | 4 | 4 | | 0 | 5 | | | | | | |
| 229 | 4 | 5 | 5 | | 4 | 5 | | | | | | |
| | 1 | 3 | 4 | | 0 | 5 | | | | | | |
| 230 | 4 | 5 | 5 | 5 | 5 | 5 | | | | 2 | 1 | 3.5 |
| | 1 | 5 | 5 | 5 | 5 | 5 | | | | 1 | 0 | 2 |

Table 8 (cont'd)

| Test compound | Dose kg/HA | Herbicidal effects | | | | | Phytotoxicity | | | | | |
|---|---|---|---|---|---|---|---|---|---|---|---|---|
| | | Barnyard-grass | Crab-grass | Green foxtail | Redroot pigweed | Rice flatsedge | Rice | Wheat | Maize | Soybean | Cotton | Sugar beet |
| 231 | 4 | 5 | 5 | | 4 | 5 | | | | | | |
| | 1 | 3 | 5 | | 1 | 5 | | | | | | |
| 232 | 4 | 5 | 5 | | 3 | 5 | | | | | | |
| | 1 | 4 | 5 | | 0 | 5 | | | | | | |
| 233 | 4 | 5 | 5 | | 2 | 5 | | | | | | |
| | 1 | 4 | 4 | | 2 | 5 | | | | | | |
| 234 | 4 | 5 | 5 | | 4 | 5 | | | | | | |
| | 1 | 5 | 5 | | 3 | 5 | | | | | | |
| 235 | 4 | 5 | 5 | 5 | 5 | 5 | | | | 2 | 1 | 1.5 |
| | 1 | 5 | 5 | 5 | 2 | 5 | | | | 0 | 0 | 0.5 |
| 236 | 4 | 5 | 5 | | 0 | 5 | | | | | | |
| | 1 | 5 | 4 | | 0 | 5 | | | | | | |
| 237 | 4 | 5 | 5 | 5 | 5 | 5 | | | | 3.5 | 1.5 | 4 |
| | 1 | 5 | 5 | 5 | 5 | 5 | | | | 1 | 0 | 2 |
| 238 | 4 | 5 | 5 | | 4 | 5 | | | | | | |
| | 1 | 3 | 5 | | 0 | 5 | | | | | | |
| 239 | 4 | 5 | 5 | | 3 | 5 | | | | | | |
| | 1 | 5 | 5 | | 2 | 5 | | | | | | |
| 240 | 4 | 5 | 5 | | 5 | 5 | | | | | | |
| | 1 | 5 | 5 | | 1 | 5 | | | | | | |
| 242 | 4 | 5 | 5 | | 5 | 5 | | | | | | |
| | 1 | 5 | 5 | | 5 | 5 | | | | | | |
| 243 | 4 | 5 | 5 | | 5 | 5 | | | | | | |
| | 1 | 5 | 5 | | 5 | 5 | | | | | | |
| 244 | 4 | 5 | 5 | | 0 | 5 | | | | | | |
| | 1 | 3 | 2 | | 0 | 5 | | | | | | |
| 245 | 4 | 5 | 5 | | 5 | 5 | | | | | | |
| | 1 | 4 | 4 | | 2 | 5 | | | | | | |
| 246 | 4 | 5 | 5 | | 4 | 5 | | | | | | |
| | 1 | 5 | 5 | | 4 | 5 | | | | | | |
| 247 | 4 | 5 | 5 | 5 | 2 | 5 | | | | 0 | 0 | 2 |
| | 1 | 5 | 5 | 5 | 2 | 5 | | | | 0 | 0 | 0.5 |
| 248 | 4 | 5 | 5 | | 5 | 5 | | | | | | |
| | 1 | 5 | 5 | | 3 | 5 | | | | | | |
| 249 | 4 | 5 | 5 | | 5 | 5 | | | | | | |
| | 1 | 5 | 5 | | 5 | 5 | | | | | | |
| 250 | 4 | 5 | 5 | | 0 | 5 | | | | | | |
| | 1 | 1 | 2 | | 0 | 4 | | | | | | |
| 251 | 4 | 5 | 5 | | 5 | 5 | | | | | | |
| | 1 | 5 | 5 | | 5 | 5 | | | | | | |

Test Example 4

Herbicidal test by upland foliage treatment

Into each plastic pot of 120 $cm_2$, upland soil was filled, then seeds of barnyardgrass, crabgrass, redroot pigweed, rice flatsedge were sown, and they were grown in a greenhouse until barnyardgrass reached 3-leaf stage. Each wettable powder prepared from the test compound in accordance with Formulation Example 3 was diluted with water to give the amount of active ingredient corresponding to 4 kg/hectare,

and the diluted solution was uniformly sprayed at an application rate of 1000 liter/hectare from the top of plants by means of a small size spray, when barnyardgrass was in the 3-leaf stage. . They were grown in a greenhouse, and twenty days after the application, the herbicidal effects were evaluated in accordance with the standards as identified in Table 5. The results thereby obtained are shown in Table 9.

Table 9

| Test compound | Herbicidal effects | | | |
|---|---|---|---|---|
| | Barnyard-grass | Crabgrass | Redroot pigweed | Rice flatsedge |
| 1 | 4 | 4 | 4 | 5 |
| 5 | 4 | 5 | 4 | 4 |
| 6 | 4 | 5 | 4 | 4 |
| 16 | 5 | 5 | 1 | 5 |
| 23 | 5 | 4 | 4 | 5 |
| 28 | 5 | 4 | 3 | 5 |
| 30 | 5 | 5 | 4 | 5 |
| 33 | 5 | 5 | 5 | 5 |
| 34 | 5 | 5 | 5 | 5 |
| 38 | 5 | 5 | 5 | 5 |
| 39 | 5 | 5 | 4 | 5 |
| 40 | 4 | 5 | 4 | 5 |
| 41 | 5 | 5 | 4 | 4 |
| 42 | 5 | 5 | 4 | 5 |
| 45 | 5 | 5 | 4 | 5 |
| 48 | 5 | 5 | 5 | 5 |
| 61 | 5 | 4 | 4 | 5 |
| 68 | 5 | 5 | 3 | 5 |
| 82 | 5 | 5 | 5 | 5 |
| 86 | 5 | 5 | 3 | 4 |
| 113 | 5 | 5 | 4 | 5 |
| 132 | 5 | 5 | 5 | 5 |
| 134 | 5 | 5 | 5 | 5 |
| 138 | 5 | 5 | 5 | 5 |
| 146 | 5 | 5 | 5 | 5 |
| 172 | 5 | 5 | 5 | 5 |
| 179 | 4 | 4 | 4 | 5 |
| 183 | 5 | 5 | 5 | 5 |
| 186 | 5 | 5 | 2 | 4 |
| 187 | 5 | 4 | 3 | 3 |
| 188 | 5 | 4 | 2 | 3 |
| 189 | 4 | 5 | 2 | 3 |
| 190 | 5 | 5 | 4 | 2 |
| 191 | 5 | 4 | 2 | 4 |
| 193 | 5 | 5 | 2 | 3 |

Table 9 (cont'd)

| Test compound | Herbicidal effects | | | |
|---|---|---|---|---|
| | Barnyard-grass | Crabgrass | Redroot pigweed | Rice flatsedge |
| 194 | 4 | 3 | 2 | 3 |
| 195 | 5 | 5 | 2 | 3 |
| 196 | 3 | 2 | 3 | 4 |
| 197 | 4 | 4 | 5 | 3 |
| 198 | 5 | 5 | 1 | 3 |
| 199 | 5 | 5 | 5 | 3 |
| 200 | 5 | 4 | 4 | 3 |
| 201 | 5 | 5 | 3 | 5 |
| 202 | 4 | 4 | 3 | 2 |
| 203 | 3 | 4 | 2 | 2 |
| 204 | 3 | 3 | 3 | 1 |
| 205 | 2 | 1 | 4 | 2 |
| 206 | 5 | 5 | 3 | 3 |
| 207 | 5 | 5 | | 2 |
| 208 | 3 | 2 | 2 | 2 |
| 209 | 3 | 2 | 3 | 3 |
| 210 | 4 | 2 | 3 | 2 |
| 213 | 4 | 4 | 5 | 3 |
| 214 | 0 | 3 | 2 | 5 |
| 217 | 5 | 4 | 1 | 3 |
| 218 | 2 | 3 | 2 | 3 |
| 219 | 5 | 5 | 2 | 2 |
| 220 | 4 | 4 | 3 | 3 |
| 222 | 5 | 4 | 3 | 5 |
| 223 | 4 | 4 | 3 | 4 |
| 225 | 5 | 5 | 3 | 3 |
| 226 | 3 | 4 | 1 | 2 |
| 227 | 2 | 3 | 0 | 3 |
| 228 | 5 | 5 | | 4 |
| 230 | 5 | 5 | 2 | 3 |
| 231 | 5 | 5 | 4 | 4 |
| 232 | 5 | 5 | 4 | 4 |
| 233 | 5 | 5 | 2 | 3 |
| 234 | 3 | 5 | 2 | 3 |

Table 9 (cont'd)

| Test compound | Herbicidal effects | | | |
|---|---|---|---|---|
| | Barnyard-grass | Crabgrass | Redroot pigweed | Rice flatsedge |
| 235 | 5 | 5 | 5 | 4 |
| 237 | 4 | 4 | 0 | 2 |
| 238 | 4 | 4 | 2 | 4 |
| 239 | 4 | 4 | 2 | 4 |
| 240 | 4 | 4 | 2 | 4 |
| 242 | 4 | 5 | 3 | 5 |
| 243 | 5 | 4 | 4 | 5 |
| 244 | 5 | 5 | 1 | 5 |
| 245 | 4 | 3 | 2 | 5 |
| 246 | 5 | 4 | 3 | 4 |
| 247 | 4 | 5 | 2 | 5 |
| 248 | 5 | 4 | 1 | 4 |
| 249 | 5 | 5 | 2 | 3 |
| 251 | 4 | 5 | 2 | 3 |

**Claims**

1. A substituted phenoxy urea having the formula:

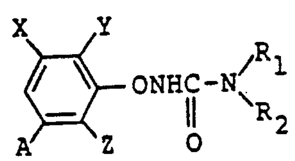

(I)

wherein A is a halogen atom or a trifluoromethyl group, each of X, Y and Z is a hydrogen atom, a halogen atom or a trifluoromethyl group, $R_1$ is a $C_1$—$C_6$ alkyl group, a lower alkoxyl group, a lower alkoxyl-substituted lower alkyl group, a cyclo-lower alkyl substituted lower alkyl group, a lower alkenyl group, a lower alkynyl group, a lower haloalkyl group, a lower haloalkenyl group or a $C_3$—$C_9$ non-aromatic cyclic hydrocarbon group, and $R_2$ is a hydrogen atom, a $C_1$—$C_6$ alkyl group, a lower alkenyl group or a lower alkynyl group, or $R_1$ and $R_2$ form, together with the nitrogen atom to which they are bonded, a 3—8 member ring (which may be a bicyclo ring) which may contain a double bond or an oxygen atom within the ring and may have one or more branches.

2. The urea according to Claim 1, wherein A is a chlorine atom, and each of X, Y and Z is a hydrogen atom or a chlorine atom.

3. The urea according to Claim 1, wherein the $C_1$—$C_6$ alkyl group for each of $R_1$ and $R_2$ is methyl, ethyl, n-propyl, isopropyl, n-butyl, isobutyl, sec-butyl, tert-butyl, n-amyl, isoamyl, sec-amyl, active amyl, tert-amyl, 1,2-dimethylpropyl, 2,2-dimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethylpropyl 2-ethylbutyl; the lower alkenyl group for each of $R_1$ and $R_2$ is allyl, 2-butenyl, 3-butenyl, 1-methyl-2-propenyl, 2-methyl-2-propenyl, 1,2-dimethyl-2-propenyl, 2-methyl-2-butenyl, 3-methyl-2-butenyl or 2,3-dimethyl-2-butenyl; the lower alkynyl group for each for $R_1$ and $R_2$ is 2-propynyl, 2-butynyl, 3-butynyl, 1-methyl-2-propynyl or 1,1-dimethyl-2-propynyl; the lower alkoxyl group for $R_1$ is a $C_1$—$C_4$ alkoxyl group such as methoxy, ethoxy, propoxy, isopropoxy or butoxy, preferably methoxy; the lower alkoxyl group of the lower alkoxyl-substituted lower alkyl group for $R_1$ is the same as the above mentioned lower alkoxyl group, and the lower alkyl group thereof is methyl, ethyl, propyl, isopropyl or butyl, a preferred lower alkoxyl-substituted lower alkyl group is 2-methoxyethyl; the cyclo-lower alkyl group of the cyclo-lower alkyl-substituted lower alkyl group for $R_1$ is a $C_3$—$C_5$ cycloalkyl group, and the lower alkyl group thereof is the same as mentioned above, preferably methyl; the lower haloalkyl group for $R_1$ is chloromethyl, 2-

chloroethyl, 2-bromoethyl, 2,2,2-trifluoroethyl or 2,2,3,3-tetrafluoropropyl; the lower haloalkenyl group for $R_1$ is 2-chloro-2-propenyl, 2-bromo-2-propenyl, 1-chloromethyl-2-propenyl or 3-chloro-2-butenyl; the $C_3$—$C_9$ non-aromatic cyclic hydrocarbon group for $R_1$ is cyclopropyl, cyclobutyl, cyclopentyl, 1-methylcyclopentyl, 2-methylcyclopentyl, 3-methylcyclopentyl, 2-cyclopentyl, cyclohexyl, 1-methylcyclohexyl, 2-methylcyclohexyl, 3-methylcyclohexyl, 4-methylcyclohexyl, 2-cyclohexenyl, 3-cyclohexenyl, cycloheptyl, cyclooctyl, 1-norbornyl or 2-norbornyl; and the 3—8 member ring formed by $R_1$ and $R_2$ together with the nitrogen atom to which they are bonded, is

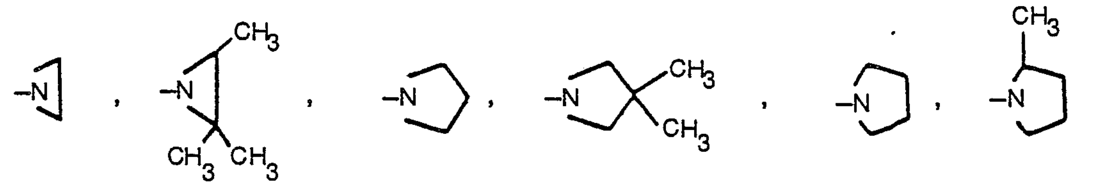

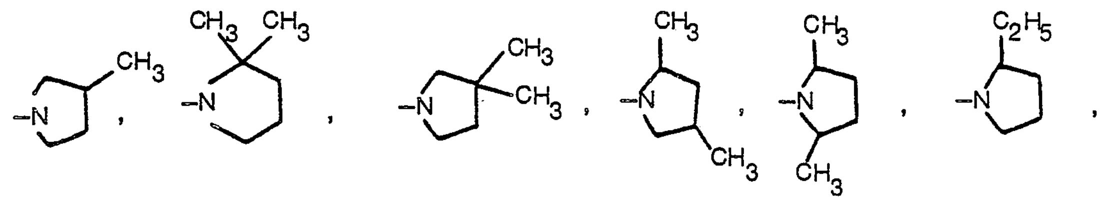

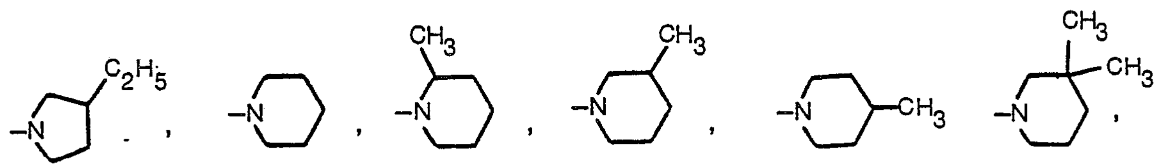

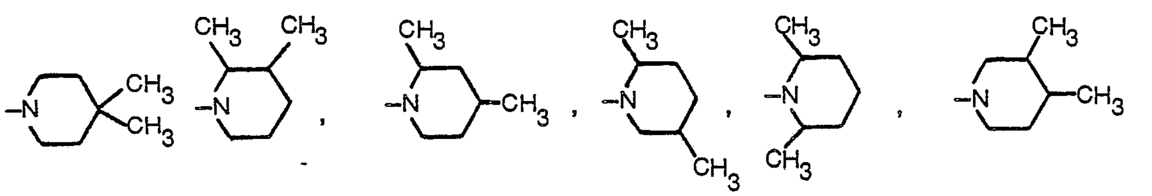

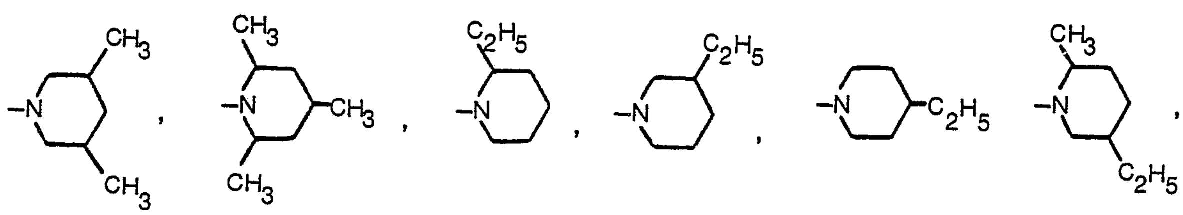

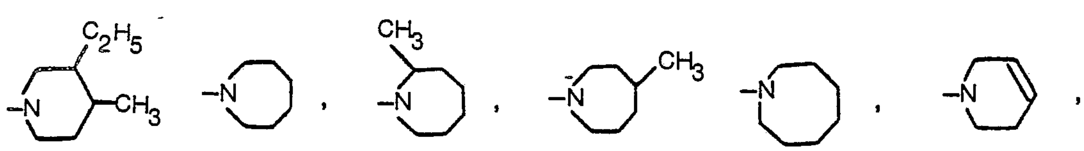

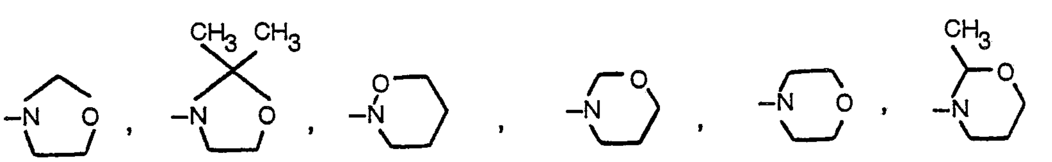

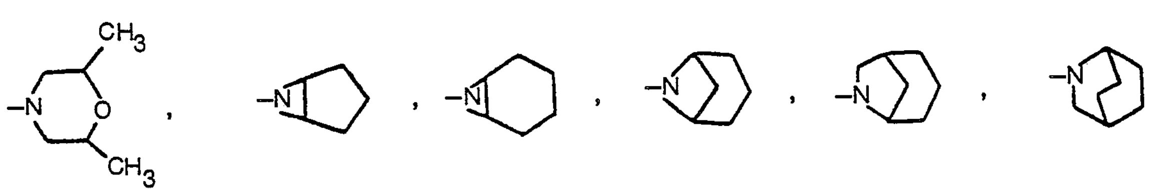

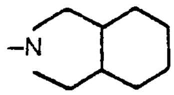 , 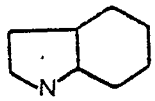 , 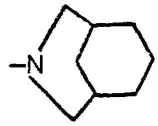 , 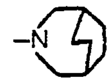 , or 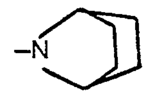 .

4. The urea according to Claim 1, wherein the aryl moiety of the formula:

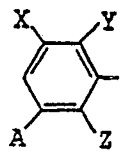

in the formula I, is 3-chlorophenyl, 2,3-dichlorophenyl, 3,5-dichlorophenyl, 2,5-dichlorophenyl, 2,5-difluorophenyl, 2,3,5-trichlorophenyl, 2,3,6-trichlorophenyl, 2,3,5,6-tetrachlorophenyl, 3-trifluoromethylphenyl, 3,5-di(trifluoromethyl)phenyl, 2,3-di(trifluoromethyl)phenyl, 2,5-di(trifluoromethyl)phenyl, 2-chloro-3-trifluoromethylphenyl, 3-chloro-2-trifluoromethylphenyl, 2-chloro-5-trifluoromethylphenyl, 5-chloro-2-trifluoromethylphenyl or 3-chloro-5-trifluoromethylphenyl.

5. The urea according to Claim 1, which has the formula:

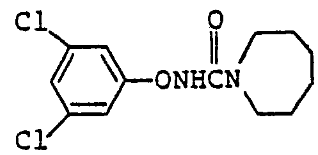

6. The urea according to Claim 1, which has the formula:

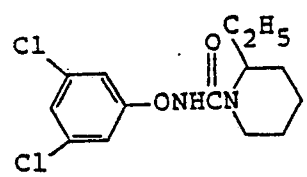

7. The urea according to Claim 1, which has the formula:

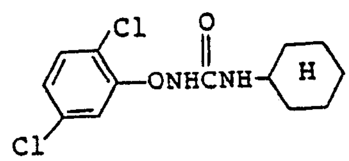

8. The urea according to Claim 1, which has the formula:

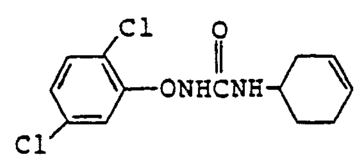

9. The urea according to Claim 1, which has the formula:

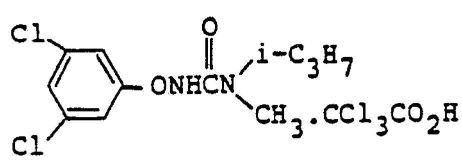

10. The process for preparing a substituted phenoxy urea having the formula:

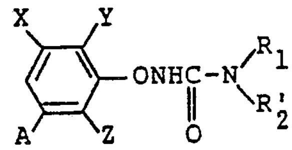

(I-I)

wherein A is a halogen atom or a trifluoromethyl group, each of X, Y and Z is a hydrogen atom, a halogen atom or a trifluoromethyl group, $R_1$ is a $C_1$—$C_6$ alkyl group, a lower alkoxyl group, a lower alkoxyl-substituted lower alkyl group, a cyclo-lower alkyl substituted lower alkyl group, a lower alkenyl group, a lower alkynyl group, a lower haloalkyl group, a lower haloalkenyl group or a $C_3$—$C_9$ non-aromatic cyclic hydrocarbon group, and $R_2'$ is a $C_1$—$C_6$ alkyl group, a lower alkenyl group or a lower alkynyl group, or $R_1$ and $R_2'$ form, together with the nitrogen atom to which they are bonded, a 3—8 member ring (which may be a bicyclo ring) which may contain a double bond or an oxygen atom within the ring and may have one or more branches, which comprise reacting a phenoxyamine having the formula:

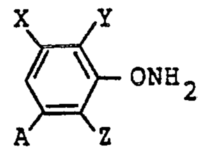

(II)

wherein A, X, Y and Z are as defined above, with a carbamic acid chloride having the formula:

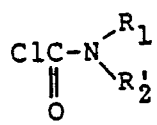

(III)

wherein $R_1$ and $R_2'$ are as defined above, in the presence of a base.

11. A process for preparing a substituted phenoxy urea having the formula:

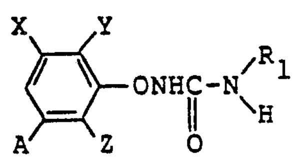

(I-II)

wherein A is a halogen atom or a trifluoromethyl group, each of X, Y and Z is a hydrogen atom, a halogen atom or a trifluoromethyl group, and $R_1$ is a $C_1$—$C_6$ alkyl group, a lower alkoxyl group, a lower alkoxyl-substituted lower alkyl group, a cyclo-lower alkyl substituted lower alkyl group, a lower alkenyl group, a lower alkynyl group, a lower haloalkyl group, a lower haloalkenyl group or a $C_3$—$C_9$ non-aromatic cyclic hydrocarbon group, which comprises reacting a phenoxyamine having the formula:

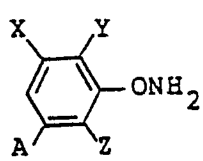

(II)

wherein A, X, Y and Z are as defined above, with an isocyanate having the formula:

$$OCN—R_1 \quad \text{(IV)}$$

wherein $R_1$ is as defined above.

12. A process for preparing a substituted phenoxy urea having the formula:

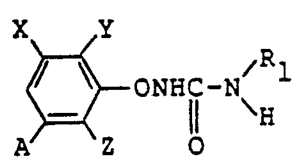

(II-I)

wherein A is a halogen atom or a trifluoromethyl group, each of X, Y and Z is a hydrogen atom, a halogen atom or a trifluoromethyl group, $R_1$ is a $C_1$—$C_6$ alkyl group, a lower alkoxyl group, a lower alkoxyl-substituted lower alkyl group, a cyclo-lower alkyl substituted lower alkyl group, a lower alkenyl group, a lower alkynyl group, a lower haloalkyl group, a lower haloalkenyl group or a $C_3$—$C_9$ non-aromatic cyclic hydrocarbon group, which comprises reacting a phenoxy carbamate having the formula:

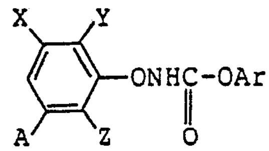

(V)

wherein A, X, Y and Z are as defined above, and Ar is an aryl group, with an amine having the formula:

$$R_1NH_2 \qquad (VI)$$

wherein $R_1$ is as defined above.

13. A herbicide comprising a herbicidally effective amount of substituted phenoxy urea having the formula:

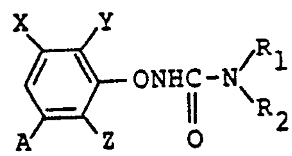

(I)

wherein A is a halogen atom or a trifluoromethyl group, each of X, Y and Z is a hydrogen atom, a halogen atom or a trifluoromethyl group, $R_1$ is a $C_1$—$C_6$ alkyl group, a lower alkoxyl group, a lower alkoxyl-substituted lower alkyl group, a cyclo-lower alkyl, substituted lower alkyl group, a lower alkenyl group, a lower alkynyl group, a lower haloalkyl group, a lower haloalkenyl group or a $C_3$—$C_9$ non-aromatic cyclic hydrocarbon group, and $R_2$ is a hydrogen atom, a $C_1$—$C_6$ alkyl group, a lower alkenyl group or a lower alkynyl group, or $R_1$ and $R_2$ form, together with the nitrogen atom to which they are bonded, a 3—8 member ring (which may be a bicyclo ring) which may contain a double bond or an oxygen atom within the ring and may have one or more branches, and a carrier.

**Patentansprüche**

1. Substituierter Phenoxyharnstoff der Formel

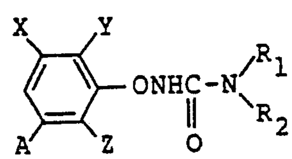

(I)

wobei A für ein Halogenatom oder eine Trifluormethylgruppe steht, jeder der Reste X, Y und Z für ein Wasserstoffatom, ein Halogenatom oder eine Trifluormethylgruppe steht, $R_1$ für eine $C_{1-6}$-Alkylgruppe, eine niedere Alkoxygruppe, eine Niederalkoxy-subst.-niederalkylgruppe, eine Cyclo-niederalkyl-subst.-niederalkylgruppe, eine niedere Alkenylgruppe, eine niedere Alkinylgruppe, eine niedere Halogenalkylgruppe, eine niedere Halogenalkenylgruppe oder eine $C_{3-9}$-nicht-aromatische cyclische Kohlenwasserstoffgruppe steht und $R_2$ für ein Wasserstoffatom, eine $C_{1-6}$-Alkylgruppe, eine niedere Alkenylgruppe oder eine niedere Alkinylgruppe steht oder wobei $R_1$ und $R_2$ gemeinsam mit dem Stickstoffatom, an das sie geknüpft, einen drei- bis achtgliedrigen Ring (der ein Bicycloring sein kann) bilden, der eine Doppelbindung enthalten kann oder ein Sauerstoffatom in Ring und eine oder mehrere Verzweigungen aufweisen kann.

2. Harnstoff gemäß Anspruch 1, wobei A für ein Chloratom steht und jeder der Reste X, Y und Z für ein Wasserstoffatom oder eine Chloratom steht.

3. Harnstoff gemäß Anspruch 1, wobei die $C_{1-6}$-Alkylgruppe für jeden der Reste $R_1$ und $R_2$ Methyl, Ethyl, n-Propyl, Isopropyl, n-Butyl, Isobutyl, sec-Butyl, tert-Butyl, n-Amyl, Isoamyl, sec-Amyl, aktives Amyl, tert-Amyl, 1,2-Dimethylpropyl, 2,2-Dimethylpropyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1,1,2-Trimethylpropyl, 1,2,2-Trimethylpropyl, 1-Ethyl-propyl oder 2-Ethylbutyl ist; die niedere Alkenylgruppe für jeden der Reste $R_1$ und

$R_2$ Allyl, 2-Butenyl, 3-Butenyl, 1-Methyl-2-propenyl, 2-Methyl-2-propenyl, 1,2-Dimethyl-2-propenyl, 2-Methyl-2-butenyl, 3-Methyl-2-butenyl oder 2,3-Dimethyl-2-butenyl ist; die niedere Alkinylgruppe für jeden der Reste $R_1$ und $R_2$ 2-Propinyl, 2-Butinyl, 3-Butinyl, 1-Methyl-2-propinyl oder 1,2-Dimethyl-2-propinyl ist; die niedere Alkoxygruppe für $R_1$ eine $C_{1-4}$-Alkoxygruppe wie Methoxy, Ethoxy, Propoxy, Isopropoxy oder Butoxy, vorzugsweise Methoxy ist; die niedere Alkoxygruppe der Niederalkoxy-subst.-niederalkylgruppe für $R_1$ die gleiche ist wie die oben erwähnte niedere Alkoxygruppe und die niedere Alkylgruppe derselben Methyl, Ethyl, Propyl, Isopropyl oder Butyl ist, wobei eine bevorzugte Niederalkoxy-subst.-niederalkylgruppe 2-Methoxyethyl ist; die Cyclo-niederalkylgruppe der Cyclo-niederalkyl-subst.-niederalkylgruppe für $R_1$ eine $C_{3-5}$-Cycloalkylgruppe ist und die Niederalkylgruppe derselben die gleiche ist wie oben erwähnt, vorzugsweise Methyl; die niedere Halogenalkylgruppe für $R_1$ Chlormethyl, 2-Chlorethyl, 2-Bromoethyl, 2,2,2-Trifluormethyl oder 2,2,3,3-Tetrafluorpropyl ist; die niedere Halogenalkenylgruppe für $R_1$ 2-Chlor-2-propenyl, 2-Brom-2-propenyl, 1-Chlormethyl-2-propenyl oder 3-Chlor-2-butenyl ist; die $C_{3-9}$-nicht-aromatische cyclische Kohlenwasserstoffgruppe für $R_1$ Cyclopropyl, Cyclobutyl, Cyclopentyl, 1-Methylcyclopentyl, 2-Methylcyclopentyl, 3-Methylcyclopenyl, 2-Cyclopentenyl, Cyclohexyl, 1-Methylcyclohexyl, 2-Methylcyclohexyl, 3-Methylcyclohexyl, 4-Methylcyclohexyl, 2-Cyclohexenyl, 3-Cyclohexenyl, Cycloheptyl, Cyclooktyl, 1-Norbonyl oder 2-Norbonyl ist; und der drei- bis achtgliedrige Ring, der durch $R_1$ und $R_2$ gemeinsam mit dem Stickstoffatom, an das sie geknüpft sind, gebildet wird, eine Struktureinheit der folgenden Formeln ist

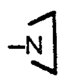 , 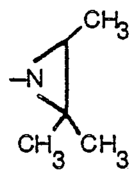 , 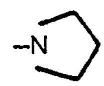 , 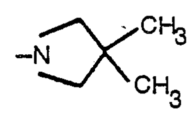 , 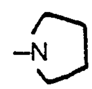 , 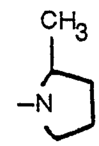 ,

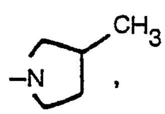 , 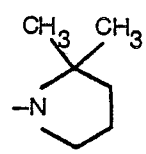 , 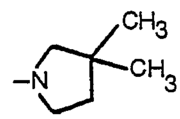 , 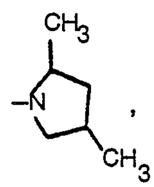 , 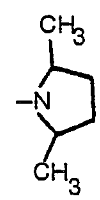 , 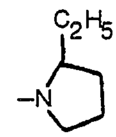 ,

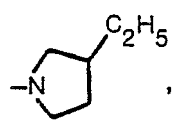 , 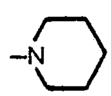 , 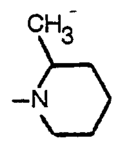 , 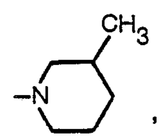 , 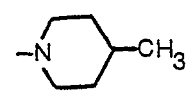 , 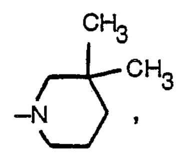 ,

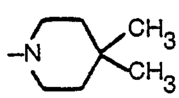 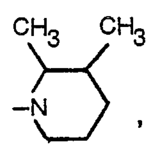 , 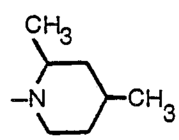 , 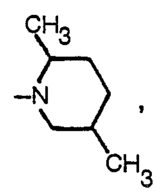 , 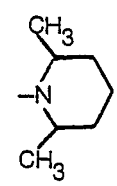 , 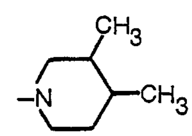 ,

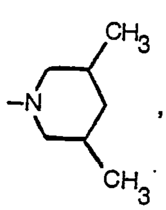 , 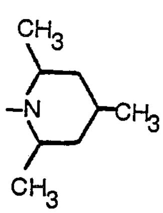 , 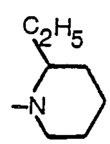 , 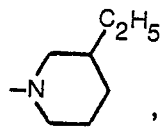 , 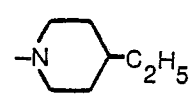 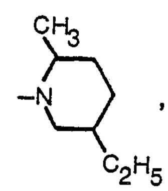 ,

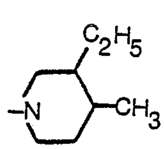 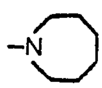 , 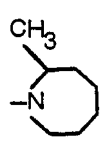 , 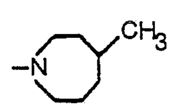 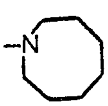 , 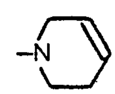 ,

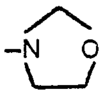 , 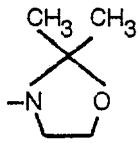 , 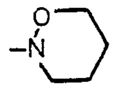 , 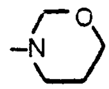 , 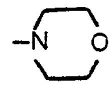 , 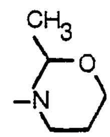 ,

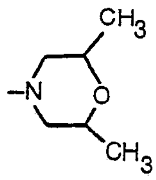 , 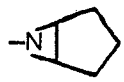 , 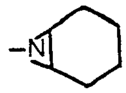 , 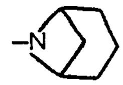 , 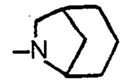 , 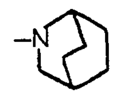 ,

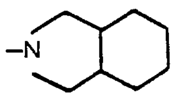 , 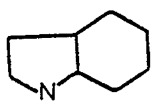 , 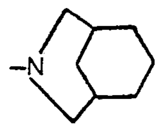 , 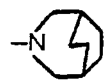 , oder 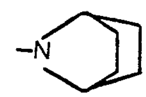 .

4. Harnstoff gemäß Anspruch 1, wobei die Aryleinheit der Formel

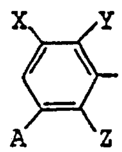

in der Formel (I) 3-Chlorphenyl, 2,3-Dichlorphenyl, 3,5-Dichlorphenyl, 2,5-Dichlorphenyl, 2,5-Difluorphenyl, 2,3,5-Trichlorphenyl, 2,3,6-Trichlorphenyl, 2,3,5,6-Tetrachlorphenyl, 3-Trifluormethylphenyl, 3,5-Di(trifluormethyl)phenyl, 2,3-Di(trifluormethyl)-phenyl, 2,5-di(trifluormethyl)-phenyl, 2-Chlor-3-trifluormethylphenyl, 3-Chlor-2-trifluormethylphenyl, 2-Chlor-5-trifluormethylphenyl, 5-Chlor-2-trifluormethylphenyl oder 3-Chlor-5-trifluormethylphenyl ist.

5. Harnstoff gemäß Anspruch 1 mit der Formel

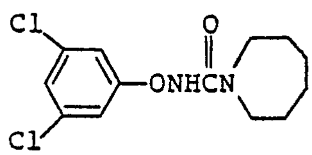

6. Harnstoff gemäß Anspruch 1 mit der Formel

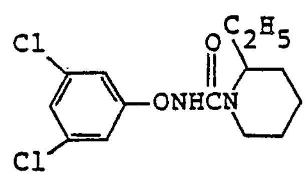

7. Harnstoff gemäß Anspruch 1 mit der Formel

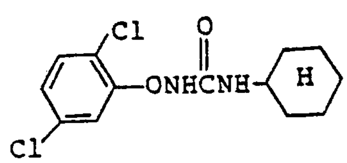

8. Harnstoff gemäß Anspruch 1 mit der Formel

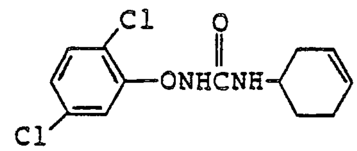

9. Harnstoff gemäß Anspruch 1 mit der Formel

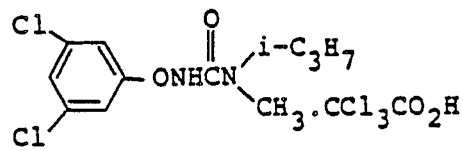

10. Verfahren zur Herstellung eines substituierten Phenoxyharnstoffs mit der Formel

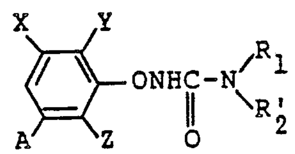

(I-I)

wobei A ein Halogenatom oder eine Trifluormethylgruppe bedeutet, jeder der Reste X, Y und Z für ein Wasserstoffatom, ein Halogenatom oder eine Trifluormethylgruppe steht, $R_1$ für eine $C_{1-6}$-Alkylgruppe, eine niedere Alkoxygruppe, eine Niederalkoxy-subst.-niederalkylgruppe, eine Cyclo-niederalkyl-subst.-niederalkylgruppe, eine niedere Alkenylgruppe, eine niedere Alkinylgruppe, eine niedere Halogenalkylgruppe, eine niedere Halogenalkenylgruppe oder eine $C_{3-9}$-nicht-aromatische cyclische Kohlenwasserstoffgruppe steht und $R_2'$ für eine $C_{1-6}$-Alkylgruppe, eine niedere Alkenylgruppe oder eine niedere Alkinylgruppe steht oder wobei $R_1$ und $R_2'$ gemeinsam mit dem Stickstoffatom, an das sie geknüpft, einen drei- bis achtgliedrigen Ring (der auch ein Bicycloring sein kann) bilden, welcher im Ring eine Doppelbindung oder ein Sauerstoffatom enthalten kann und eine oder mehrere Verzweigungen aufweisen kann, umfassend die Umsetzung eines Phenoxyamins der Formel

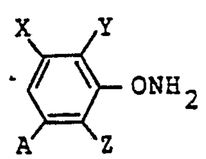

(II)

wobei A, X, Y und Z wie oben definiert sind, mit einem Carbaminsäurechlorid der Formel

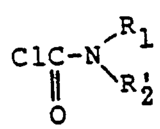

(III)

wobei $R_1$ und $R_2'$ wie oben definiert sind, in Gegenwart einer Base.

11. Verfahren zur Herstellung eines substituiertes Phenoxyharnstoffs mit der Formel

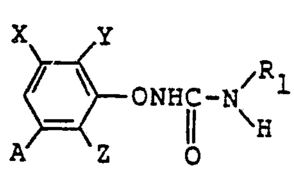

(I-II)

wobei A ein Halogenatom oder eine Trifluormethylgruppe bedeutet, jeder der Reste X, Y und Z für ein Wasserstoffatom, ein Halogenatom oder eine Trifluormethylgruppe steht und $R_1$ für eine $C_{1-6}$-Alkylgruppe, eine niedere Alkoxygruppe, eine Niederalkoxy-subst.-niederalkylgruppe, eine Cyclo-niederalkyl-subst.-niederalkylgruppe, eine niedere Alkenylgruppe, eine niedere Alkinylgruppe, eine niedere

Halogenalkylgruppe, eine niedere Halogenalkenylgruppe oder eine $C_{3-9}$-nicht-aromatische cyclische Kohlenwasserstoffgruppe steht, umfassend die Umsetzung eines Phenoxyamins der Formel

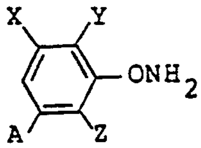

(II)

wobei A, X, Y und Z wie oben definiert sind, mit einem Isocyanat der Formel

$$OCN—R_1 \quad (IV)$$

wobei $R_1$ wie oben definiert ist.

12. Verfahren zur Herstellung eines substituierten Phenoxyharnstoffs mit der Formel

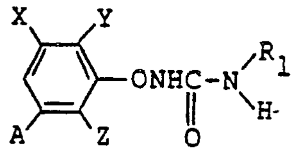

(I-II)

wobei A ein Halogenatom oder eine Trifluormethylgruppe bedeutet, jeder der Reste X, Y und Z für ein Wasserstoffatom, ein Halogenatom oder eine Trifluormethylgruppe steht und $R_1$ für eine $C_{1-6}$-Alkylgruppe, eine niedere Alkoxygruppe, eine Niederalkoxy-subst.-niederalkylgruppe, eine Cyclo-niederalkyl-subst.-niederalkylgruppe, eine niedere Alkenylgruppe, eine niedere Alkinylgruppe, eine niedere Halogenalkylgruppe, eine niedere Halogenalkenylgruppe oder eine $C_{3-9}$-nicht-aromatische cyclische Kohlenwasserstoffgruppe steht, umfassend die Umsetzung eines Phenoxycarbamats der Formel

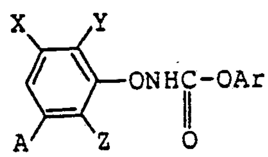

(V)

wobei A, X, Y und Z wie oben definiert sind und Ar für eine Arylgruppe steht, mit einem Amin der Formel

$$R_1NH_2 \quad (VI)$$

wobei $R_1$ wie oben definiert ist.

13. Herbizid, umfassend eine herbizid wirksame Menge eines substituierten Phenoxyharnstoffs mit der Formel

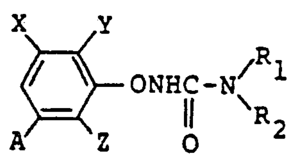

(I)

wobei A ein Halogenatom oder eine Trifluormethylgruppe bedeutet, jeder der Reste X, Y und Z für ein Wasserstoffatom, ein Halogenatom oder eine Trifluormethylgruppe steht, $R_1$ für eine $C_{1-6}$-Alkylgruppe, eine niedere Alkoxygruppe, eine Niederalkoxy-subst.-niederalkylgruppe, eine Cyclo-niederalkyl-subst.-niederalkylgruppe, eine niedere Alkenylgruppe, eine niedere Alkinylgruppe, eine niedere Halogenalkylgruppe, eine niedere Halogenalkenylgruppe oder eine $C_{3-9}$-nicht-aromatische cyclische Kohlenwasserstoffgruppe steht und $R_2$ für ein Wasserstoffatom, eine $C_{1-6}$-Alkylgruppe eine niedere Alkenylgruppe oder eine niedere Alkinylgruppe steht oder $R_1$ und $R_2$ gemeinsam mit dem Stickstoffatom, an das sie geknüpft sind, einen drei- bis achtgliedrigen Ring (der auch ein Bicycloring sein kann) bilden, welcher im Ring eine Doppelbindung oder ein Sauerstoffatom enthalten kann und eine odere mehrere Verzweigungen aufweisen kann,

und einen Träger.

**Revendications**

1. Phénoxy-urée substituée présentant la formule:

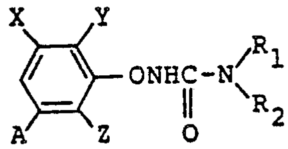

(I)

dans laquelle:

A représente un atome d'halogène ou un groupe trifluorométhyle; X, Y et Z représentent chacun un atome d'hydrogène, un atome d'halogène ou un groupe trifluorométhyle; $R_1$ représente un groupe alkyle en $C_1$—$C_6$, un groupe alcoxyle inférieur, un groupe alkyle inférieur substitué par alcoxyle inférieur, un groupe alkyle inférieur substitué par cycloalkyle inférieur, un groupe alcényle inférieur, un groupe alcynyle inférieur, un groupe haloalkyle inférieur, un groupe haloalcényle inférieur ou un groupe hydrocarboné cyclique non-aromatique en $C_3$—$C_9$; et $R_2$ représente un atome d'hydrogène, un groupe alkyle en $C_1$—$C_6$, un groupe alcényle inférieur ou un groupe alcynyle inférieur; ou bien, $R_1$ et $R_2$ forment, conjointement avec l'atome d'azote auquel ils sont liés, un groupe cyclique à 3—8 chaînons (qui peut être un groupe bicyclique), qui peut contenir une double liaison ou un atome d'oxygène dans le cycle et qui peut présenter au moins un ramification.

2. Urée selon la revendication 1, dans laquelle A représente un atome de chlore, et X, Y et Z représentent chacun un atome d'hydrogène ou un atome de chlore.

3. Urée selon la revendication 1, dans laquelle: le groupe alkyle en $C_1$—$C_6$ entrant dans la définition de chacun parmi $R_1$ et $R_2$ est méthyle, éthyle, n-propyle, isopropyle, n-butyle, isobutyle, sec.-butyle, tert.-butyle, n-amyle, isoamyle, sec.-amyle, amyle activé, tert.-amyle, diméthyl-1,2 propyle, diméthyl-2,2 propyle, diméthyl-1,1 butyle, diméthy-1,2 butyle, diméthyl-1,3 butyle, diméthyl-2,2 butyle, diméthyl-2,3 butyle, diméthyl-3,3 butyle, triméthyl-1,1,2 propyle, triméthyl-1,2,2 propyle, éthyl-1 propyle ou éthyl-2 butyle;

le groupe alcényle inférieur entrant dans la définition de chacun parmi $R_1$ et $R_2$ est allyle, butényle-2, butényle-3, méthyl-1 propényle-2, méthyl-2 propényle-2, diméthyl-1,2 propényle-2, méthyl-2 butényle-2, méthyl-3 butényle-2 ou diméthyl-2,3 butényle-2;

le groupe alcynyle inférieur entrant dans la définition de chacun parmi $R_1$ et $R_2$ est propynyle-2, butynyle-2, butynyle-3, méthyl-1 propynyle-2 ou diméthyl-1,1 propynyle-2;

le groupe alcoxyle inférieur entrant dans la définition de $R_1$ est un groupe alcoxyle en $C_1$—$C_4$, tel que méthoxy, éthoxy, propoxy, isopropoxy ou butoxy, de préférence, méthoxy;

le groupe alcoxyle inférieur du groupe alkyle inférieur substitué par alcoxyle inférieur entrant dans la définition de $R_1$ est le même que le groupe alcoxyle inférieur mentionné ci-dessus, et son groupe alkyle inférieur est méthyle, éthyle, propyle, isopropyle ou butyle, le groupe alkyle inférieur substitué par alcoxyle inférieur que l'on préfère étant méthoxy-2 éthyle;

le groupe cycloalkyle inférieur du groupe alkyle inférieur substitué par cycloalkyle inférieur entrant dans la définition de $R_1$ est un groupe cycloalkyle en $C_3$—$C_5$, et son groupe alkyle inférieur est le même que celui mentionné ci-dessus, de préférence, méthyle;

le groupe haloalkyle inférieur entrant dans la définition de $R_1$ est chlorométhyle, chloro-2 éthyle, bromo-2 éthyle, trifluoro-2,2,2 éthyle ou tétrafluoro-2,2,3,3 propyle;

le groupe haloalcényle inférieur entrant dans la définition de $R_1$ est chloro-2 propényle-2, bromo-2 propényle-2, chlorométhyl-1 propényle-2 ou chlor-3 butényle-2;

le groupe hydrocarbone cyclique non aromatique en $C_3$—$C_9$ entrant dans la définition de $R_1$ est cyclopropyle, cyclobutyle, cyclopentyle, méthyl-1 cyclopentyle, méthyl-2 cyclopentyle, méthyl-3 cyclopentyle, cyclopenténуle-2, cyclohexyle, méthyl-1 cyclohexyle, méthyl-2 cyclohexyle, méthyl-3 cyclohexyle, méthyl-4 cyclohexyle, cyclohexényle-2, cyclohexényle-3, cycloheptyle, cyclooctyle, norbornyle-1 ou norbornyle-2; et le groupe cyclique à 3—8 chaînons formé par $R_1$ et $R_2$ conjointement avec l'atome d'azote auquel ils sont liés, est

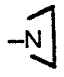 , 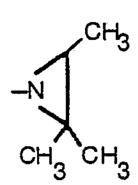 , 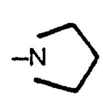 , 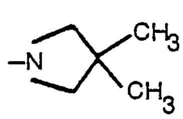 , 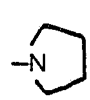 , 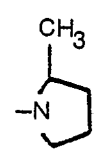 ,

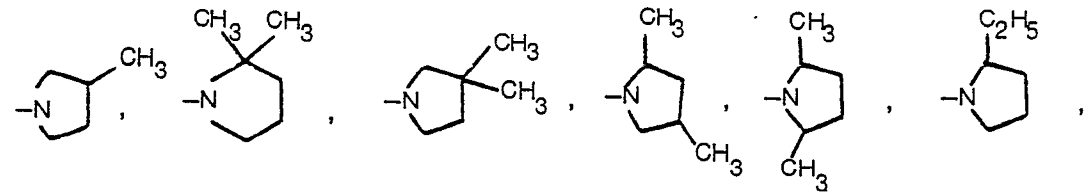

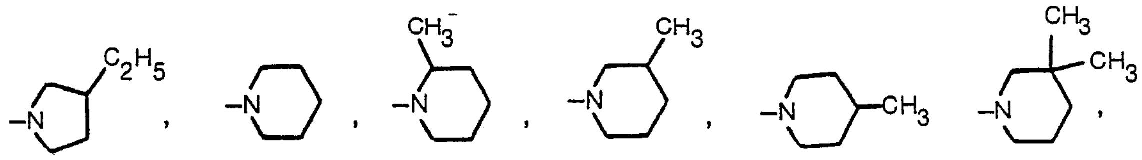

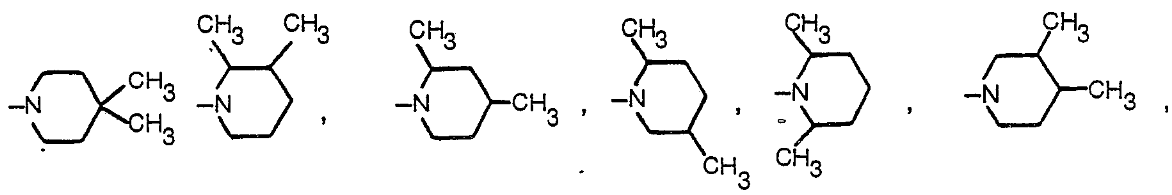

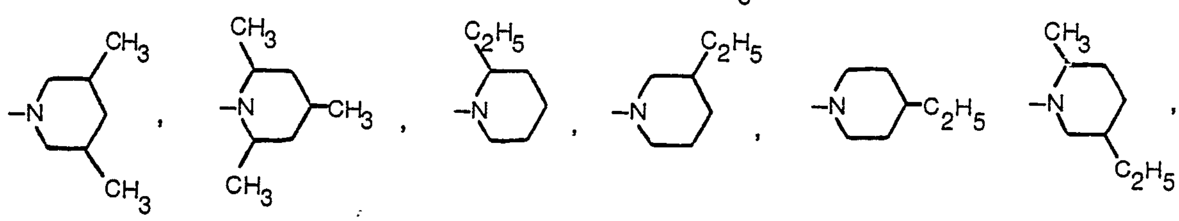

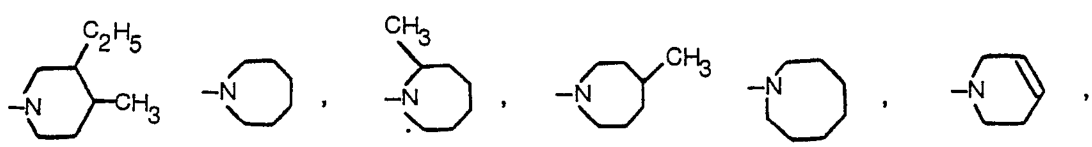

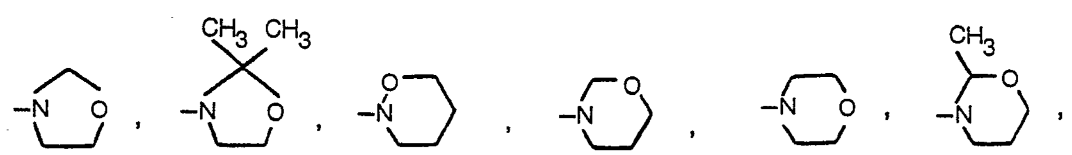

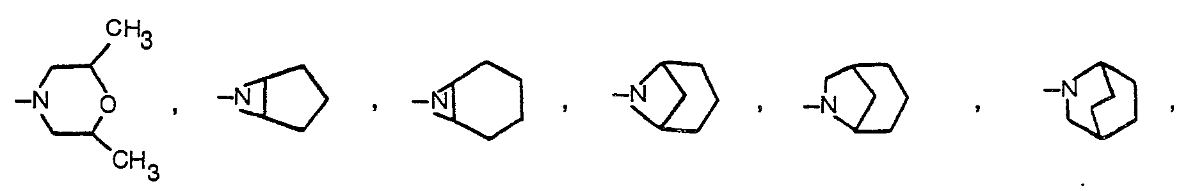

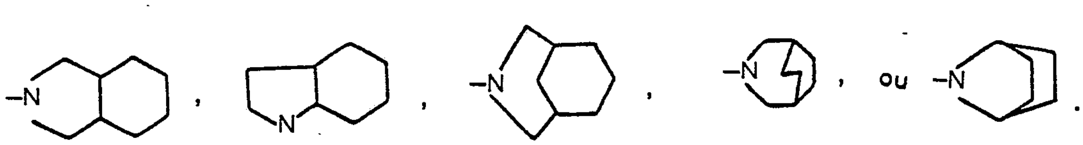

ou

4. Urée selon la revendicsation 1, dans laquelle la fraction aryle de formule:

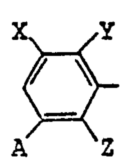

dans la formule (I), est chloro-3 phényle, dichloro-2,3 phényle, dichloro-3,5 phényle, dichloro-2,5 phényle,

difluoro-2,5 phényle, trichloro-2,3,5 phényle, trichloro-2,3,6 phényle, tétrachloro-2,3,5,6 phényle, trifluorométhyl-3 phényle, di(trifluorométhyl)-3,5 phényle, di(trifluorométhyl)-2,3 phényle, di(trifluorométhyl)-2,5-phényl, chloro-2 trifluorométhyl-3 phényle, chloro-3 trifluorométhyl-2 phényle, chloro-2 trifluorométhyl-5 phényle, chloro-5 trifluorométhyl-2 phényle ou chloro-3 trifluorométhyl-5 phényle.

5. Urée selon la revendication 1, qui présente la formule:

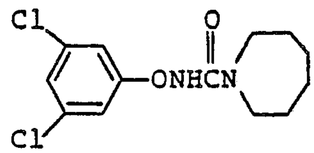

6. Urée selon la revendication 1, qui présente la formule:

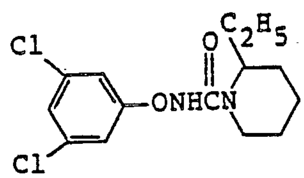

7. Urée selon la revendication 1, qui présente la formule:

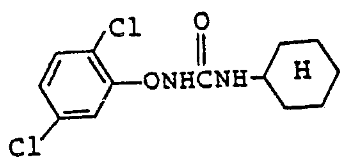

8. Urée selon la revendication 1, qui présente la formule:

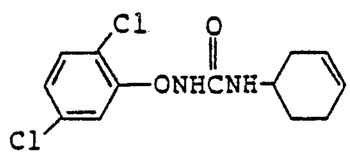

9. Urée selon la revendication 1, qui présente la formule:

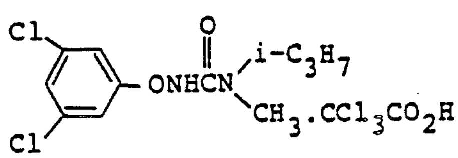

10. Procédé de préparation d'une phénoxy-urée substituée présentant la formule:

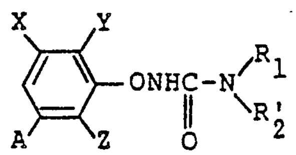

(I-I)

dans laquelle:

A représente un atome d'halogène ou un groupe trifluorométhyle; X, Y et Z représentent chacun un atome d'hydrogène, un atome d'halogène ou un groupe trifluorométhyle; $R_1$ représente un groupe alkyle en $C_1$—$C_6$, un groupe alcoxyle inférieur, un groupe alkyle inférieur substitué par alcoxyle inférieur, un groupe alkyle inférieur substitué par cycloalkyle inférieur, un groupe alcényle inférieur, un groupe alcynyle inférieur, un groupe haloalkyle inférieur, un groupe haloalcényle inférieur ou un groupe hydrocarboné cyclique non-aromatique en $C_3$—$C_9$; et $R_2'$ représente un groupe alkyle en $C_1$—$C_6$, un groupe alcényle

inférieur ou un groupe alcynyle inférieur; ou bien, $R_1$ et $R'_2$ forment, conjointement avec l'atome d'azote auquel ils sont liés, un groupe cyclique à 3—8 chaînons (qui peut être un groupe bicyclique), qui peut contenir une double liaison ou un atome d'oxygène dans le cycle et qui peut présenter au moins un ramification, qui comprend la réaction d'une phénoxyamine présentant la formule:

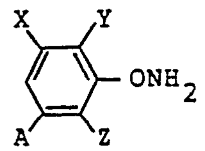

(II)

dans laquelle A, X, Y et Z sont tels que définis ci-dessus avec un chlorure d'acide carbamique présentant la formule:

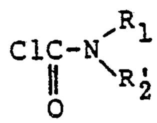

(III)

dans laquelle $R_1$ et $R'_2$ sont tels que définis ci-dessus en présence d'une base.

11. Procédé de préparation d'une phénoxy-urée substituée présentant la formule:

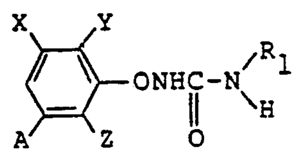

(I-II)

dans laquelle:

A représente un atome d'halogène ou un groupe trifluorométhyle; X, Y et Z représentent chacun un atome d'hydrogène, un atome d'halogène ou un groupe trifluorométhyle; et $R_1$ représente un groupe alkyle en $C_1$—$C_6$, un groupe alcoxyle inférieur, un groupe alkyle inférieur substitué par alcoxyle inférieur, un groupe alkyle inférieur substitué par cycloalkyle inférieur, un groupe alcényle inférieur, un groupe alcynyle inférieur, un groupe haloalkyle inférieur, un groupe haloalcényle inférieur ou un groupe hydrocarboné cyclique non-aromatique en $C_3$—$C_9$; qui comprend la réaction d'une phénoxyamine présentant la formule:

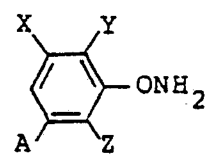

(II)

dans laquelle A, X, Y et Z sont tels que définis ci-dessus avec un isocyanate présentant la formule:

$$OCN—R_1 \qquad (IV)$$

dans laquelle $R_1$ est tel que défini ci-dessus.

12. Procédé de préparation d'une phénoxy-urée substituée présentant la formule:

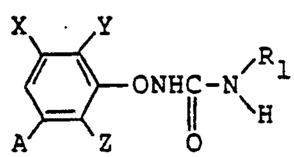

(I-II)

dans laquelle:

A représente un atome d'halogène ou un groupe trifluorométhyle; X, Y et Z représentent chacun un atome d'hydrogène, un atome d'halogène ou un groupe trifluorométhyle; $R_1$ représente un groupe alkyle en $C_1$—$C_6$, un groupe alcoxyle inférieur, un groupe alkyle inférieur substitué par alcoxyle inférieur, un groupe alkyle inférieur substitué par cycloalkyle inférieur, un groupe alcényle inférieur, un groupe alcynyle inférieur, un groupe haloalkyle inférieur, un groupe haloalcényle inférieur ou un groupe hydrocarboné cyclique non-aromatique en $C_3$—$C_9$; qui comprend la réaction d'une phénoxy-carbamate présentant la formule:

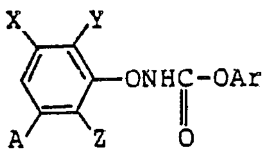

(V)

dans laquelle A, X, Y et Z sont tels que définis ci-dessus, et Ar représente un groupe aryle, avec un amine présentant la formule:

$$R_1NH_2 \qquad (VI)$$

dans laquelle $R_1$ est tel que défini ci-dessus.

13. Herbicide comprenant un quantité efficace pour détruire les mauvaises herbes d'une phénoxy-urée substituée présentant la formule:

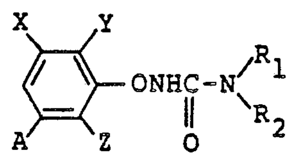

(I)

dans laquelle:

A représente un atome d'halogène ou un groupe trifluorométhyle; X, Y et Z représentent chacun un atome d'hydrogène, un atome d'halogène ou un groupe trifluorométhyle; $R_1$ représente un groupe alkyle en $C_1$—$C_6$, un groupe alcoxyle inférieur, un groupe alkyle inférieur substitué par alcoxyle inférieur, un groupe alkyle inférieur substitué par cycloalkyle inférieur, un groupe alcényle inférieur, un groupe alcynyle inférieur, un groupe haloalkyle inférieur, un groupe haloalcényle inférieur ou un groupe hydrocarboné cyclique non-aromatique en $C_3$—$C_9$; et $R_2$ représente un atome d'hydrogène, un groupe alkyle en $C_1$—$C_6$, un groupe alcényle inférieur ou un groupe alcynyle inférieur; ou bien, $R_1$ et $R_2$ forment, conjointement avec l'atome d'azote auquel ils sont liés, un groupe cyclique à 3—8 chaînons (qui peut être un groupe bicyclique), qui peut contenir une double liaison ou un atome d'oxygène dans le cycle et qui peut présenter au moins un ramification,

et un support.